# EUROPEAN PATENT APPLICATION

(11) **EP 4 752 223 A2**
(43) Date of publication of application: **03.06.2026**
(21) Application number: 26172541.0
(22) Date of filing: 19.02.2020
(51) Int. Cl.: C12N 15/113

(54) **CANCER-TARGETED, VIRUS-ENCODED, REGULATABLE T (CATVERT) OR NK CELL (CATVERN) LINKERS**

(30) Priority: 20.02.2019 US 201962808264 P
(62) Divisional of application: 20760094.1
(71) Applicant: Research Institute at Nationwide Children's Hospital, Columbus, Ohio 43205 (US)
(72) Inventor: CRIPE, Timothy, P., Colombus, OH 43205 (US); CHEN, Chun-Yu, Columbus, OH 43205 (US); HUTZEN, Brian, Columbus, OH 43205 (US); CURRIER, Mark, Columbus, OH 43205 (US); WANG, Pin-Yi, Columbus, OH 43205 (US); CHANDLER, Dawn, Columbus, OH 43205 (US)
(74) Representative: FRKelly

(57) **Abstract**

Recombinant polynucleotides and vectors containing an engineered (artificial) exon-intron-exon gene structure in a transgene are provided, which undergoes splicing when it is expressed in a target cell.

## Description

### STATEMENT OF GOVERNMENT SUPPORT

This invention was made with government support under the Grant No. W81XWH-19-1-0371 awarded by the Department of Defense. The government has certain rights to the invention.

### CROSS-REFERENCE TO RELATED APPLICATIONS

This application claims priority under 35 U.S.C. § 119(e) to U.S. Provisional Application No. 62/808,264, filed February 20, 2019, the content of which is hereby incorporated by reference in its entirety. This application is divided out of European patent application no. 20760094.1 having a filing date of 19 February 2020, the content of which is hereby incorporated by reference in its entirety. The subject matter of PCT application no. PCT/US2020/018806, filed on 19 February 2020, is hereby incorporated by reference in its entirety.

### BACKGROUND

Gene transfer as a treatment for disease (i.e., gene therapy) is now a reality. In 2017 the FDA approved Luxturna, an Adeno-associated virus (AAV) designed to stably express a normal copy of the RPE65 gene in retinal cells, for the treatment of congenital retinal dystrophy. In 2019 the FDA approved Zolgensma, an AAV designed to express the SMN1 in motor neurons to treat spinal muscular atrophy. There are highly encouraging results in ongoing clinical trials for the use of AAV vectors to replace gene function for other diseases as well, including a mini-version of the dystrophin gene for Duchenne muscular dystrophy and factor VIII and IX genes for hemophilia A and B, respectively.

In all of the above examples, the goal is long-term gene expression to replace a missing or defective gene. Yet there may be gene therapy applications in which only short-term expression of a transgene (the gene being expressed as a therapeutic in gene therapy), such as in the treatment of time-limited disease such as infection or cancer. Another illustrative example is the expression of the bacterial protein Cas9 to induce CRISPR/Cas9 gene editing, in which only transient expression of Cas9 is ideal to minimize a potential immune reaction to Cas9 and to minimize off-target gene mutations. Another application would be a circumstance in which the gene therapy might cause unwanted side effects, and there would be a desire to inactivate the gene. In these cases, a method to either activate and/or repress transgene expression is needed.

Thus, a novel method is needed to be able to activate transgene expression to utilize a gene therapy platform for short-term gene expression (weeks to months). The ideal system would place transgene expression under control of a drug, such that administration of the drug activates gene expression, and withdrawal of the drug reverts to inactive gene expression. In such a scenario, if there are side effects or if expression of the transgene is no longer needed, drug administration is no longer required. This disclosure satisfies this need and provides related advantages as well.

### SUMMARY

This disclosure provides a method or a vector to express a therapeutic gene for a disease or condition, optionally under an external control. In some instances, the disease or condition is a cancer. In some cases, the vector expresses a polypeptide (referred to herein as "Dimert") which serves to join together an immune executioner cell with a cancer cell to trigger killing of the cancer cell.

Provided herein is a recombinant polynucleotide or a vector comprising, or alternatively consisting essentially of, or yet further consisting of: (a) a first polynucleotide sequence comprising a first portion of an open reading frame encoding a first antibody or an antigen-binding fragment thereof; (b) a second polynucleotide sequence comprising a second portion of the open reading frame encoding the first antibody or an antigen-binding fragment thereof; (c) a third polynucleotide sequence encoding a second antibody or an antigen-binding fragment thereof; and (d) a gene regulation polynucleotide sequence located between the first polynucleotide and the second polynucleotide. In a further aspect, the gene regulation polynucleotide sequence comprises, or consists essentially of, or yet further consists of, a splice donor site, an upstream intron, an exon comprising more than one stop codon sequences in their respective reading frames, a downstream intron, and a splice acceptor site. In a further aspect, the gene-regulation polynucleotide sequence comprises one or more of a binding sequence for an antisense oligonucleotide. In a further aspect, the antisense oligonucleotide is a morpholino oligonucleotide. In a further aspect, the binding sequence for the morpholino oligonucleotide comprises a polynucleotide sequence at least 95% identical to SEQ ID NO. 24 (AATATGATCCAACAATAGAGGTAAATCTTG) or SEQ ID NO. 25 (GATCCAACAATAGAGGTAAATCTTGTTTTA). In another embodiment, the stop codon comprises an oligonucleotide of the group of: TAA, TAG, or TGA. In a further aspect, the stop codon sequence comprises a polynucleotide sequence of TAAxTAGxTGAxTAGxTAAxTGAx (SEQ ID NO. 1), wherein x is any nucleotide or alternatively, the stop codon sequence comprises a polynucleotide sequence of TAATTAGTTGATTAGTTAATTGAT (SEQ ID NO. 2) or an equivalent thereof. In another embodiment, the recombinant polynucleotide or a vector encodes a bispecific or trispecific engager. In another embodiment, the bispecific cell engager is a bispecific engager. In another embodiment, the bispecific cell engager is a trispecific engager.

In further embodiments, the first antibody or the antigen-binding fragment thereof specifically binds to an activating antigen on an immune effector cell and the second antibody or its antigen-binding fragment binds to a tumor antigen. In another aspect, the first antibody or its antigen-binding fragment specifically binds to a tumor antigen and the second antibody or its antigen-binding fragment binds to an activating antigen on an immune effector cell. In a further aspect, the recombinant polynucleotide or vector also comprises a fourth polynucleotide sequence encoding a third antibody or an antigen-binding fragment thereof, wherein the third antibody or the antigen-binding fragment thereof binds to an activating antigen on an immune effector cell or a tumor antigen. In one aspect, the immune effector cell comprises a dendritic cell, a natural killer ("NK") cell, a macrophage, a T cell, a B cell, a neutrophil, an eosinophil, a basophil, a mast cell, or combination thereof. In one aspect, the immune effector cell is a T cell. In another aspect, the immune effector cell is a NK cell. In another embodiment, the third antibody or the antigen-binding fragment thereof binds to an activating antigen on a NK cell, and induces an immune response mediated by the NK cell.

In one embodiment, the activating antigen is a T cell surface molecule. In another embodiment, the activating antigen is a NK cell surface molecule. Non-limiting examples of the activating antigens on the immune effector cell comprise CD3, CD2, CD4, CD8, LFA1, CD45, NKG2D, NKp44, NKp46, NKp30, EphA2, DNAM, BT-H3, CD20, CD22, or combination thereof.

In one aspect, the dimert (e.g., the bispecific or trispecific cell engager) comprises a polypeptide sequence at least 95% identical to any one of SEQ ID NOs. 7-10. In another embodiment, the polypeptide sequence encodes an antigen-binding fragment for CD3, CD2, CD4, CD8, LFA1, CD45, IL21R, NKG2D, NKp44, NKp46, NKp30, or DNAM. In another embodiment, the polypeptide sequence encodes an antigen-binding fragment for CD3, CD19, GD2, or NKG2D. In another embodiment, the polypeptide encodes a first antigen-binding fragment and a second antigen-binding fragment. In another embodiment, the first antigen-binding fragment binds to CD3 and the second antigen-binding fragment binds to CD19. In another embodiment, the first antigen-binding fragment binds to CD3 and the second antigen-binding fragment binds to GD2. In another embodiment, the first antigen-binding fragment binds to NKG2D and the second antigen-binding fragment binds to GD2.

In one embodiment, the dimert (e.g., the trispecific engager or antibody) comprises the first antigen-binding fragment, the second antigen-binding fragment, and the third antigen-binding fragment. In another embodiment, the trispecific engager or antibody comprises three antigen-binding fragments that binds to NKG2D, IL21R, and GD2, individually. In a further aspect, the trispecific engager or antibody comprises a polypeptide sequence at least at least 95% identical to SEQ ID NO. 11.

In one embodiment, the antigen-binding fragment that binds to IL-21R is IL-21. The amino acid and cDNA sequences of IL-12 are shown in SEQ ID NO. 3 and SEQ ID NO. 4, respectively.
**SEQ ID NO. 3: amino acid sequence of IL-21** 
MRSSPGNMERIVICLMVIFLGTLVHKSSSQGQDRHMIRMRQLIDIVDQLKNYVNDLVPEFLPAPEDVETNCEWSAFS
CFQKAQLKSANTGNNERIINVSIKKLKRKPPSTNAGRRQKHRLTCPSCDSYEKKPPKEFLERFKSLLQKMIHQHLSS
RTHGSEDS
**SEQ ID NO. 4: cDNA sequence of IL-21**

ATGAGAAGCAGCCCCGGCAACATGGAGAGAATCGTGATCTGCCTGATGGTGATCTTCCTGGGCACCCTGGTGCACAA
GAGCAGCAGCCAGGGCCAGGACAGACACATGATCAGAATGAGACAGCTGATCGACATCGTGGACCAGCTGAAGAACT
ACGTGAACGACCTGGTGCCCGAGTTCCTGCCCGCCCCCGAGGACGTGGAGACCAACTGCGAGTGGAGCGCCTTCAGC
TGCTTCCAGAAGGCCCAGCTGAAGAGCGCCAACACCGGCAACAACGAGAGAATCATCAACGTGAGCATCAAGAAGCT
GAAGAGAAAGCCCCCCAGCACCAACGCCGGCAGAAGACAGAAGCACAGACTGACCTGCCCCAGCTGCGACAGCTACG
AGAAGAAGCCCCCCAAGGAGTTCCTGGAGAGATTCAAGAGCCTGCTGCAGAAGATGATCCACCAGCACCTGAGCAGC
AGAACCCACGGCAGCGAGGACAGC

In one embodiment, the antigen-binding fragment that binds to NKG2D comprises MICA, MICB, ULBP1, ULBP2, ULBP3, ULBP4, ULBP5, ULBP6, Rae-1α, Rae-1β, Rae-1γ, Rae-1δ, Rae-1ε, H60a, H60b, H60c, MULT1, or a fragment thereof. In one embodiment, the antigen-binding fragment that binds to NKG2D is MICA or its fragment. The amino acid and cDNA sequences of MICA are shown in SEQ ID NO. 5 and SEQ ID NO. 6, respectively.
**SEQ ID NO. 5 amino acid sequence of MICA** 
EPHSLRYNLTVLSWDGSVQSGFLAEVHLDGQPFLRYDRQKCRAKPQGQWAEDVLGNKTWDRETRDLTGNGKDLRMTL
AHIKDQKEGLHSLQEIRVCEIHEDNSTRSSQHFYYDGELFLSQNLETEEWTVPQSSRAQTLAMNVRNFLKEDAMKTK
THYHAMHADCLQELRRYLESSVVLRRTVPPMVNVTRSEASEGNITVTCRASSFYPRNIILTWRQDGVSLSHDTQQWG
DVLPDGNGTYQTWVATRICRGEEQRFTCYMEHSGNHSTHPVPS
**SEQ ID NO. 6 cDNA sequence of MICA**

gagccccaca gtcttcgtta taacctcacg gtgctgtcct gggatggatc tgtgcagtca gggtttcttg
ctgaggtaca tctggatggt cagcccttcc tgcgctatga caggcagaaa tgcagggcaa agccccaggg
acagtgggca gaagatgtcc tgggaaataa gacatgggac agagagacca gggacttgac agggaacgga
aaggacctca ggatgaccct ggctcatatc aaggaccaga aagaaggctt gcattccctc caggagatta
gggtctgtga gatccatgaa
gacaacagca ccaggagctc ccagcatttc tactacgatg gggagctctt cctctcccaa aacctggaga
ctgaggaatg gacagtgccc cagtcctcca gagctcagac cttggccatg aacgtcagga atttcttgaa
ggaagatgcc atgaagacca agacacacta tcacgctatg catgcagact gcctgcagga actacggcga
tatctagaat ccagcgtagt cctgaggaga acagtgcccc ccatggtgaa tgtcacccgc agcgaggcct
cagagggcaa catcaccgtg acatgcaggg cttccagctt ctatccccgg aatatcatac tgacctggcg
tcaggatggg gtatctttga gccacgacac ccagcagtgg ggggatgtcc tgcctgatgg gaatggaacc
taccagacct gggtggccac caggatttgc cgaggagagg agcagaggtt cacctgctac atggaacaca
gcgggaatca cagcactcac cctgtgccct ct

The MICA sequence, in one embodiment, comprises a mutant of the wide-type. In one embodiment, the MICA mutant is a sequence variant of MUC-30, which comprises a methionine mutation instead of alanine at position 129 of the wide-type MICA sequence. The amino acid and cDNA sequences of MUC-30 variant are shown in SEQ ID NO. 7 and SEQ ID NO. 8, respectively.
**SEQ ID NO. 7 amino acid sequence of MUC-30** 
EPHSLRYNLTVLSWDGSVQSGFLAEVHLDGQPFLRCDRQKCRAKPQGQWAEDVLGNKTWDRETRDLTGNGKDLRMTL
AHIKDQKEGLHSLQEIRVCEIHEDNSTRSSQHFYYDGELFLSQNLETEEWTMPQSSRAQTLAMNIRNFLKEDAMKTK
THYHAMHADCLQELRRYLKSGVVLRRTVPPMVNVTRSEASEGNITVTCRASGFYPWNITLSWRQDGVSLSHDTQQWG
DVLPDGNGTYQTWVATRICQGEEQRFTCYMEHSGNHSTHPVPS
SEQ ID NO. 8 cDNA sequence of MUC-30

gagccccaca gtcttcgtta taacctcacg gtgctgtcct gggatggatc tgtgcagtca
gggtttctcg ctgaggtaca tctggatggt cagcccttcc tgcgctgtga caggcagaaa
tgcagggcaa agccccaggg acagtgggca gaagatgtcc tgggaaataa gacatgggac
agagagacca gggacttgac agggaacgga aaggacctca ggatgaccct ggctcatatc
aaggaccaga aagaaggctt gcattccctc caggagatta gggtctgtga gatccatgaa
gacaacagca ccaggagctc ccagcatttc tactacgatg gggagctctt cctctcccaa
aacctggaga ctgaggaatg gacaatgccc cagtcctcca gagctcagac cttggccatg
aacatcagga atttcttgaa ggaagatgcc atgaagacca agacacacta tcacgctatg
catgcagact gcctgcagga actacggcga tatctaaaat ccggcgtagt cctgaggaga

acagtgcccc ccatggtgaa tgtcacccgc agcgaggcct cagagggcaa cattaccgtg
acatgcaggg cttctggctt ctatccctgg aatatcacac tgagctggcg tcaggatggg
gtatctttga gccacgacac ccagcagtgg ggggatgtcc tgcctgatgg gaatggaacc
taccagacct gggtggccac caggatttgc caaggagagg agcagaggtt cacctgctac
atggaacaca gcgggaatca cagcactcac cctgtgccct ct

In a further aspect, expresses a pre-mRNA that encodes a trispecific antibody, when the pre-mRNA is in contact with the morpholino oligonucleotide.

In one aspect, the antigen-binding domain is a single-chain variable fragment or an antibody.

In a further aspect, the recombinant polynucleotide or vector comprises a polynucleotide sequence encoding a secretory peptide. In another aspect, the recombinant polynucleotide or vector further comprises a polynucleotide sequence encoding a dimerization domain. In another aspect, the recombinant polynucleotide or vector comprises a 5' inverted terminal repeat (ITR) and a 3' ITR. In another aspect, the vector comprises a sequence of SEQ ID Nos. 4, 6, 8, 12, 14, 16-23, 30-33, or 40-46. Non-limiting examples of such vector include: a recombinant viral vector that comprises a backbone vector selected from the group of a retroviral vector, a lentiviral vector, a murine leukemia viral ("MLV") vector, an Epstein-Barr viral ("EBV") vector, an adenoviral vector, a herpes viral ("HSV") vector, an Adeno-associated viral ("AAV") vector, an AAV vector, or optionally a self-complementary AAV vector.

The recombinant polynucleotide or vector can be contained within host cells, e.g., prokaryotic or eukaryotic cells.

The recombinant polynucleotide, vectors and cells can be contained within a composition that contains the vector and/or host cell and a carrier, e.g., a pharmaceutically acceptable carrier. They can be formulated for various modes of administration and contain an effective amount of the polynucleotide, vector and/or host cell that is effective for the patient, the disorder or disease, the vector and mode of administration. In one aspect, the mode of administration is systemic or intravenous. In another aspect, administration is local by direct injection. In one aspect, the morpholino oligonucleotide is contacted as the same time or subsequent to the polynucleotide or vector, e.g., as a systemic or local injection. Alternatively, the contacting is prior to the polynucleotide or vector.

The recombinant polynucleotides or vectors are useful to treat a variety of diseases or disorder. In one aspect, a method for delivering a transgene is provided. The method comprises administering an effective amount of the recombinant polynucleotide or vector comprising the transgene to the cell, tissue, or patient to be treated. In one aspect, an effective amount of a morpholino oligonucleotide is administered to the cell, tissue, or patient to be treated. Non-limiting examples of transgenes are provided and are selected based on the purpose of the method. The cells or tissue can be mammalian, e.g., human. In one aspect, the morpholino oligonucleotide is contacted as the same time or subsequent to the vector. Alternatively, it is prior to the vector. In another aspect, the vector is introduced to the cell by transfection, infection, transformation, electroporation, injection, microinjection, or the combination thereof.

A method of treating a cancer in a subject in need thereof is provided. The method, comprises, or alternatively consists essentially of, or yet further consists of, administering to the subject an effective amount of the recombinant polynucleotide or vector or cell as described herein. In a further the method further comprises administering to the subject an effective amount of a morpholino oligonucleotide. In one aspect, an effective amount of an anti-cancer agent is administered to the subject. Non-limiting examples of anti-cancer agents include anti-cancer peptides, polypeptides, nucleic acid molecules, small molecules, viral particles, or combinations thereof. In another aspect, the vector is introduced to the cell by transfection, infection, transformation, electroporation, injection, microinjection, or the combination thereof.

In one aspect of the disclosed methods, the viral particle is an oncolytic HSV particle.

An additional method provided by this disclosure is a method of producing a bispecific antibody or a trispecific antibody in a cell, comprising, or consisting essentially of, or yet further consisting of, contacting the cell comprising a polynucleotide or a vector as described herein, with an effective amount of a morpholino oligonucleotide. In one aspect, the morpholino oligonucleotide is contacted as the same time or subsequent to the vector. Alternatively, it is prior to the polynucleotide or vector. In one aspect, the morpholino oligonucleotide comprise a sequence at least 95% identical with SEQ ID NO. 27 or 28. A non-limiting example of the bispecific antibody comprises a polypeptide sequence at least 95% identical to SEQ ID NOs. 13 or 15. In one embodiment, the bispecific antibody is encoded by a polynucleotide sequence at least 95% identical to SEQ ID Nos. 14, 16, 22, 23, 30-33, or 40-46. In another aspect, trispecific antibody comprises a polypeptide sequence at least 95% identical to SEQ ID NO. 11. In one embodiment, the trispecific antibody is encoded by a polynucleotide sequence at least 95% identical to SEQ ID NO. 12.

In another aspect, the polynucleotide or vector is introduced to the cell by transfection, infection, transformation, electroporation, injection, microinjection, or the combination thereof. Non-limiting examples of cells include a fibroblast, a skeletal cell, an epithelial cell, a muscle cell, a neural cell, an endocrine cell, a melanocyte, a blood cell, or combination thereof.

Further provided are kits comprising one or more of the polynucleotide, the vector, the cell or a composition as described herein, optionally with instructional material.

### BRIEF DESCRIPTION OF THE DRAWINGS

**FIG. 1** shows a strategy overview of the general concept of splicing using a 3-exon gene structure. Exons are labeled 1, 2, or 3, and introns are represented by a straight line. Different splicing patterns are shown with lower case letters (a, b, c) and the structures of the resulting RNAs are shown based on which of those are utilized. Oligos that can interfere with splice donor (1D, 2D) or acceptor (2A, 3A) sites are shown as short blue lines, and the expected expression of different possible RNA isoforms are shown as "+," depending on which sites are blocked by an oligo(s).
**FIG. 2** shows an overview of strategy of **FIG. 1****,** using splice type 1 in which exon 2 is normally included in the gene transcript. The transcript that includes all 3 exons spliced together is expected to be predominant, but in the presence of oligos that block exon 2 splice acceptor or donor sites, the transcript with exon 1 fused to exon 3 will predominate.
**FIG. 3** illustrates an overview of strategy #1 to create an engineered intron-exonSTOP-intron within a transgene. Those transcripts that retain the introns will be nonfunctional because the introns will have premature stop codons and/or be out of frame. The "a+b" transcript will be nonfunctional because of the stop codons in exon 2. Only the transcript in which exon 2 is skipped (utilizing splice c) is functional, which will be low at baseline and activated by oligos that block the splice sites on exon 2.
**FIG. 4** shows a map of the transgene regulation of the strategy shown in **FIG. 1****.**
**FIG. 5** illustrates a second strategy to create an engineered intron-exonSTOP-intron within a transgene utilizing a "type 2 splice" in which exon 2 is normally excluded in cancer cells but included in normal cells. In this scenario, the exon skipping oligos activate transgene expression (exon 1+3) in normal cells, leaving it unchanged (or perhaps even higher) in certain off-target skipping of a cellular gene as potentially a "bonus" therapeutic effect.
**FIG. 6** illustrates a third strategy to create an engineered intron-exonSTOP-intron within a transgene utilizing a "type 3 splice" in which exon 2 is normally included in certain cancer cells but excluded in normal cells. In this scenario, there is baseline high levels of the active transgene (exon 1+3) in normal cells, and exon-skipping oligos activate expression in tumor cells.
**FIG. 7** illustrates exemplary CD3xGD2-HDD AAV constructs.
**FIG. 8** illustrates a cartoon representation of assays to determine the structure and function of Dimerts disclosed herein.
**FIG. 9** illustrates SDS-PAGE of whole cell lysates from transfected 293T cells. The three constructs of FIG. 7, #1101, #1040, and #1124, which comprises a secretion peptide showed less CD3xGD2-HDD Dimert retained in the transfected 293T cells relative to construct #1104 and the control pcDNA3-GFP construct.
**FIG. 10** illustrates AAV vector secreted CD3xGD2-HDD Dimert which binds and activates human T cells.
**FIG. 11** illustrates secreted CD3xGD2-HDD Dimert which activates human T cells.
**FIG. 12A** illustrates binding of CD3xGD2-HDD to T cells is dose-dependent.
**FIG. 12B** illustrates a bar graph of median staining levels normalized to unstained controls.
**FIG. 13** illustrates a cartoon representation of a binding assay for the anti-GD2 arm of a CD3xGD2-HDD Dimert. This assay confirmed that both GD2 and CD3 bind resides on a single molecule.
**FIG. 14** illustrates an exemplary CD3xGD2-HDD Dimert which binds to both CD3 and GD2.
**FIG. 15** illustrates an assay flow-chart to determine if CD3xGD2-HDD induces GD2+ target cell killing by T cells.
**FIG. 16** illustrates secreted CD3xGD2-HDD induces T cell killing of neuroblastoma cells.
**FIG. 17** illustrates T cell mediated cytotoxicity by CD3xGD2-HDD Dimert which is related to GD2 expression.
**FIG. 18A** illustrates maps of exemplary AAV constructs for testing CD19xCD3 Dimert expression.
**FIG. 18B** illustrates an exemplary CD19 TransJoin genomic structure. The elements of the AAV encoded transgene are shown in the figure.
**FIG. 18C** illustrates a cartoon representation of a CD19 dimert interacting with a cancer cell and a T cell. The CD19 dimert is produced by a cell comprising a CD19 TransJoin. As shown in the figure, "Ca" represents cancer cell, and "T" represents a T cell.
**FIG. 19** illustrates supernatants from cells transfected with AAV CD19xCD3 construct comprising a secretion sequence binds and activate T cells.
**FIG. 20** illustrates supernatants from cells transfected with AAV vectors comprising a secretion peptide activate human T cells.
**FIG. 21** illustrates AAV secreted CD19xCD3 specifically binds CD19 but not CD45.
**FIG. 22A** illustrates binding of CD19xCD3 to T cells is dose-dependent.
**FIG. 22B** illustrates bar graph of median staining levels normalized to unstained controls.
**FIG. 23A** illustrates binding of CD19xCD3 to B cells is dose-dependent.
**FIG. 23B** illustrates bar graph of median staining levels normalized to unstained controls.
**FIG. 24** illustrates CD3 Dimerts activate T cells with anti-CD28 co-stimulation better than anti-CD3 antibodies.
**FIG. 25** illustrates 293T cells yield highest transduction efficiency for AAV8 vector.
**FIG. 26** illustrates Dimert concentration in supernatants of AAV8-infected cells is depending on AAV dose and transduction efficiency.
**FIG. 27** illustrates a single intravenous injection of CD19xCD3 TransJoin which selectively eliminates B cells in humanized mice.
**FIG. 28** illustrates a single intravenous injection of CD19xCD3 TransJoin which results in prolonged B cell depletion in humanized mice.
**FIG. 29** illustrates a single intravenous injection of CD19xCD3 TransJoin eliminates CD19+ lymphoma in humanized mice.
**FIG. 30** illustrates an overview of OncoSkip and TransSkip described herein.
**FIG. 31** illustrates KRAS OncoSkip antisense morpholinos induce exon skipping of endogenous KRAS in lung cancer cells.
**FIG. 32** illustrates exemplary AAV vector maps of CD3xGD2-HDD TransSkip showing reverse engineered introns flanking an exon inserted into the CD3xGD2-HDD Dimert coding sequence.
**FIG. 33** illustrates an exemplary strategy for testing activity of OncoSkip and TransSkip described herein.
**FIG. 34** illustrates antisense morpholinos which induce exon skipping of the CD3xGD2-HDD TransSkip transgene.
**FIG. 35** illustrates KRAS OncoSkip which induces secreted expression of CD3xGD2-HDD Dimert in cells transfected with the CD3xGD2-HDD TransSkip AAV vector.
**FIG. 36** illustrates exon skipping of CD3xGD2-HDD TransSkip by KRAS OncoSkip is an on-target effect.
**FIG. 37** illustrates induction of CD3xGD2-HDD Dimert expression as determined by T cell binding is an on-target effect.
**FIG. 38** illustrates AAV genome maps of exemplary TransSkip splice variants to decrease baseline TransSkip "leak" but maintain inducible exon-skipping.
**FIG. 39** illustrates CD3xGD2-HDD TransSkip splice variant K3 which eliminates baseline yet maintains inducible exon skipping.
**FIG. 40** illustrates CD3xGD2-HDD TransSkip variant K3 which shows no baseline Dimert production but is more indicible by KRAS OncoSkip relative to other TransSkip variants tested.
**FIG. 41** illustrates OncoSkip-mediated induction of Dimert expression from CD3xGD2-HDD TransSkip variants K3 and K5 is an on-target effect.
**FIG. 42** illustrates secreted Dimert induced by OncSkip from AAV CD3xGD2-HDD TransSkip vectors is functional in mediating T cell killing of neuroblastoma cells.
**FIG. 43** illustrates transgene exon-skipping in cells infected with AV CD3xGD2-HDD TransSkip variant K3 is inducible on-target by KRAS OncoSkip.
**FIG. 44A** illustrates AAV genome maps of exemplary CD19xCD3 TransSkip splice variants.
**FIG. 44B** illustrates an exemplary CD19 TransSkip genomic structure. The elements of the AAV encoded transgene are shown in the figure.
**FIG. 44C** illustrates a cartoon representation of a CD19 dimert interacting with a cancer cell and a T cell. The CD19 dimert is produced by a cell comprising a CD19 TransSkip. As shown in the figure, "Ca" represents cancer cell, and "T" represents a T cell.
**FIG. 45** illustrates OncoSkip morpholinos KTS1 and KTS2 induce on-target exon skipping of CD19xCD3 TransSkip K1.
**FIG. 46** illustrates KRAS OncoSkip induces secreted expression of CD19xCD3 Dimert in cells transfected with the CD19xCD3 TransSkip K1 AAV vector.
**FIG. 47** illustrates CD19xCD3 TransSkip splice variant K3 eliminates baseline yet maintains inducible exon skipping.
**FIG. 48** illustrates induction of CD19xCD3 Dimert expression from CD19xCD3 TransSkip K3 as determined by T cell binding is an on-target effect.
**FIG. 49** illustrates induction of CD19xCD3 Dimert expression from CD19xCD3 TransSkip K3 is repeatable.

### DETAILED DESCRIPTION

Embodiments according to the present disclosure will be described more fully hereinafter. Aspects of the disclosure may, however, be embodied in different forms and should not be construed as limited to the embodiments set forth herein. Rather, these embodiments are provided so that this disclosure will be thorough and complete, and will fully convey the scope of the invention to those skilled in the art. The terminology used in the description herein is for the purpose of describing particular embodiments only and is not intended to be limiting.

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the present application and relevant art and should not be interpreted in an idealized or overly formal sense unless expressly so defined herein. While not explicitly defined below, such terms should be interpreted according to their common meaning.

The terminology used in the description herein is for the purpose of describing particular embodiments only and is not intended to be limiting of the invention. All publications, patent applications, patents, and other references mentioned herein are incorporated by reference in their entirety.

The practice of the present technology will employ, unless otherwise indicated, conventional techniques of tissue culture, immunology, molecular biology, microbiology, cell biology, and recombinant DNA, which are within the skill of the art.

Unless the context indicates otherwise, it is specifically intended that the various features of the invention described herein can be used in any combination. Moreover, the disclosure also contemplates that in some embodiments, any feature or combination of features set forth herein can be excluded or omitted. To illustrate, if the specification states that a complex comprises components A, B and C, it is specifically intended that any of A, B or C, or a combination thereof, can be omitted and disclaimed singularly or in any combination.

Unless explicitly indicated otherwise, all specified embodiments, features, and terms intend to include both the recited embodiment, feature, or term and biological equivalents thereof.

All numerical designations, e.g., pH, temperature, time, concentration, and molecular weight, including ranges, are approximations which are varied (+) or (-) by increments of 1.0 or 0.1, as appropriate, or alternatively by a variation of +/- 15 %, or alternatively 10%, or alternatively 5%, or alternatively 2%. It is to be understood, although not always explicitly stated, that all numerical designations are preceded by the term "about." It also is to be understood, although not always explicitly stated, that the reagents described herein are merely exemplary and that equivalents of such are known in the art.

Throughout this disclosure, various publications, patents, and published patent specifications are referenced by an identifying citation or by an Arabic numeral. The full citation for the publications identified by an Arabic numeral are found immediately preceding the claims. The disclosures of these publications, patents, and published patent specifications are hereby incorporated by reference into the present disclosure in their entirety to more fully describe the state of the art to which this invention pertains.

### Definitions

The practice of the present technology will employ, unless otherwise indicated, conventional techniques of organic chemistry, pharmacology, immunology, molecular biology, microbiology, cell biology and recombinant DNA, which are within the skill of the art. See, e.g., Sambrook, Fritsch and Maniatis, Molecular Cloning: A Laboratory Manual, 2nd edition (1989); Current Protocols In Molecular Biology (F. M. Ausubel, et al. eds., (1987)); the series Methods in Enzymology (Academic Press, Inc.): PCR 2: A Practical Approach (M.J. MacPherson, B.D. Hames and G.R. Taylor eds. (1995)), Harlow and Lane, eds. (1988) Antibodies, a Laboratory Manual, and Animal Cell Culture (R.I. Freshney, ed. (1987)).

As used in the description of the invention and the appended claims, the singular forms "a," "an," and "the" are intended to include the plural forms as well, unless the context clearly indicates otherwise.

As used herein, the term "comprising" is intended to mean that the compositions and methods include the recited elements, but do not exclude others. As used herein, the transitional phrase consisting essentially of (and grammatical variants) is to be interpreted as encompassing the recited materials or steps and those that do not materially affect the basic and novel characteristic(s) of the recited embodiment. Thus, the term "consisting essentially of" as used herein should not be interpreted as equivalent to "comprising." "Consisting of" shall mean excluding more than trace elements of other ingredients and substantial method steps for administering the compositions disclosed herein. Aspects defined by each of these transition terms are within the scope of the present disclosure.

The term "about," as used herein when referring to a measurable value such as an amount or concentration and the like, is meant to encompass variations of 20%, 10%, 5%, 1%, 0.5%, or even 0.1 % of the specified amount.

The terms or "acceptable," "effective," or "sufficient" when used to describe the selection of any components, ranges, dose forms, etc. disclosed herein intend that said component, range, dose form, etc. is suitable for the disclosed purpose.

Also as used herein, "and/or" refers to and encompasses any and all possible combinations of one or more of the associated listed items, as well as the lack of combinations when interpreted in the alternative ("or").

The term "Adeno-associated virus" or "AAV" as used herein refers to a member of the class of viruses associated with this name and belonging to the genus Dependoparvovirus, family Parvoviridae. Multiple serotypes of this virus are known to be suitable for gene delivery; all known serotypes can infect cells from various tissue types. At least 11 sequentially numbered, AAV serotypes are known in the art. Non-limiting exemplary serotypes useful in the methods disclosed herein include any of the 11 serotypes, e.g., AAV2, AAV8, AAV9, or variant serotypes, e.g., AAV-DJ and AAV PHP.B. The AAV particle comprises three major viral proteins: VP1, VP2, and VP3. In one embodiment, the AAV refers to of the serotype AAV1, AAV2, AAV4, AAV5, AAV6, AAV7, AAV8, AAV9, AAV10, AAV11, AAV12, AAV13, AAV PHP.B, AAV rh74, or AAV-DJ (a chimera obtained by shuffling of eight different AAV wild-types).

In some instances, a serotype of AAV preferentially targets a tissue type. In some cases, the following chart illustrates exemplary tissue types and AAV serotypes that target each tissue type.

| **Tissue** | **Serotype** |
|---|---|
| CNS | AAV1, AAV2, AAV4, AAV5, AAV8, AAV9 |
| Heart | AAV1, AAV8, AAV9 |
| Kidney | AAV2 |
| Liver | AAV7, AAV8, AAV9 |
| Lung | AAV4, AAV5, AAV6, AAV9 |
| Pancreas | AAV8 |
| Photoreceptor Cells | AAV2, AAV5, AAV8 |
| RPE (Retinal Pigment Epithelium) | AAV1, AAV2, AAV4, AAV5, AAV8 |
| Skeletal Muscle | AAV1, AAV6, AAV7, AAV8, AAV9 |

The term "cell" as used herein may refer to either a prokaryotic or eukaryotic cell, optionally obtained from a subject or a commercially available source.

"Eukaryotic cells" comprise all of the life kingdoms except monera. They can be easily distinguished through a membrane-bound nucleus. Animals, plants, fungi, and protists are eukaryotes or organisms whose cells are organized into complex structures by internal membranes and a cytoskeleton. The most characteristic membrane-bound structure is the nucleus. Unless specifically recited, the term "host" includes a eukaryotic host, including, for example, yeast, higher plant, insect, and mammalian cells. Non-limiting examples of eukaryotic cells or hosts include simian, bovine, porcine, murine, rat, avian, reptilian, and human, e.g., HEK293 cells and 293T cells.

"Prokaryotic cells" that usually lack a nucleus or any other membrane-bound organelles and are divided into two domains, bacteria, and archaea. In addition to chromosomal DNA, these cells can also contain genetic information in a circular loop called on episome. Bacterial cells are very small, roughly the size of an animal mitochondrion (about 1-2 µm in diameter and 10 µm long). Prokaryotic cells feature three major shapes: rod shaped, spherical, and spiral. Instead of going through elaborate replication processes like eukaryotes, bacterial cells divide by binary fission. Examples include but are not limited to Bacillus bacteria, E. coli bacterium, and Salmonella bacterium.

The term "encode" as it is applied to nucleic acid sequences refers to a polynucleotide which is said to "encode" a polypeptide if, in its native state or when manipulated by methods well known to those skilled in the art, can be transcribed and/or translated to produce the mRNA for the polypeptide and/or a fragment thereof. The antisense strand is the complement of such a nucleic acid, and the encoding sequence can be deduced therefrom.

The terms "equivalent" or "biological equivalent" are used interchangeably when referring to a particular molecule, biological, or cellular material and intend those having minimal homology while still maintaining desired structure or functionality. Non-limiting examples of equivalent polypeptides, include a polypeptide having at least 60%, or alternatively at least 65%, or alternatively at least 70%, or alternatively at least 75%, or alternatively 80%, or alternatively at least 85%, or alternatively at least 90%, or alternatively at least 95% identity or alternatively at least 96% identity, or alternatively at least 97% identity, or alternatively at least 98% identity, or alternatively at least 99% identity for polypeptide sequences, or a polypeptide which is encoded by a polynucleotide or its complement that hybridizes under conditions of high stringency to a polynucleotide encoding such polypeptide sequences that has substantially identical or identical function as the reference polypeptide and in one aspect, encodes the reference polypeptide. Conditions of high stringency are described herein and incorporated herein by reference. Alternatively, an equivalent thereof is a polypeptide encoded by a polynucleotide or a complement thereto, having at least 70%, or alternatively at least 75%, or alternatively 80%, or alternatively at least 85%, or alternatively at least 90%, or alternatively at least 95% identity, or at least 96% identity, or at least 97% sequence identity, or alternatively at least 98% identity, or alternatively at least 99% identity to the reference polynucleotide, e.g., the wild-type polynucleotide or referenced polynucleotide.

Non-limiting examples of equivalent polynucleotides, include a polynucleotide having at least 60%, or alternatively at least 65%, or alternatively at least 70%, or alternatively at least 75%, or alternatively 80%, or alternatively at least 85%, or alternatively at least 90%, or alternatively at least 95%, or at least 96% identity, or at least 97% sequence identity, or alternatively at least 98% identity, or alternatively at least 99% identity to a reference polynucleotide. An equivalent also intends a polynucleotide or its complement that hybridizes under conditions of high stringency to a reference polynucleotide.

A polynucleotide or polynucleotide region (or a polypeptide or polypeptide region) having a certain percentage (for example, 80%, 85%, 90%, or 95%) of "sequence identity" to another sequence means that, when aligned, that percentage of bases (or amino acids) are the same in comparing the two sequences. The alignment and the percent homology or sequence identity can be determined using software programs known in the art, for example those described in Current Protocols in Molecular Biology (Ausubel et al., eds. 1987) Supplement 30, section 7.7.18, Table 7.7.1. In certain embodiments, default parameters are used for alignment. A non-limiting exemplary alignment program is BLAST, using default parameters. In particular, exemplary programs include BLASTN and BLASTP, using the following default parameters: Genetic code=standard; filter-none; strand=both; cutoff=60; expect=10; Matrix=BLOSUM62; Descriptions=50 sequences; sort by=HIGH SCORE; Databases=non-redundant, GenBank+EMBL+DDBJ+PDB+GenBank CDS translations+SwissProtein+SPupdate+PIR. Details of these programs can be found at the following Internet address: ncbi.nlm.nih.gov/cgi-bin/BLAST. Sequence identity and percent identity can be determined by incorporating them into clustalW (available at the web address: genome.jp/tools/clustalw/, last accessed on Jan. 13, 2017).

"Homology" or "identity" or "similarity" refers to sequence similarity between two peptides or between two nucleic acid molecules. Homology can be determined by comparing a position in each sequence that may be aligned for purposes of comparison. When a position in the compared sequence is occupied by the same base or amino acid, then the molecules are homologous at that position. A degree of homology between sequences is a function of the number of matching or homologous positions shared by the sequences. An "unrelated" or "non-homologous" sequence shares less than 40% identity, or alternatively less than 25% identity, with one of the sequences of the present disclosure.

"Homology" or "identity" or "similarity" can also refer to two nucleic acid molecules that hybridize under stringent conditions.

"Hybridization" refers to a reaction in which one or more polynucleotides react to form a complex that is stabilized via hydrogen bonding between the bases of the nucleotide residues. The hydrogen bonding may occur by Watson-Crick base pairing, Hoogstein binding, or in any other sequence-specific manner. The complex may comprise two strands forming a duplex structure, three or more strands forming a multi-stranded complex, a single self-hybridizing strand, or any combination of these. A hybridization reaction may constitute a step in a more extensive process, such as the initiation of a PCR reaction, or the enzymatic cleavage of a polynucleotide by a ribozyme.

Examples of stringent hybridization conditions include: incubation temperatures of about 25° C. to about 37° C.; hybridization buffer concentrations of about 6× saline sodium citrate (SSC) to about 10×SSC; formamide concentrations of about 0% to about 25%; and wash solutions from about 4×SSC to about 8×SSC. Examples of moderate hybridization conditions include: incubation temperatures of about 40° C. to about 50° C.; buffer concentrations of about 9×SSC to about 2×SSC; formamide concentrations of about 30% to about 50%; and wash solutions of about 5×SSC to about 2×SSC. A high stringency hybridization refers to a condition in which hybridization of an oligonucleotide to a target sequence comprises no mismatches (or perfect complementarity). Examples of high stringency conditions include: incubation temperatures of about 55° C. to about 68° C.; buffer concentrations of about 1×SSC to about 0.1 ×SSC; formamide concentrations of about 55% to about 75%; and wash solutions of about 1×SSC, 0.1×SSC, or deionized water. In general, hybridization incubation times are from 5 minutes to 24 hours, with 1, 2, or more washing steps, and wash incubation times are about 1, 2, or 15 minutes. SSC is 0.15 M NaCl and 15 mM citrate buffer. It is understood that equivalents of SSC using other buffer systems can be employed.

As used herein, "expression" refers to the process by which polynucleotides are transcribed into mRNA and/or the process by which the transcribed mRNA is subsequently being translated into peptides, polypeptides, or proteins. If the polynucleotide is derived from genomic DNA, expression may include splicing of the mRNA in a eukaryotic cell.

A "gene" refers to a polynucleotide containing at least one open reading frame (ORF) that is capable of encoding a particular polypeptide or protein after being transcribed and translated. A "gene product" or alternatively a "gene expression product" refers to the amino acid (e.g., peptide or polypeptide) generated when a gene is transcribed and translated.

"Under transcriptional control" is a term well understood in the art and indicates that transcription of a polynucleotide sequence, usually a DNA sequence, depends on its being operatively linked to an element which contributes to the initiation of, or promotes, transcription. "Operatively linked" intends the polynucleotides are arranged in a manner that allows them to function in a cell. In one aspect, this invention provides promoters operatively linked to the downstream sequences.

The term "exon" refers to a nucleic acid sequence that comprises a protein-coding sequence. The gene typically includes more than one exons, which are separated by an intron in between.

The term "intron" as used here refers to a nucleic acid sequence is flanked by a splice donor site on the 5' end and a splice acceptor site on the 3' end. In some embodiments, the intron is spliced out of or removed from an RNA or mRNA sequence expressed from the vector in which it is present.

The term "splice donor site" is a nucleic acid sequence or domain on the 5' end of an intron. The splice donor site, in one embodiment, marks the start of the intron and/or the intron's boundary with an immediately preceding coding sequence (or exon).

The term "splice acceptor site" as used herein refers to a nucleic acid sequence or domain on the 3' end of an intron. In one embodiment, the splice acceptor site marks the start of the intron and its boundary with the following coding sequence-exon. In another embodiment, the splice acceptor site comprises an intron branch point is the point to which the 5' end of the intron becomes joined during the process of splicing. In some embodiments, the splice acceptor sequence and the intron branch site are placed adjacent to each other as a single unite. In some embodiments, the splice acceptor sequence and the intron branch site may be further separated, by moving the branch site further 5' of the splice acceptor sequence.

The term "splice site" as used herein refers to a sequence or domain of a nucleic acid present at either the 5' end or the 3' end of an intron as defined above.

The term "exon skipping" as used herein refers to the modification of pre-mRNA splicing by the targeting of splice donor and/or acceptor sites within a pre-mRNA with one or more complementary antisense oligonucleotides. By blocking access of a spliceosome to one or more splice donor or acceptor site, the one or more complementary antisense oligonucleotides can prevent a splicing reaction, thereby causing the deletion of one or more exons from a fully-processed mRNA. In one embodiment, exon skipping is achieved in the nucleus during the maturation process of pre-mRNAs. It includes the masking of key sequences involved in the splicing of targeted exons by using antisense oligonucleotides that are complementary to splice donor sequences within a pre-mRNA.

The term "gene regulation sequence" as used herein refers to a nucleic acid sequence capable of controlling the transcription, splicing, or modification of a gene, an open reading frame, or an exon or intron. A gene regulation sequence of the invention may include a promoter, a binding site for an antisense oligonucleotide, and/or an enhancer. Therefore, placing a gene under the regulatory control of a promoter or a regulatory element means positioning the gene such that the expression of the gene is controlled by the regulatory sequence(s). Thus, in the construction of promoter-gene combinations, the promoter is preferably positioned upstream of the gene and at a distance from the transcription start site that approximates the distance between the promoter and the gene it controls in the natural setting. The variation in this distance can be tolerated without loss of promoter function. Similarly, the preferred positioning of a regulatory element, such as an enhancer, with respect to a heterologous gene placed under its control reflects its natural position relative to the structural gene it naturally regulates. Enhancers are believed to be relatively position and orientation independent in contrast to promoter elements. In some embodiments, the gene regulation sequence comprises one or more of a binding sequence for an antisense oligonucleotide, a binding sequence for doxycycline, or a polynucleotide sequence encoding a riboswitch. In some embodiments, the antisense oligonucleotide (ASO) comprises one or more modified nucleotides. In one embodiment, the antisense oligonucleotide is a morpholino oligonucleotide.

In some embodiments, an antisense oligonucleotide (ASO) described herein comprises from about 8 to about 50 nucleotides in length. In some instances, the ASO comprises from about 8 to about 30, from about 8 to about 25, from about 8 to about 20, from about 8 to about 18, from about 8 to about 15, from about 10 to about 50, from about 10 to about 30, from about 10 to about 25, from about 10 to about 20, from about 10 to about 18, from about 10 to about 15, from about 12 to about 50, from about 12 to about 30, from about 12 to about 25, from about 12 to about 20, from about 12 to about 18, or from about 12 to about 15 nucleotides in length. In some embodiments, the ASO comprises 8, 9, 10, 11, 12, 13, 14, 15, 16, 17, 18, 19, 20, 21, 22, 23, 24, 25, 30, 40, or 50 nucleotides in length.

In some instances, the ASO comprises one or more modified nucleotides. In some instances, the ASO comprises about 5, 10, 20, 30, 40, 50, 60, 70, 80, 90, or 100% modified nucleotides. In other instances, the ASO comprises about 1, 2, 3, 4, 5, 6, 7, 8, 9, 10, 11, 12, 13, 14, 15, 20, 25, 30, 35, 40, or more modified nucleotides. In some instances, the modification is at a 2' hydroxyl group of the ribose moiety. In some instances, the modification includes an H, OR, R, halo, SH, SR, NH2, NHR, NR2, or CN, wherein R is an alkyl moiety. Exemplary alkyl moiety includes, but is not limited to, halogens, sulfurs, thiols, thioethers, thioesters, amines (primary, secondary, or tertiary), amides, ethers, esters, alcohols and oxygen. In some instances, the alkyl moiety further comprises a modification. In some instances, the modification comprises an azo group, a keto group, an aldehyde group, a carboxyl group, a nitro group, a nitroso, group, a nitrile group, a heterocycle (e.g., imidazole, hydrazino or hydroxylamino) group, an isocyanate or cyanate group, or a sulfur containing group (e.g., sulfoxide, sulfone, sulfide, and disulfide). In some instances, the alkyl moiety further comprises a hetero substitution. In some instances, the carbon of the heterocyclic group is substituted by a nitrogen, oxygen or sulfur. In some instances, the heterocyclic substitution includes but is not limited to, morpholino, imidazole, and pyrrolidino. In some instances, the modification at the 2' hydroxyl group is a 2'-O-methyl modification or a 2'-O-methoxyethyl (2'-O-MOE) modification. In some instances, the modified nucleotide is a locked or bridged ribose modification (e.g., locked nucleic acid or LNA), an ethylene nucleic acid (ENA) (e.g., a 2'-4'-ethylene-bridged nucleic acid), a peptide nucleic acid, or a morpholino. In some instances, the modified nucleotide further comprises one or more modified internucleotide linkages. Exemplary modified internucleotide linkage includes, but is not limited to, phosphorothioates, phosphorodithioates, methylphosphonates, 5'-alkylenephosphonates, 5'-methylphosphonate, 3'-alkylene phosphonates, borontrifluoridates, borano phosphate esters and selenophosphates of 3'-5'linkage or 2'-5'linkage, phosphotriesters, thionoalkylphosphotriesters, hydrogen phosphonate linkages, alkyl phosphonates, alkylphosphonothioates, arylphosphonothioates, phosphoroselenoates, phosphorodiselenoates, phosphinates, phosphoramidates, 3'-alkylphosphoramidates, aminoalkylphosphoramidates, thionophosphoramidates, phosphoropiperazidates, phosphoroanilothioates, phosphoroanilidates, ketones, sulfones, sulfonamides, carbonates, carbamates, methylenehydrazos, methylenedimethylhydrazos, formacetals, thioformacetals, oximes, methyleneiminos, methylenemethyliminos, thioamidates, linkages with riboacetyl groups, aminoethyl glycine, silyl or siloxane linkages, alkyl or cycloalkyl linkages with or without heteroatoms of, for example, 1 to 10 carbons that are saturated or unsaturated and/or substituted and/or contain heteroatoms, linkages with morpholino structures, amides, polyamides wherein the bases are attached to the aza nitrogens of the backbone directly or indirectly, and combinations thereof.

The term "morpholino" as used herein refers to a polymeric molecule having a backbone which supports bases capable of forming a hydrogen bond with a polynucleotide. In some embodiments, the polymer on the morpholino lacks a pentose sugar backbone moiety, and more specifically a ribose backbone linked by phosphodiester bonds which is typical of nucleotides and nucleosides. In one embodiment, the morpholino oligonucleotide contains a nitrogen ring. In another embodiment, the morpholino is a stereopure oligonucleotide (e.g., see wavelifesciences.com, last accessed on January 25, 2019) or its derivatives. In another embodiment, the morpholino comprise a sequence at least 95% identical with a stereopure polynucleotide.

In another embodiment, morpholino comprises a structure from about 8 to about 50, from about 8 to about 30, from about 10 to about 50, from about 10 to about 30, or from about 12 to about 30 nucleotides, including a targeting base sequence that is complementary to a target region of a selected preprocessed mRNA or pre-mRNA, such as the intron region of a pre-mRNA. In another embodiment, the morpholino antisense oligonucleotide promotes splicing a target exon, which results in a transcript that lacks the target exon.

In some instances, an antisense oligonucleotide (ASO) is referred to herein as an OncoSkip. As used herein, the term "OncoSkip" refers to an ASO designed to induce skipping of a target exon during splicing of a target transgene, thereby inducing expression of the target transgene. In some instances, the target transgene encodes a polypeptide that binds to a surface polypeptide (e.g., a surface receptor) of a target cell. In some instances, the target cell is a tumor cell or an immune cell. In some instances, the target transgene is an oncogene. In such instances, the use of the OncoSkip induces skipping of a target exon during splicing to induce expression of the oncogene.

In some embodiments, an OncoSkip described herein comprises from about 8 to about 50 nucleotides in length. In some instances, the OncoSkip comprises from about 8 to about 30, from about 8 to about 25, from about 8 to about 20, from about 8 to about 18, from about 8 to about 15, from about 10 to about 50, from about 10 to about 30, from about 10 to about 25, from about 10 to about 20, from about 10 to about 18, from about 10 to about 15, from about 12 to about 50, from about 12 to about 30, from about 12 to about 25, from about 12 to about 20, from about 12 to about 18, or from about 12 to about 15 nucleotides in length. In some embodiments, the OncoSkip comprises 8, 9, 10, 11, 12, 13, 14, 15, 16, 17, 18, 19, 20, 21, 22, 23, 24, 25, 30, 40, or 50 nucleotides in length.

In some embodiments, the OncoSkip comprises one or more modified nucleotides, e.g., comprises about 5, 10, 20, 30, 40, 50, 60, 70, 80, 90, or 100% modified nucleotides. In some instances, the OncoSkip comprises about 1, 2, 3, 4, 5, 6, 7, 8, 9, 10, 11, 12, 13, 14, 15, 20, 25, 30, 35, 40, or more modified nucleotides. In some cases, the OncoSkip comprises one or more morpholino-modified nucleotides. In some cases, the OncoSkip comprises about 5, 10, 20, 30, 40, 50, 60, 70, 80, 90, or 100% morpholino-modified nucleotides. In some cases, the OncoSkip comprises about 1, 2, 3, 4, 5, 6, 7, 8, 9, 10, 11, 12, 13, 14, 15, 20, 25, 30, 35, 40, or more morpholino-modified nucleotides.

In some cases, the OncoSkip works in concert with a TransSkip. As used herein, the term "TransSkip" refers to a recombinant vector (e.g., a recombinant viral vector such as an AAV vector) that comprises a transgene that is interrupted by an intron-exon-intron region, in which the exon comprises a stop codon that prevents normal expression of the transgene-encoded polypeptide. In some instances, the transgene is further encompassed by a construct that comprises a polynucleotide encoding a dimert. In some cases, in conjunction with an OncoSkip, the OncoSkip skips the exon from the intron-exon-intron region during splicing to generate a mRNA which enables expression of the transgene-encoded polypeptide. In the absence of the OncoSkip, the transgene expression from the TransSkip is silenced due to the presence of the stop codon in the intron-exon-intron region.

As used herein, the term "dimert" refers to an engineered protein molecule comprising two or more single chain variable fragments (scFv) in which each scFv recognizes a surface polypeptide (e.g., a surface receptor) expressed on a cell and the two cells are different. In some instances, the dimert target two different cell types, e.g., a cancer cell and an immune cell, or two different immune cell types. Exemplary immune cell types include dendritic cell, nature killer (NK) cell, macrophage, T cell, B cell, monocyte, or neutrophil. In some instances, the immune cell is an effector immune cell. In some cases, the effector immune cell comprises an effector T (T_{eff}) cell (also referred to herein as tumor-infiltrating T cell). Exemplary T_{eff} cells include CD8+ T cells and non-regulatory CD4+ helper T cells. In some instances, the dimert targets a cancer cell and an effector immune cell. In some instances, the dimert targets a cancer cell and a T_{eff} cell. In some instances, the dimert targets two different cancer cells, e.g., two different cancer cells from the same cancer.

In some instances, the two or more scFvs of the dimert are linked by a linker. In some instances, the linker is a peptide linker that facilitates binding of each scFv to its respective target polypeptide. In some instances, the linker comprises a series of poly-Ala, poly-Gly, or a combination thereof. In some instances, the poly-Ala linker, the poly-Gly linker, or a peptide linker comprising a combination of Ala and Gly is each independently from about 2 residues to about 50 residues in length. In some instances, the poly-Ala linker, the poly-Gly linker, or a peptide linker comprising a combination of Ala and Gly is each independently from about 2 residues to about 45 residues, from about 4 residues to about 45 residues, from about 5 residues to about 45 residues, from about 8 residues to about 45 residues, from about 10 residues to about 45 residues, from about 15 residues to about 45 residues, from about 20 residues to about 45 residues, from about 30 residues to about 45 residues, about 2 residues to about 40 residues, from about 4 residues to about 40 residues, from about 5 residues to about 40 residues, from about 8 residues to about 40 residues, from about 10 residues to about 40 residues, from about 15 residues to about 40 residues, from about 20 residues to about 40 residues, from about 30 residues to about 40 residues, about 2 residues to about 30 residues, from about 4 residues to about 30 residues, from about 5 residues to about 30 residues, from about 8 residues to about 30 residues, from about 10 residues to about 30 residues, from about 15 residues to about 30 residues, from about 20 residues to about 30 residues, about 2 residues to about 20 residues, from about 4 residues to about 20 residues, from about 5 residues to about 20 residues, from about 8 residues to about 20 residues, from about 10 residues to about 20 residues, or from about 15 residues to about 20 residues in length. In some instances, the poly-Ala linker, the poly-Gly linker, or a peptide linker comprising a combination of Ala and Gly is each independently about 2, 4, 5, 6, 8, 10, 12, 14, 15, 16, 18, 20, 25, 30, 35, 40, 45, or 50 residues in length.

In some instances, the linker is a (Gly₄Ser)n linker, wherein n is an integer from 1 to 10. In some instances, n is an integer from 1 to 6, from 1 to 4, or from 1 to 3. In some instances, n is 1, 2, 3, 4, 5, 6, 7, 8, 9, or 10. In some cases, the linker is from about 10 to about 50 amino acid residues in length, optionally from about 10 to about 30, from about 10 to about 25, or from about 10 to about 20 amino acid residues in length. In some cases, the linker comprises one or more unnatural amino acids.

In some instances, the dimert further comprises an additional polypeptide. In some instances, the additional polypeptide enhances avidity of the dimert toward a target cell. In some instances, the additional polypeptide is the dimerization domain of human hepatocyte nuclear factor 1α (HNF1α). In some instances, the HNF1α comprises a polypeptide sequence comprising at least 90%, 95%, 96%, 97%, 98%, 99%, or 100% sequence identity to MVSKLSQLQTELLAALLESGLSKEALIQALGE (SEQ ID NO: 47). In some instances, the HNF1α is encoded by a polynucleotide comprising at least 90%, 95%, 96%, 97%, 98%, 99%, or 100% sequence identity to ATGGTGAGCAAGCTGAGCCAGCTGCAGACCGAGCTGCTGGCCGCCCTGCTGGAGAG CGGCCTGAGCAAGGAGGCCCTGATCCAGGCCCTGGGCGAG (SEQ ID NO: 48). In some cases, the additional polypeptide does not induce any additional immunogenicity or toxicity.

In some embodiments, the additional polypeptide is linked to the remainder portion of the dimert by a linker. In some instances, the linker comprises GSGGAP. As utilized herein, the GSGGAP peptide is also referred to herein as a spacer. In some instances, the linker comprises TPLGDTTHTSG. In some instances, the peptide TPLGDTTHTSG is derived from the hinge region of an IgG3.

As used herein, the term "TransJoin" refers to a recombinant vector (e.g., a recombinant viral vector such as an AAV vector) that comprises a polypeptide that encodes a dimert described herein, but without the presence of an intron-exon-intron region, in which the exon comprises a stop codon. As such, a TransJoin is different than a TransSkip because the TransJoin can express a dimert without the need of an OncoSkip.

In some embodiments, a TransJoin described herein is optimized for delivery of a dimert to a target cell or target tissue of interest. In some instances, the TransJoin comprises a promoter and enhancer pair, or a promoter and regulatory element pair that is optimized for delivery and/or expression to the target cell or target tissue. In some instances, the TransJoin is optimized (e.g., with a promoter and enhancer pair, or a promoter and regulatory element pair) for constitutive expression of the dimert for a period of time. In some instances, the TransJoin is optimized (e.g., with a promoter and enhancer pair, or a promoter and regulatory element pair) for constitutive and stable expression of the dimert for a period of time. In some instances, the TransJoin comprises a promoter and a regulatory element such as Woodchuck Hepatitis Virus (WHP) post-transcriptional regulatory element (WPRE) for enhanced expression, constitutive expression, stable expression, or a combination thereof.

In some embodiments, a TransJoin described herein comprises a promoter and enhancer pair, or a promoter and regulatory element pair that is optimized for a balanced expression of the dimert at the target cell or target tissue. In some instances, a balanced expression refers to a range of expression in which expression that is above this range would induce toxicity while below this range would not exert a therapeutic effect. In some cases, the promoter and enhancer, or the promoter and the regulatory element (e.g., WPRE) act in concert to balance the expression of the dimert to reach a target range. In some instances, the balanced expression comprises a wide range, for example, to provide a broad therapeutic window for the dimert.

In some embodiments, a TransJoin described herein further comprises a secretory consensus sequence described *infra* that is optimized for a balanced expression of the dimert at the target cell or target tissue. In some instances, a balanced expression refers to a range of expression in which expression that is above this range would induce toxicity while below this range would not exert a therapeutic effect. In some cases, the secretory consensus sequence acts in concert with a promoter, enhancer, a regulatory element (e.g., WPRE), or a combination thereof to balance the expression of the dimert to reach a target range. In some instances, the balanced expression comprises a wide range, for example, to provide a broad therapeutic window for the dimert.

As used above, the period of time comprises one day, two days, three days, four days, five days, 7 days, 21 days, 28 days, one week, two weeks, three weeks, four weeks, one month, two months, three months, four months, five months, six months, 8 months, 10 months, 1 year, 2 years, or more.

The term "isolated" as used herein refers to molecules or biologicals or cellular materials being substantially free from other materials.

As used herein, the term "functional" may be used to modify any molecule, biological, or cellular material to intend that it accomplishes a particular, specified effect.

As used herein, the terms "nucleic acid sequence" and "polynucleotide" are used interchangeably to refer to a polymeric form of nucleotides of any length, either ribonucleotides or deoxyribonucleotides. Thus, this term includes, but is not limited to, single-, double-, or multi-stranded DNA or RNA, genomic DNA, cDNA, DNA-RNA hybrids, or a polymer comprising purine and pyrimidine bases or other natural, chemically, or biochemically modified, non-natural, or derivatized nucleotide bases.

The term "promoter" as used herein refers to any sequence that regulates the expression of a coding sequence, such as a gene. Promoters may be constitutive, inducible, repressible, or tissue-specific, for example. A "promoter" is a control sequence that is a region of a polynucleotide sequence at which initiation and rate of transcription are controlled. It may contain genetic elements at which regulatory proteins and molecules may bind such as RNA polymerase and other transcription factors. Non-limiting exemplary promoters include Rous sarcoma virus (RSV) LTR promoter (optionally with the RSV enhancer), a cytomegalovirus (CMV) promoter, an SV40 promoter, a dihydrofolate reductase promoter, a β-actin promoter, a phosphoglycerol kinase (PGK) promoter, a U6 promoter, or an EF1alpha short form (EFS) promoter.

In some embodiments, the promoter is the EF1 alpha short form (EFS) promoter. In some cases, the EF1 alpha short form (EFS) promoter comprises GTCCGTTATCAACTTGAAAAAGTGGCACCGAGTCGGTGCTTTTTTGAATTCGCTAGC TAGGTCTTGAAAGGAGTGGGAATTGGCTCCGGTGCCCGTCAGTGGGCAGAGCGCAC ATCGCCCACAGTCCCCGAGAAGTTGGGGGGAGGGGTCGGCAATTGATCCGGTGCCT AGAGAAGGTGGCGCGGGGTAAACTGGGAAAGTGATGTCGTGTACTGGCTCCGCCTT TTTCCCGAGGGTGGGGGAGAACCGTATATAAGTGCAGTAGTCGCCGTGAACGTTCT TTTTCGCAACGGGTTTGCCGCCAGAACACAGG (SEQ ID NO: 49), or an equivalent thereof.

Additional non-limiting exemplary promoters with certain target specificity are provided herein below including but not limited to cytomegalovirus (CMV), human polypeptide chain elongation factor (EF1a), SV40, phosphoglycerate kinase (PGK) such as PGK1 (human or mouse), P5, Ubc, human beta actin, CAG, TRE, UAS, Ac5, Polyhedrin, CaMKIIa, Gal1, TEF1, GDS, ADH1, CaMV35S, ubiquitin (Ubi) such ubiquitin C (UbiC), H1, U6, Alpha-1-antitrypsin, spleen focus-forming virus (SFFV), and chicken beta-actin (CBA). Synthetically-derived promoters may be used for ubiquitous or tissue specific expression. Further, virus-derived promoters, some of which are noted above, may be useful in the methods disclosed herein, e.g., CMV, HIV, adenovirus, and AAV promoters.

In some embodiments, the promoter is a tissue-specific promoter. In some instances, the tissue-specific promoter is an endogenous promoter, or a promoter that is derived from genes solely expressed in a target cell type. Exemplary tissue specific promoters include, but are not limited to, liver-specific promoters such as ApoE/hAAT, LP1, SV40/hAlb (InvivoGen); photoreceptor-specific promoters such as human rhodopsin kinase (GRK1) and cone arrestin (CAR); B cell specific promoter such as B29 (InvivoGen); haematopoietic cell specific promoter such as CD45 promoter and SV40/CD45 from InvivoGen; muscle cell specific promoter such as desmin promoter (InvivoGen); pancreatic acinar cell specific promoter such as Elastase-1 promoter (InvivoGen); endothelial cell specific promoter such as Flt-1 promoter (InvivoGen); and neuron specific promoter such as SYN1 promoter (InvivoGen).

In some embodiments, the promoter is coupled to an enhancer to increase the transcription efficiency. Non-limiting examples of enhancers include an RSV enhancer, a CMV enhancer, and α-fetoprotein MERII enhancer.

An enhancer is a regulatory element that increases the expression of a target sequence. A "promoter/enhancer" is a polynucleotide that contains sequences capable of providing both promoter and enhancer functions. For example, the long terminal repeats of retroviruses contain both promoter and enhancer functions. The enhancer/promoter may be "endogenous" or "exogenous" or "heterologous." An "endogenous" enhancer/promoter is one which is naturally linked with a given gene in the genome. An "exogenous" or "heterologous" enhancer/promoter is one which is placed in juxtaposition to a gene by means of genetic manipulation (i.e., molecular biological techniques) such that transcription of that gene is directed by the linked enhancer/promoter.

In some embodiments, a vector utilized herein (e.g., a viral vector such as an AAV vector) further comprises one or more additional regulatory elements. Exemplary regulatory elements include, but are not limited to, transcription terminators, polyadenylation sites, and inverted terminal repeats (ITRs) such as 5' ITR and 3' ITR. In some instances, a regulatory element comprises Woodchuck Hepatitis Virus (WHP) post-transcriptional regulatory element (WPRE). In some cases, an exemplary WPRE comprises a nucleic acid sequence of SEQ ID NO: 50, or an equivalent thereof. The sequence of SEQ ID NO: 50 is set forth below:

TCGACAATCAACCTCTGGATTACAAAATTTGTGAAAGATTGACTGGTATTCTT
AACTATGTTGCTCCTTTTACGCTATGTGGATACGCTGCTTTAATGCCTTTGTATCATG
CTATTGCTTCCCGTATGGCTTTCATTTTCTCCTCCTTGTATAAATCCTGGTTGCTGTCT
CTTTATGAGGAGTTGTGGCCCGTTGTCAGGCAACGTGGCGTGGTGTGCACTGTGTTT
GCTGACGCAACCCCCACTGGTTGGGGCATTGCCACCACCTGTCAGCTCCTTTCCGGG
ACTTTCGCTTTCCCCCTCCCTATTGCCACGGCGGAACTCATCGCCGCCTGCCTTGCCC
GCTGCTGGACAGGGGCTCGGCTGTTGGGCACTGACAATTCCGTGGTGTTGTCGGGG
AAGCTGACGTCCTTTCCATGGCTGCTCGCCTGTGTTGCCACCTGGATTCTGCGCGGG
ACGTCCTTCTGCTACGTCCCTTCGGCCCTCAATCCAGCGGACCTTCCTTCCCGCGGC
CTGCTGCCGGCTCTGCGGCCTCTTCCGCGTCTTCGCCTTCGCCCTCAGACGAGTCGG
ATCTCCCTTTGGGCCGCCTCCCCGCCTG (SEQ ID NO: 50)

The term "protein," "peptide," and "polypeptide" are used interchangeably and in their broadest sense to refer to a compound of two or more subunits of amino acids, amino acid analogs or peptidomimetics. The subunits may be linked by peptide bonds. In another aspect, the subunit may be linked by other bonds, e.g., ester, ether, etc. A protein or peptide must contain at least two amino acids and no limitation is placed on the maximum number of amino acids which may comprise a protein's or peptide's sequence. As used herein the term "amino acid" refers to either natural and/or unnatural or synthetic amino acids, including glycine and both the D and L optical isomers, amino acid analogs, and peptidomimetics.

As used herein, the term "vector" refers to a non-chromosomal nucleic acid comprising an intact replicon such that the vector may be replicated when placed within a cell, for example by a process of transformation. Vectors may be viral or non-viral. Viral vectors include Retroviruses, Adenoviruses, Herpesvirus, Baculoviruses, modified Baculoviruses, Papovavirus, or otherwise modified naturally occurring viruses. Exemplary non-viral vectors for delivering nucleic acid include naked DNA; DNA complexed with cationic lipids, alone or in combination with cationic polymers; anionic and cationic liposomes; DNA-protein complexes and particles comprising DNA condensed with cationic polymers such as heterogeneous polylysine, defined-length oligopeptides, and polyethylene imine, in some cases contained in liposomes; and the use of ternary complexes comprising a virus and polylysine-DNA. In another embodiment, the vector is a recombinant viral vector comprising a backbone vector selected from the group of a retroviral vector, a lentiviral vector, a murine leukemia viral ("MLV") vector, an Epstein-Barr viral ("EBV") vector, an adenoviral vector, a herpes viral ("HSV") vector, or an Adeno-associated viral ("AAV") vector. In another embodiment, the vector is an AAV vector, or optionally a self-complementary AAV vector.

A "viral vector" is defined as a recombinantly produced virus or viral particle that comprises a polynucleotide to be delivered into a host cell, either in vivo, ex vivo or in vitro. Examples of viral vectors include retroviral vectors, AAV vectors, lentiviral vectors, adenovirus vectors, alphavirus vectors and the like. Alphavirus vectors, such as Semliki Forest virus-based vectors and Sindbis virus-based vectors, have also been developed for use in gene therapy and immunotherapy. See, Schlesinger and Dubensky (1999) Curr. Opin. Biotechnol. 5: 434-439 and Ying, et al. (1999) Nat. Med. 5(7): 823-827. In some instances, the viral vector is an AAV vector, e.g., AAV1, AAV2, AAV4, AAV5, AAV6, AAV7, AAV8, AAV9, AAV10, AAV11, AAV12, AAV13, AAV PHP.B, AAV rh74, or AAV-DJ. In some cases, the viral vector is AAV rh74. In some cases, the AAV rh74 comprises the vector sequence with the GenBank accession number LP899424.1 (accessed on February 7, 2020).

In some embodiments, there is a limited carrying capacity for a viral vector (e.g., an AAV vector). For example, an AAV vector has a carrying capacity limit of 4.7 kb. As such, the combination of the dimert along with the promoter, enhancer, and other regulatory elements need to be within the 4.7 kb capacity. In such instances, a promoter utilized herein is selected based on its nucleic acid length to enable packaging of the dimert and other regulatory elements into a viral vector, e.g., an AAV vector. In some cases, the promoter for such use is SFFV, EF1α, PGK, UbiC, CMV, CBA, or EFS. In some cases, the promoter is EFS.

In another embodiment, the promoter is an inducible promoter. In a specific related embodiment, the promoter an inducible tetracycline promoter. The Tet-Off and Tet-On Gene Expression Systems give researchers ready access to the regulated, high-level gene expression systems described by Gossen & Bujard (1992; Tet-Off) and Gossen et al. (1995; Tet-On). In the Tet-Off system, gene expression is turned on when tetracycline (Tc) or doxycycline (Dox; a Tc derivative) is removed from the culture medium. In contrast, expression is turned on in the Tet-On system by the addition of Dox. Both systems permit gene expression to be tightly regulated in response to varying concentrations of Tc or Dox. Maximal expression levels in Tet systems are very high and compare favorably with the maximal levels obtainable from strong, constitutive mammalian promoters such as CMV (Yin et al., 1996). Unlike other inducible mammalian expression systems, gene regulation in the Tet Systems is highly specific, so interpretation of results is not complicated by pleiotropic effects or nonspecific induction. In *E. coli*, the Tet repressor protein (TetR) negatively regulates the genes of the tetracycline-resistance operon on the Tn10 transposon. TetR blocks transcription of these genes by binding to the tet operator sequences (tetO) in the absence of Tc. TetR and tetO provide the basis of regulation and induction for use in mammalian experimental systems. In the Tet-On system, the regulatory protein is based on a "reverse" Tet repressor (rTetR) which was created by four amino acid changes in TetR (Hillen & Berens, 1994; Gossen et al., 1995). The resulting protein, rtTA (reverse tTA also referred to tetracycline activator protein), is encoded by the pTet-On regulator plasmid.

In a related embodiment, the vector further comprises, or alternatively consists essentially of, or yet further consists of a nucleic acid encoding a tetracycline activator protein; and a promoter that regulates expression of the tetracycline activator protein.

Other inducible systems useful in vectors, isolated cells, viral packaging systems, and methods described herein include regulation by ecdysone, by estrogen, progesterone, chemical inducers of dimerization, and isopropyl-beta-D1-thiogalactopyranoside (EPTG).

As used herein, the term "recombinant expression system" or "recombinant vector" refers to a genetic construct or constructs for the expression of certain genetic material formed by recombination.

A "gene delivery vehicle" is defined as any molecule that can carry inserted polynucleotides into a host cell. Examples of gene delivery vehicles are liposomes, micelles biocompatible polymers, including natural polymers and synthetic polymers; lipoproteins; polypeptides; polysaccharides; lipopolysaccharides; artificial viral envelopes; metal particles; and bacteria, or viruses, such as Baculovirus, Adenovirus and Retrovirus, Bacteriophage, cosmid, plasmid, fungal vectors, and other recombination vehicles typically used in the art which have been described for expression in a variety of eukaryotic and prokaryotic hosts, and may be used for gene therapy as well as for simple protein expression.

A polynucleotide disclosed herein can be delivered to a cell or tissue using a gene delivery vehicle. "Gene delivery," "gene transfer," "transducing," and the like as used herein, are terms referring to the introduction of an exogenous polynucleotide (sometimes referred to as a "transgene") into a host cell, irrespective of the method used for the introduction. Such methods include a variety of well-known techniques such as vector-mediated gene transfer (by, e.g., viral infection/transfection, or various other protein-based, or lipid-based gene delivery complexes) as well as techniques facilitating the delivery of "naked" polynucleotides (such as electroporation, "gene gun" delivery and various other techniques used for the introduction of polynucleotides). The introduced polynucleotide may be stably or transiently maintained in the host cell. Stable maintenance typically requires that the introduced polynucleotide either contains an origin of replication compatible with the host cell or integrates into a replicon of the host cell such as an extrachromosomal replicon (e.g., a plasmid) or a nuclear or mitochondrial chromosome. A number of vectors are known to be capable of mediating transfer of genes to mammalian cells, as is known in the art and described herein.

A "plasmid" is an extra-chromosomal DNA molecule separate from the chromosomal DNA which is capable of replicating independently of the chromosomal DNA. In many cases, it is circular and double-stranded. Plasmids provide a mechanism for horizontal gene transfer within a population of microbes and typically provide a selective advantage under a given environmental state. Plasmids may carry genes that provide resistance to naturally occurring antibiotics in a competitive environmental niche, or alternatively the proteins produced may act as toxins under similar circumstances.

"Plasmids" used in genetic engineering are called "plasmid vectors." Many plasmids are commercially available for such uses. The gene to be replicated is inserted into copies of a plasmid containing genes that make cells resistant to particular antibiotics and a multiple cloning site (MCS, or polylinker), which is a short region containing several commonly used restriction sites allowing the easy insertion of DNA fragments at this location. Another major use of plasmids is to make large amounts of proteins. In this case, researchers grow bacteria containing a plasmid harboring the gene of interest. Just as the bacterium produces proteins to confer its antibiotic resistance, it can also be induced to produce large amounts of proteins from the inserted gene.

In aspects where gene transfer is mediated by a DNA viral vector, such as an Adenovirus (Ad) or Adeno-associated virus (AAV), a vector construct refers to the polynucleotide comprising the viral genome or part thereof, and a transgene. Adenoviruses (Ads) are a relatively well characterized, homogenous group of viruses, including over 50 serotypes. Ads do not require integration into the host cell genome. Recombinant Ad derived vectors, particularly those that reduce the potential for recombination and generation of wild-type virus, have also been constructed. Such vectors are commercially available from sources such as Takara Bio USA (Mountain View, CA), Vector Biolabs (Philadelphia, PA), and Creative Biogene (Shirley, NY). Wild-type AAV has high infectivity and specificity integrating into the host cell's genome. See, Wold and Toth (2013) Curr. Gene. Ther. 13(6): 421-433, Hermonat & Muzyczka (1984) Proc. Natl. Acad. Sci. USA 81: 6466-6470, and Lebkowski et al. (1988) Mol. Cell. Biol. 8: 3988-3996.

Vectors that contain both a promoter and a cloning site into which a polynucleotide can be operatively linked are well known in the art. Such vectors are capable of transcribing RNA in vitro or in vivo, and are commercially available from sources such as Agilent Technologies (Santa Clara, Calif.) and Promega Biotech (Madison, Wis.). In order to optimize expression and/or in vitro transcription, it may be necessary to remove, add or alter 5' and/or 3' untranslated portions of the clones to eliminate extra, potential inappropriate alternative translation initiation codons or other sequences that may interfere with or reduce expression, either at the level of transcription or translation. Alternatively, consensus ribosome binding sites can be inserted immediately 5' of the start codon to enhance expression.

As used herein, the term "engager," "engager molecule," or "activating antigen" refers to a molecule that is secreted from a cell that is capable of activating immune cells. In particular embodiments, the engager activates specific an immune effector cell according to the domains present in the engager. Illustrative examples of cells that secrete engagers, but are not limited to, include a T-cell, an NK cell, an NKT cells, a CAR T-cell, a mesenchymal stem cell (MSC), a neuronal stem cell, a hematopoietic stem cell, or a mixture thereof. In another embodiment, the immune effector cells comprises a dendritic cell, a natural killer ("NK") cell, a macrophage, a T cell, a B cell, or combination thereof.

The term "antigen recognition domain" or "antigen-binding domain" refers to a part of the engager molecule that recognizes an antigen. In particular embodiments, antigens can be of any nature including, but not limited to, proteins, carbohydrates, and/or synthetic molecules.

As used herein, the term "activating antigen" or "activation domain" refers to a part of the engager molecules that interacts with the immune cells and induces a positive or negative immunomodulatory signal. Illustrative examples of positive immunomodulatory signals include signals that induce cell proliferation, cytokine secretion, or cytolytic activity. Illustrative examples of negative immunomodulatory signals include signals that inhibit cell proliferation, inhibit the secretion of immunosuppressive factors, or induce cell death.

As used herein, the term "native immune cell" refers to an immune cell that naturally occurs in the immune system. Illustrative examples include, but are not limited to, T-cells, NK cells, NKT cells, B cells, and dendritic cells.

As used herein, the term "engineered immune cell" refers to an immune cell that is genetically modified.

As used herein, the term "T-cell" includes naive T cells, CD4+ T cells, CD8+ T cells, memory T cells, activated T cells, anergic T cells, tolerant T cells, chimeric B cells, and antigen-specific T cells.

As used herein, the term "antibody" means not only intact antibody molecules, but also fragments of antibody molecules that retain immunogen-binding ability. Such fragments are also well known in the art and are regularly employed both *in vitro* and *in vivo.* Accordingly, as used herein, the term "antibody" means not only intact immunoglobulin molecules but also the well-known active fragments F(ab')₂, and Fab. F(ab')₂, and Fab fragments that lack the Fc fragment of intact antibody, clear more rapidly from the circulation, and may have less nonspecific tissue binding of an intact antibody (Wahl et al., J. Nucl. Med. 24: 316-325 (1983)). The antibodies of the invention comprise whole native antibodies, monoclonal antibodies, human antibodies, humanized antibodies, camelised antibodies, multispecific antibodies, bispecific antibodies, chimeric antibodies, Fab, Fab', single chain V region fragments (scFv), single domain antibodies (e.g., nanobodies and single domain camelid antibodies), V_{NAR} fragments, Bi-specific T-cell engager (BiTE) antibodies, minibodies, disulfide-linked Fvs (sdFv), and anti-idiotypic (anti-Id) antibodies, intrabodies, fusion polypeptides, unconventional antibodies, and antigen-binding fragments of any of the above. In particular, antibodies include immunoglobulin molecules and immunologically active fragments of immunoglobulin molecules, i.e., molecules that contain an antigen-binding site. Immunoglobulin molecules can be of any type (e.g., IgG, IgE, IgM, IgD, IgA, and IgY), class (e.g., IgG1, IgG2, IgG3, IgG4, IgAl, and IgA2) or subclass.

In certain embodiments, an antibody is a glycoprotein comprising at least two heavy (H) chains and two light (L) chains inter-connected by disulfide bonds. Each heavy chain is comprised of a heavy chain variable region (abbreviated herein as V_{H}) and a heavy chain constant (Cu) region. The heavy chain constant region is comprised of three domains, CH1, CH2, and CH3. Each light chain is comprised of a light chain variable region (abbreviated herein as V_{L}) and a light chain constant C_{L} region. The light chain constant region is comprised of one domain, C_{L}. The V_{H} and V_{L} regions can be further subdivided into regions of hypervariability, termed complementarity determining regions (CDR), interspersed with regions that are more conserved, termed framework regions (FR). Each V_{H} and V_{L} is composed of three CDRs and four FRs arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component (Cl q) of the classical complement system. As used herein interchangeably, the terms "antigen-binding portion," "antigen-binding fragment," or "antigen-binding region" of an antibody, refer to the region or portion of an antibody that binds to the antigen and which confers antigen specificity to the antibody; fragments of antigen-binding proteins, for example, antibodies include one or more fragments of an antibody that retain the ability to specifically bind to an antigen (e.g., an peptide/HLA complex). It has been shown that the antigen-binding function of an antibody can be performed by fragments of a full-length antibody. Examples of antigen-binding portions encompassed within the term "antibody fragments" of an antibody include a Fab fragment, a monovalent fragment consisting of the V_{L}, V_{H}, C_{L} and CHI domains; a F(ab)₂ fragment, a bivalent fragment comprising two Fab fragments linked by a disulfide bridge at the hinge region; a Fd fragment consisting of the V_{H} and CHI domains; a Fv fragment consisting of the V_{L} and V_{H} domains of a single arm of an antibody; a dAb fragment (Ward et al., Nature 341 : 544-546 (1989)), which consists of a V_{H} domain; and an isolated complementarity determining region (CDR).

Antibodies and antibody fragments can be wholly or partially derived from mammals (e.g., humans, non-human primates, goats, guinea pigs, hamsters, horses, mice, rats, rabbits, and sheep) or non-mammalian antibody producing animals (e.g., chickens, ducks, geese, snakes, urodele amphibians). The antibodies and antibody fragments can be produced in animals or produced outside of animals, such as from yeast or phage (e.g., as a single antibody or antibody fragment or as part of an antibody library).

Furthermore, although the two domains of the Fv fragment, V_{L} and V_{H}, are coded for by separate genes, they can be joined, using recombinant methods, by a synthetic linker that enables them to be made as a single protein chain in which the V_{L} and V_{H} regions pair to form monovalent molecules. These are known as single chain Fv (scFv); see e.g., Bird et al., Science 242: 423-426 (1988); and Huston et al., Proc. Natl. Acad. Sci. 85: 5879-5883 (1988). These antibody fragments are obtained using conventional techniques known to those of ordinary skill in the art, and the fragments are screened for utility in the same manner as are intact antibodies.

An "isolated antibody" or "isolated antigen-binding protein" is one which has been identified and separated and/or recovered from a component of its natural environment. "Synthetic antibodies" or "recombinant antibodies" are generally generated using recombinant technology or using peptide synthetic techniques known to those of skill in the art.

As used herein, the term "single-chain variable fragment" or "scFv" is a fusion protein of the variable regions of the heavy (V_{H}) and light chains (V_{L}) of an immunoglobulin (e.g., mouse or human) covalently linked to form a V_{H}: V_{L} heterodimer. The heavy (V_{H}) and light chains (V_{L}) are either joined directly or joined by a peptide-encoding linker (e.g., about 10, 15, 20, 25 amino acids), which connects the N-terminus of the V_{H} with the C-terminus of the V_{L}, or the C-terminus of the V_{H} with the N-terminus of the V_{L}. The linker is usually rich in glycine for flexibility, as well as serine or threonine for solubility. The linker can link the heavy chain variable region and the light chain variable region of the extracellular antigen-binding domain.

Despite removal of the constant regions and the introduction of a linker, scFv proteins retain the specificity of the original immunoglobulin. Single chain Fv polypeptide antibodies can be expressed from a nucleic acid comprising V_{H}- and V_{L}-encoding sequences as described by Huston, et al. (Proc. Nat. Acad. Sci. USA, 85: 5879-5883 (1988)). See, also, U.S. Patent Nos. 5,091,513, 5,132,405 and 4,956,778; and U.S. Patent Publication Nos. 20050196754 and 20050196754. Antagonistic scFvs having inhibitory activity have been described (see, e.g., Zhao et al., Hybridoma (Larchmt) 27(6): 455-51 (2008); Peter et al., J Cachexia Sarcopenia Muscle (2012); Shieh et al., J. Imunol 183(4): 2277-85 (2009); Giomarelli et al., Thromb Haemost 97(6): 955-63 (2007); Fife eta., J Clin Invst 116(8): 2252-61 (2006); Brocks et al., Immunotechnology 3(3): 173-84 (1997); Moosmayer et al., Ther Immunol 2(10): 31- 40 (1995) Agonistic scFvs having stimulatory activity have been described (see, e.g., Peter et al., J Biol Chem 25278(38): 36740-7 (2003); Xie et al., Nat Biotech 15(8): 768-71 (1997); Ledbetter et al., Crit Rev Immunol 17(5-6): 427-55 (1997); Ho et al., Bio Chim Biophys Acta 1638(3): 257-66 (2003)).

As used herein, "F(ab)" refers to a fragment of an antibody structure that binds to an antigen but is monovalent and does not have a Fc portion, for example, an antibody digested by the enzyme papain yields two F(ab) fragments and an Fc fragment (e.g., a heavy (H) chain constant region; Fc region that does not bind to an antigen).

As used herein, "F(ab')₂" refers to an antibody fragment generated by pepsin digestion of whole IgG antibodies, wherein this fragment has two antigen binding (ab') (bivalent) regions, wherein each (ab¹) region comprises two separate amino acid chains, a part of a H chain and a light (L) chain linked by an S-S bond for binding an antigen and where the remaining H chain portions are linked together. A "F(ab')₂" fragment can be split into two individual Fab' fragments.

As used herein, "CDRs" are defined as the complementarity determining region amino acid sequences of an antibody which are the hypervariable regions of immunoglobulin heavy and light chains. See, e.g., Kabat et al., Sequences of Proteins of Immunological Interest, 4th U.S. Department of Health and Human Services, National Institutes of Health (1987). Generally, antibodies comprise three heavy chain and three light chain CDRs or CDR regions in the variable region. CDRs provide the majority of contact residues for the binding of the antibody to the antigen or epitope. In certain embodiments, the CDRs regions are delineated using the Kabat system (Kabat, E. A., et al. Sequences of Proteins of Immunological Interest, Fifth Edition, U.S. Department of Health and Human Services, NIH Publication No. 91-3242(1991)).

As used herein, the term "affinity" is meant a measure of binding strength. Without being bound to theory, affinity depends on the closeness of stereochemical fit between antibody combining sites and antigen determinants, on the size of the area of contact between them, and on the distribution of charged and hydrophobic groups. Affinity also includes the term "avidity," which refers to the strength of the antigen-antibody bond after formation of reversible complexes (e.g., either monovalent or multivalent). Methods for calculating the affinity of an antibody for an antigen are known in the art, comprising use of binding experiments to calculate affinity. Antibody activity in functional assays (e.g., flow cytometry assay) is also reflective of antibody affinity. Antibodies and affinities can be phenotypically characterized and compared using functional assays (e.g., flow cytometry assay). Nucleic acid molecules useful in the presently disclosed subject matter include any nucleic acid molecule that encodes a polypeptide or a fragment thereof. In certain embodiments, nucleic acid molecules useful in the presently disclosed subject matter include nucleic acid molecules that encode an antibody or an antigen-binding portion thereof. Such nucleic acid molecules need not be 100% identical with an endogenous nucleic acid sequence, but will typically exhibit substantial identity. Polynucleotides having "substantial homology" or "substantial identity" to an endogenous sequence are typically capable of hybridizing with at least one strand of a double-stranded nucleic acid molecule. By "hybridize" is meant pair to form a double-stranded molecule between complementary polynucleotide sequences (e.g., a gene described herein), or portions thereof, under various conditions of stringency. (See, e.g., Wahl, G. M. and S. L. Berger, Methods Enzymol. 152: 399 (1987); Kimmel, A. R. Methods Enzymol. 152: 507 (1987)).

The terms "substantially homologous" or "substantially identical" mean a polypeptide or nucleic acid molecule that exhibits at least 50% or greater homology or identity to a reference amino acid sequence (for example, any one of the amino acid sequences described herein) or nucleic acid sequence (for example, any one of the nucleic acid sequences described herein). For example, such a sequence is at least about 60%, about 65%, about 70%, about 75%, about 80%, about 85%, about 90%, about 95%, or about 96%, or about 97%, or about 98%, or about 99% homologous or identical at the amino acid level or nucleic acid to the sequence used for comparison (e.g., a wild-type, or native, sequence). In some embodiments, a substantially homologous or substantially identical polypeptide contains one or more amino acid amino acid substitutions, insertions, or deletions relative to the sequence used for comparison. In some embodiments, a substantially homologous or substantially identical polypeptide contains one or more non-natural amino acids or amino acid analogs, including, D-amino acids, and retroinverso amino, to replace homologous sequences.

Sequence homology or sequence identity is typically measured using sequence analysis software (for example, Sequence Analysis Software Package of the Genetics Computer Group, University of Wisconsin Biotechnology Center, 1710 University Avenue, Madison, Wis. 53705, BLAST, BESTFIT, GAP, or PILEUP/PRETTYBOX programs). Such software matches identical or similar sequences by assigning degrees of homology to various substitutions, deletions, and/or other modifications. In an exemplary approach to determining the degree of identity, a BLAST program may be used, with a probability score between e⁻³ and ^{e-100} indicating a closely related sequence.

As used herein, the term "analog" refers to a structurally related polypeptide or nucleic acid molecule having the function of a reference polypeptide or nucleic acid molecule.

As used herein, the term "a conservative sequence modification" refers to an amino acid modification that does not significantly affect or alter the binding characteristics of the presently disclosed engineered receptor (e.g., the extracellular antigen-binding domain of the engineered receptor) comprising the amino acid sequence. Conservative modifications can include amino acid substitutions, additions, and deletions. Modifications can be introduced into the human scFv of the presently disclosed engineered receptor by standard techniques known in the art, such as site-directed mutagenesis and PCR-mediated mutagenesis. Amino acids can be classified into groups according to their physicochemical properties such as charge and polarity. Conservative amino acid substitutions are ones in which the amino acid residue is replaced with an amino acid within the same group. For example, amino acids can be classified by charge: positively-charged amino acids include lysine, arginine, histidine, negatively-charged amino acids include aspartic acid, glutamic acid, neutral charge amino acids include alanine, asparagine, cysteine, glutamine, glycine, isoleucine, leucine, methionine, phenylalanine, proline, serine, threonine, tryptophan, tyrosine, and valine. In addition, amino acids can be classified by polarity: polar amino acids include arginine (basic polar), asparagine, aspartic acid (acidic polar), glutamic acid (acidic polar), glutamine, histidine (basic polar), lysine (basic polar), serine, threonine, and tyrosine; non-polar amino acids include alanine, cysteine, glycine, isoleucine, leucine, methionine, phenylalanine, proline, tryptophan, and valine. Thus, one or more amino acid residues within a CDR region can be replaced with other amino acid residues from the same group and the altered antibody can be tested for retained function (i.e., the functions set forth in (c) through (1) above) using the functional assays described herein. In certain embodiments, no more than one, no more than two, no more than three, no more than four, no more than five residues within a specified sequence, or a CDR region are altered.

As used herein, the term "ligand" refers to a molecule that binds to a receptor. In particular, the ligand binds a receptor on another cell, allowing for cell-to-cell recognition and/or interaction.

As used herein, the term, "co-stimulatory signaling domain," or "co-stimulatory domain", refers to the portion of the engineered receptor comprising the intracellular domain of a co-stimulatory molecule. Co-stimulatory molecules are cell surface molecules other than antigen receptors or Fc receptors that provide a second signal required for efficient activation and function of T lymphocytes upon binding to antigen. Examples of such co-stimulatory molecules include CD27, CD28, 4-1BB (CD137), OX40 (CD134), CD30, CD40, PD-1, ICOS (CD278), LFA-1, CD2, CD7, LIGHT, NKD2C, B7-H2, and a ligand that specifically binds CD83. Accordingly, while the present disclosure provides exemplary costimulatory domains derived from CD28 and 4-1BB, other costimulatory domains are contemplated for use with the engineered receptors described herein. The inclusion of one or more co-stimulatory signaling domains can enhance the efficacy and expansion of T cells expressing engineered receptors. The intracellular signaling and co-stimulatory signaling domains can be linked in any order in tandem to the carboxyl terminus of the transmembrane domain.

As used herein, the term "chimeric co-stimulatory receptor" or "CCR" refers to a chimeric receptor that binds to an antigen and provides co-stimulatory signals, but does not provide a T-cell activation signal.

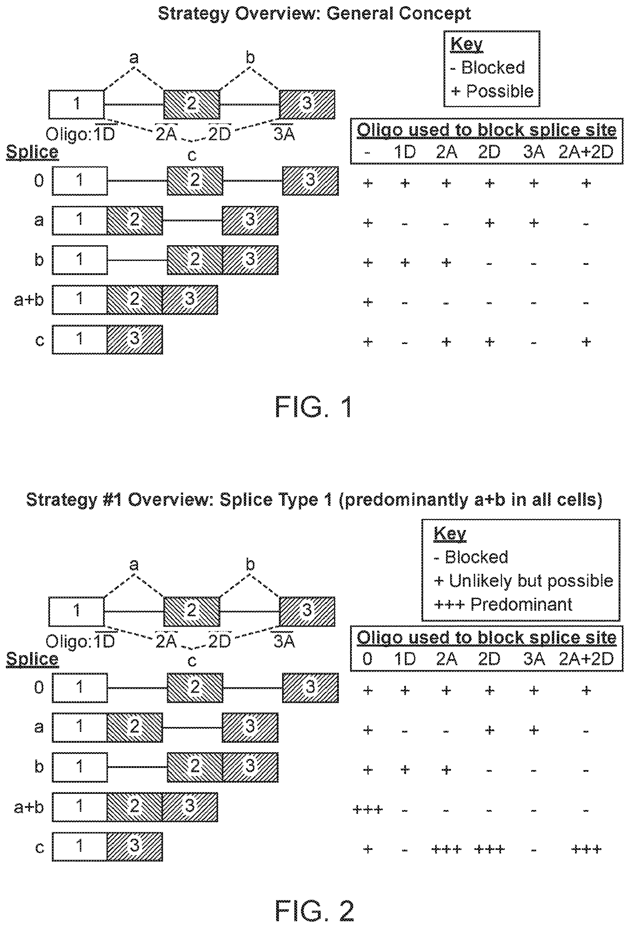

The term "bispecific antibody" or "trispecific antibody" refers to an antibody having two different antigen-binding regions (bispecific antibody) or three different antigen-binding regions (trispecific antibody). In some embodiments, the bispecific antibody comprises an antibody format (or antibody structure) as disclosed Figure 2 of Brinkmann et al., "The making of bispecific antibodies," MABS 9(2): 182-212 (2017); or Figure 2 of Labrijn, et al., "Bispecific antibodies: a mechanistic review of the pipeline," Nature Reviews 18: 585-608 (2019).

In some embodiments, at least one of the antigen-binding regions of the bispecific antibody or trispecific antibody binds to an activating antigen on an immune effector cell. This can be understood as different target bindings but includes as well binding to different epitopes in one target. Non-limiting examples of the activating antigens include, but are not limited to CD3, CD2, CD4, CD8, CD19, LFA1, CD45, NKG2D, NKp44, NKp46, NKp30, DNAM, B7-H3 (CD276), CD20, CD22, or combination thereof. Non-limiting examples of bispecific antibodies include but not limited to CD3xCD19, CD3xGD2, CD3xEphA2, and NKG2DxGD2 antibodies.

In one embodiment, a dimert described above comprises a bispecific antibody. In some instances, a dimert comprises a bispecific T cell engager ("BiTE") antibody, a bispecific killer cell engager ("BiKE") antibody, or other bispecific antibodies described herein, such as those associated with a different immune cell. In another embodiment, the dimert comprises a trispecific antibody. In some instances, the dimert comprises a trispecific T cell engager ("TriTE") antibody or a trispecific killer cell engager ("TriKE") antibody, or other trispecific antibodies described herein, such as associated with a different immune cell.

The term "bispecific T-cell engager" or "BiTE" as used herein refers to a bispecific monoclonal antibody that comprises a first antigen-binding fragment that binds to a T cell receptor and a second antigen-binding fragment that binds to a tumor cell via a tumor-specific molecule. The term "trispecific T-cell engager" ("TiTE" or "TriTE") as used herein refers to a trispecific monoclonal antibody that comprises a first antigen-binding fragment that binds to a T cell engager, a second antigen-binding fragment that binds to a tumor cell via a tumor-specific molecule, and a third antigen-binding fragment that binds to a T cell engager or a cytokine T cell activating domain. The tumor-specific molecule, in one embodiment, comprises a tumor antigen selected from an ephrin type-A receptor 2 (EphA2), interleukin (IL)-13r alpha 2, an EGFR VIII, a PSMA, an EpCAM, a GD2 or GD3, a fucosyl GM1, a PSCA, a PLAC1, a sarcoma breakpoint, a Wilms Tumor 1, alphafetoprotein (AFP), carcinoembryonic antigen (CEA), CA-125, MUC-1, epithelial tumor antigen (ETA), tyrosinase, melanoma-associated antigen (MAGE), a hematologic differentiation antigen, a surface glycoprotein, a gangliosides (GM2), a growth factor receptor, a stromal antigen, a vascular antigen, receptor tyrosine kinase like orphan receptor 1 (ROR1), mesothelin, CD38, CD123, human epidermal growth factor receptor 2 (HER2), B-cell maturation antigen (BCMA), fibroblast activation protein (FAP) alpha, or a combination thereof. Illustrative examples of BiTE include, but not limited to, Blinatumomab (MT103) and Solitomab (MT110).

The term "bispecific killer engager," or "BiKE" as used herein refers to a bispecific monoclonal antibody that comprises a first antigen-binding fragment that binds to a natural killer (NK) cell engager domain and a second antigen-binding fragment that binds to a tumor cell via a tumor-specific molecule. The term "trispecific NK cell engager" ("TiKE" or "TriKE") as used herein refers to a trispecific monoclonal antibody that comprises a first antigen-binding fragment that binds to a NK cell engager, a second antigen-binding fragment that binds to a tumor cell via a tumor-specific molecule, and a third antigen-binding fragment that binds to a NK cell engager or a cytokine NK cell activating domain. NKG2DxIL21RxGD2 antibody is one exemplary TriKE antibody. Examples of NK cell engager domains include, but not are not limited to, ligands or molecules that bind CD16, CD16⁺CD2, CD16⁺DNAM, and CD16⁺NKp46. Examples of cytokine NK activating domains include but are not limited to IL-15, IL-12, IL-18, IL-21, or other NK cell enhancing cytokine, chemokine, and/or activating molecule. The NK engaging domain can include any moiety that binds to and/or activates an NK cell and/or any moiety that blocks inhibition of an NK cell. In some embodiments, the NK engaging domain can include an antibody that selectively binds to a component of the surface of an NK cell. In other embodiments, the NK engaging domain can include a ligand or small molecule that selectively binds to a component of the surface of an NK cell.

In some embodiments, the NK engaging domain can selectively bind to a receptor at least partially located at the surface of an NK cell. In certain embodiments, the NK engaging domain can serve a function of binding an NK cell and thereby bring the NK into spatial proximity with a target to which the targeting domains electively binds. In certain embodiments, however, the NK engaging domain can selectively bind to a receptor that activates the NK cell and, therefore, also possess an activating function. As described above, activation of the CD 16 receptor can elicit antibody-dependent cell-mediated cytotoxicity. Thus, in certain embodiments, the NK engaging domain can include at least a portion of an anti-CD 16 receptor antibody effective to selectively bind to the CD 16 receptor. In other embodiments, the NK engager cell domain may interrupt mechanisms that inhibit NK cells. In such embodiments, the NK engager domain can include, for example, anti-PD1/PDL1, anti-NKG2A, anti-TIGIT, anti-killer-immunoglobulin receptor (KIR), and/or any other inhibition blocking domain.

In particular embodiments, cells are genetically modified (including immune cells) with engager molecules comprising at least an antigen recognition domain and an activation domain and, optionally, a cytokine, costimulatory domain, and/or domain for inhibition of negative regulatory molecules of T-cell activation. The engager molecule's antigen recognition domain binds to one or more molecules present in and/or on target cells or that are secreted by target cells. In specific aspects, the target cells are cancer cells, including at least solid tumor cells. Once engager molecules have bound a target molecule, they can activate cells that express the molecule recognized by the activation domain. Engager molecules can activate cells that are genetically modified with engager molecules or can activate unmodified cells.

Depending on the desired effect, activation can result in a positive or negative signal. Example of positive signals include signals that induce cell proliferation, cytokine secretion, or cytolytic activity. Examples of negative signals include signals that inhibit T-cell proliferation, inhibit the secretion of immunosuppressive factors, or induce cell death.

In particular aspects, immune cells that secrete engager molecules are able to redirect resident (naturally endogenous to a specific individual) immune cells to cancer cells.

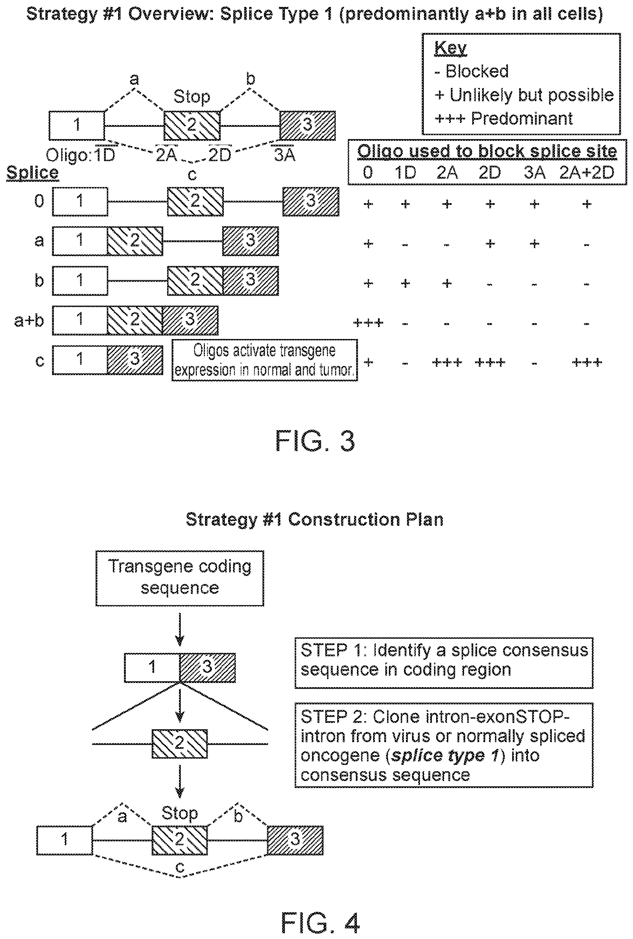

Embodiments of the disclosure provide delivery of modified immune cells that secrete an engager molecule to an individual in need thereof (known to have cancer or suspected of having cancer, including a particular cancer) in contrast to delivering the engager molecule only to the individual itself (in the absence of being produced by modified immune cells). In the present disclosure, the individual receives the modified immune cells that allow production of the engager molecule(s). In particular embodiments, the cells produce immunostimulatory cytokines; proliferate in an antigen-specific manner; kill the appropriate target cells; redirect bystander immune cells (including at least T-cells or NK cells) to cancer cells; secrete engager molecules upon activation; and/or are effective against cancer in a loco-regional or systemic manner. FIG. 3 illustrates examples of modified T-cells or NK cells that secrete engager molecules. Although a particular T-cell or NK-cell may produce an engager that can target the same cancer cell-specific antigen, the activation domains for a T-cell or NK cell must be different, because NK cells do not express CD3. Examples of activation domains for a NK cell include at least CD16, NKG2D, or NKp30, for example.

Gene delivery vehicles also include DNA/liposome complexes, micelles and targeted viral protein-DNA complexes. Liposomes that also comprise a targeting antibody or fragment thereof can be used in the methods disclosed herein. In addition to the delivery of polynucleotides to a cell or cell population, direct introduction of the proteins described herein to the cell or cell population can be done by the non-limiting technique of protein transfection, alternatively culturing conditions that can enhance the expression and/or promote the activity of the proteins disclosed herein are other non-limiting techniques.

As used herein, the term "signal peptide" or "signal polypeptide" intends an amino acid sequence usually present at the N-terminal end of newly synthesized secretory or membrane polypeptides or proteins. It acts to direct the polypeptide to a specific cellular location, e.g., across a cell membrane, into a cell membrane, or into the nucleus. In some embodiments, the signal peptide is removed following localization. Examples of signal peptides are well known in the art. Non-limiting examples are those described in U.S. Patent Nos. 8,853,381, 5,958,736, and 8,795,965.

As used herein, the term "viral capsid" or "capsid" refers to the proteinaceous shell or coat of a viral particle. Capsids function to encapsidate, protect, transport, and release into host cell a viral genome. Capsids are generally comprised of oligomeric structural subunits of protein ("capsid proteins"). As used herein, the term "encapsidated" means enclosed within a viral capsid.

As used herein, the term "helper" in reference to a virus or plasmid refers to a virus or plasmid used to provide the additional components necessary for replication and packaging of a viral particle or recombinant viral particle, such as the modified AAV disclosed herein. The components encoded by a helper virus may include any genes required for virion assembly, encapsidation, genome replication, and/or packaging. For example, the helper virus may encode necessary enzymes for the replication of the viral genome. Non-limiting examples of helper viruses and plasmids suitable for use with AAV constructs include pHELP (plasmid), Adenovirus (virus), or Herpesvirus (virus).

As used herein, the term "AAV" is a standard abbreviation for Adeno-associated virus. Adeno-associated virus is a single-stranded DNA parvovirus that grows only in cells in which certain functions are provided by a co-infecting helper virus. General information and reviews of AAV can be found in, for example, Carter, 1989, Handbook of Parvoviruses, Vol. 1, pp. 169-228, and Berns, 1990, Virology, pp. 1743-1764, Raven Press, (New York). It is fully expected that the same principles described in these reviews will be applicable to additional AAV serotypes characterized after the publication dates of the reviews because it is well known that the various serotypes are quite closely related, both structurally and functionally, even at the genetic level. (See, for example, Blacklowe, 1988, pp. 165-174 of Parvoviruses and Human Disease, J. R. Pattison, ed.; and Rose, Comprehensive Virology 3: 1-61 (1974)). For example, all AAV serotypes apparently exhibit very similar replication properties mediated by homologous rep genes; and all bear three related capsid proteins such as those expressed in AAV2. The degree of relatedness is further suggested by heteroduplex analysis which reveals extensive cross-hybridization between serotypes along the length of the genome; and the presence of analogous self-annealing segments at the termini that correspond to "inverted terminal repeat sequences" (ITRs). The similar infectivity patterns also suggest that the replication functions in each serotype are under similar regulatory control.

An "AAV vector" as used herein refers to a vector comprising one or more polynucleotides of interest (or transgenes) that are flanked by AAV terminal repeat sequences (ITRs). Such AAV vectors can be replicated and packaged into infectious viral particles when present in a host cell that has been transfected with a vector encoding and expressing rep and cap gene products.

An "AAV virion" or "AAV viral particle" or "AAV vector particle" refers to a viral particle composed of at least one AAV capsid protein and an encapsidated polynucleotide AAV vector. If the particle comprises a heterologous polynucleotide (i.e., a polynucleotide other than a wild-type AAV genome such as a transgene to be delivered to a mammalian cell), it is typically referred to as an "AAV vector particle," or simply an "AAV vector." Thus, production of AAV vector particle necessarily includes production of AAV vector, as such a vector is contained within an AAV vector particle.

In some embodiments, the AAV is a replication-deficient parvovirus, the single-stranded DNA genome of which is about 4.7 kb in length including two 145 nucleotide inverted terminal repeat (ITRs). There are multiple serotypes of AAV. The nucleotide sequences of the genomes of the AAV serotypes are known. For example, the complete genome of AAV-1 is provided in GenBank Accession No. NC_002077; the complete genome of AAV-2 is provided in GenBank Accession No. NC_001401 and Srivastava et al., J. Virol., 45: 555-564 (1983); the complete genome of AAV-3 is provided in GenBank Accession No. NC_1829; the complete genome of AAV-4 is provided in GenBank Accession No. NC_001829; the AAV-5 genome is provided in GenBank Accession No. AF085716; the complete genome of AAV-6 is provided in GenBank Accession No. NC_00 1862; at least portions of AAV-7 and AAV-8 genomes are provided in GenBank Accession Nos. AX753246 and AX753249, respectively; the AAV-9 genome is provided in Gao et al., J. Virol., 78: 6381-6388 (2004); the AAV-10 genome is provided in Mol. Ther., 13(1): 67-76 (2006); and the AAV-11 genome is provided in Virology, 330(2): 375-383 (2004). The sequence of the AAV rh.74 genome is provided in U.S. Patent 9,434,928, incorporated herein by reference. US Patent No. 9,434,928 also provide the sequences of the capsid proteins and a self-complementary genome. In one aspect, the genome is a self-complementary genome. Cis-acting sequences directing viral DNA replication (rep), encapsidation/packaging and host cell chromosome integration are contained within the AAV ITRs. Three AAV promoters (named p5, pl9, and p40 for their relative map locations) drive the expression of the two AAV internal open reading frames encoding rep and cap genes. The two rep promoters (p5 and pi 9), coupled with the differential splicing of the single AAV intron (at nucleotides 2107 and 2227), result in the production of four rep proteins (rep 78, rep 68, rep 52, and rep 40) from the rep gene. Rep proteins possess multiple enzymatic properties that are ultimately responsible for replicating the viral genome. The cap gene is expressed from the p40 promoter and it encodes the three capsid proteins VP1, VP2, and VP3. Alternative splicing and non-consensus translational start sites are responsible for the production of the three related capsid proteins. A single consensus polyadenylation site is located at map position 95 of the AAV genome. The life cycle and genetics of AAV are reviewed in Muzyczka, Current Topics in Microbiology and Immunology, 158: 97-129 (1992).

AAV possesses unique features that make it attractive as a vector for delivering foreign DNA to cells, for example, in gene therapy. AAV infection of cells in culture is noncytopathic, and natural infection of humans and other animals is silent and asymptomatic. Moreover, AAV infects many mammalian cells allowing the possibility of targeting many different tissues in vivo. Moreover, AAV transduces slowly dividing and non-dividing cells, and can persist essentially for the lifetime of those cells as a transcriptionally active nuclear episome (extrachromosomal element). The AAV proviral genome is inserted as cloned DNA in plasmids, which makes construction of recombinant genomes feasible. Furthermore, because the signals directing AAV replication and genome encapsidation are contained within the ITRs of the AAV genome, some or all of the internal approximately 4.3 kb of the genome (encoding replication and structural capsid proteins, rep-cap) may be replaced with foreign DNA. To generate AAV vectors, the rep and cap proteins may be provided in trans. Another significant feature of AAV is that it is an extremely stable and hearty virus. It easily withstands the conditions used to inactivate Adenovirus (56° to 65°C for several hours), making cold preservation of AAV less critical. AAV may even be lyophilized. Finally, AAV-infected cells are not resistant to superinfection.

Multiple studies have demonstrated long-term (> 1.5 years) recombinant AAV- mediated protein expression in muscle. See, Clark et al., Hum Gene Ther, 8: 659-669 (1997); Kessler et al., Proc Nat. Acad Sc. USA, 93: 14082-14087 (1996); and Xiao et al., J Virol, 70: 8098-8108 (1996). See also, Chao et al., Mol Ther, 2: 619-623 (2000) and Chao et al., Mol Ther, 4: 217-222 (2001). Moreover, because muscle is highly vascularized, recombinant AAV transduction has resulted in the appearance of transgene products in the systemic circulation following intramuscular injection as described in Herzog et al., Proc Natl Acad Sci USA, 94: 5804-5809 (1997) and Murphy et al., Proc Natl Acad Sci USA, 94: 13921- 13926 (1997). Moreover, Lewis et al., J Virol, 76: 8769-8775 (2002) demonstrated that skeletal myofibers possess the necessary cellular factors for correct antibody glycosylation, folding, and secretion, indicating that muscle is capable of stable expression of secreted protein therapeutics. AAV DNA in the rAAV genomes may be from any AAV serotype for which a recombinant virus can be derived including, but not limited to, AAV serotypes AAV-1, AAV-2, AAV-3, AAV-4, AAV-5, AAV-6, AAV-7, AAV-8, AAV-9, AAV- 10, AAV-11, AAV- 12, AAV-13, AAV PHP.B, AAV rh74, and AAV-DJ. Production of pseudotyped rAAV is disclosed in, for example, WO 01/83692. Other types of rAAV variants, for example rAAV with capsid mutations, are also contemplated. See, for example, Marsic et al., Molecular Therapy, 22(11): 1900-1909 (2014). The nucleotide sequences of the genomes of various AAV serotypes are known in the art.

As used herein, the term "exterior" in reference to a viral capsid protein refers to the surface, domain, region, or terminal end of the capsid protein that is exterior-facing in an assembled viral capsid. The term "interior" in reference to a viral capsid protein refers to the surface, domain, region, or terminal end (amino-terminus end or carboxy terminus) of the capsid protein that is interior-facing in an assembled viral capsid. When used in reference to an assembled viral capsid, the term "interior" refers to the encapsidated space inside the viral capsid and the inward-facing surface of the capsid that is exposed to the enclosed space. The interior space is encapsidated by viral capsid proteins and may comprise nucleic acids such as the viral genome, viral proteins, proteins of the host or packaging cell, and any other components or factors packaged or encapsidated during replication, virion assembly, encapsidation, and/or packaging.

As used herein, the term "label" intends a directly or indirectly detectable compound or composition that is conjugated directly or indirectly to the composition to be detected, e.g., polynucleotide or protein such as an antibody so as to generate a "labeled" composition. The term also includes sequences conjugated to the polynucleotide that will provide a signal upon expression of the inserted sequences, such as green fluorescent protein (GFP) and the like. The label may be detectable by itself (e.g., radioisotope labels or fluorescent labels) or, in the case of an enzymatic label, may catalyze chemical alteration of a substrate compound or composition which is detectable. The labels can be suitable for small scale detection or more suitable for high-throughput screening. As such, suitable labels include, but are not limited to radioisotopes, fluorochromes, chemiluminescent compounds, dyes, and proteins, including enzymes. The label may be simply detected or it may be quantified. A response that is simply detected generally comprises a response whose existence merely is confirmed, whereas a response that is quantified generally comprises a response having a quantifiable (e.g., numerically reportable) value such as an intensity, polarization, and/or other property. In luminescence or fluorescence assays, the detectable response may be generated directly using a luminophore or fluorophore associated with an assay component actually involved in binding, or indirectly using a luminophore or fluorophore associated with another (e.g., reporter or indicator) component.

Examples of luminescent labels that produce signals include, but are not limited to bioluminescence and chemiluminescence. Detectable luminescence response generally comprises a change in, or an occurrence of, a luminescence signal. Suitable methods and luminophores for luminescently labeling assay components are known in the art and described for example in Haugland, Richard P. (1996) Handbook of Fluorescent Probes and Research Chemicals (6th ed.). Examples of luminescent probes include, but are not limited to, aequorin and luciferases.

Examples of suitable fluorescent labels include, but are not limited to, fluorescein, rhodamine, tetramethylrhodamine, eosin, erythrosin, coumarin, methyl-coumarins, pyrene, Malacite green, stilbene, Lucifer Yellow, Cascade Blue.TM., and Texas Red. Other suitable optical dyes are described in the Haugland, Richard P. (1996) Handbook of Fluorescent Probes and Research Chemicals (6th ed.).

In another aspect, the fluorescent label is functionalized to facilitate covalent attachment to a cellular component present in or on the surface of the cell or tissue such as a cell surface marker. Suitable functional groups, including, but not are limited to, isothiocyanate groups, amino groups, haloacetyl groups, maleimides, succinimidyl esters, and sulfonyl halides, all of which may be used to attach the fluorescent label to a second molecule. The choice of the functional group of the fluorescent label will depend on the site of attachment to either a linker, the agent, the marker, or the second labeling agent.

Attachment of the fluorescent label may be either directly to the cellular component or compound or alternatively, can by via a linker. Suitable binding pairs for use in indirectly linking the fluorescent label to the intermediate include, but are not limited to, antigens/antibodies, e.g., rhodamine/anti-rhodamine, biotin/avidin and biotin/strepavidin.

The phrase "solid support" refers to non-aqueous surfaces such as "culture plates," "gene chips," or "microarrays." Such gene chips or microarrays can be used for diagnostic and therapeutic purposes by a number of techniques known to one of skill in the art. In one technique, oligonucleotides are attached and arrayed on a gene chip for determining the DNA sequence by the hybridization approach, such as that outlined in U.S. Patent Nos. 6,025,136 and 6,018,041. The polynucleotides of this invention can be modified to probes, which in turn can be used for detection of a genetic sequence. Such techniques have been described, for example, in U.S. Patent Nos. 5,968,740 and 5,858,659. A probe also can be attached or affixed to an electrode surface for the electrochemical detection of nucleic acid sequences such as described by Kayem et al. U.S. Patent No. ,952,172 and by Kelley et al. (1999) Nucleic Acids Res. 27: 4830-4837.

A "composition" is intended to mean a combination of active polypeptide, polynucleotide or antibody and another compound or composition, inert (e.g., a detectable label) or active (e.g., a gene delivery vehicle).

A "pharmaceutical composition" is intended to include the combination of an active polypeptide, polynucleotide or antibody with a carrier, inert or active such as a solid support, making the composition suitable for diagnostic or therapeutic use in vitro, in vivo or ex vivo.

As used herein, the term "pharmaceutically acceptable carrier" encompasses any of the standard pharmaceutical carriers, such as a phosphate buffered saline solution, water, and emulsions, such as an oil/water or water/oil emulsion, and various types of wetting agents. The compositions also can include stabilizers and preservatives. For examples of carriers, stabilizers and adjuvants, see Martin (1975) Remington's Pharm. Sci., 15th Ed. (Mack Publ. Co., Easton).

As used herein, the term "cancer" comprises solid tumors and hematologic malignancies. Exemplary solid tumors include, but are not limited to, bladder cancer, bone cancer, brain cancer (e.g., glioblastoma), breast cancer, colorectal cancer, esophageal cancer, eye cancer, head and neck cancer, kidney cancer, lung cancer, melanoma, mesothelioma, ovarian cancer, pancreatic cancer, prostate cancer, or stomach cancer. Exemplary hematologic malignancy include, but are not limited to, acute myeloid leukemia (AML), chronic lymphocytic leukemia (CLL), small lymphocytic lymphoma (SLL), follicular lymphoma (FL), diffuse large B-cell lymphoma (DLBCL), mantle cell lymphoma (MCL), Waldenstrom's macroglobulinemia, multiple myeloma, extranodal marginal zone B cell lymphoma, nodal marginal zone B cell lymphoma, Burkitt's lymphoma, non-Burkitt high grade B cell lymphoma, primary mediastinal B-cell lymphoma (PMBL), immunoblastic large cell lymphoma, precursor B-lymphoblastic lymphoma, B cell prolymphocytic leukemia, lymphoplasmacytic lymphoma, splenic marginal zone lymphoma, plasma cell myeloma, plasmacytoma, mediastinal (thymic) large B cell lymphoma, intravascular large B cell lymphoma, primary effusion lymphoma, or lymphomatoid granulomatosis. In some instances, the cancer is a metastatic cancer (e.g., a metastatic solid tumor or metastatic hematologic malignancy). In some instances, the cancer is a relapsed or refractory cancer (e.g., a relapsed or refractory solid tumor or a relapsed or refractory hematologic malignancy).

In some embodiments, the cancer is characterized with an upregulated expression of fibroblast activation protein (FAP) (in which the amino acid sequence of the human FAP is disclosed in GenPept Accession Number I38593, accessed on February 10, 2020). FAP, also known as FAP-alpha and prolyl endopeptidase FAP, is a membrane-bound glycoprotein and part of the dipeptidyl peptidase (DPP) family. FAP has both post-proline exopeptidase activity and gelatinase activity. In some instances, cancers that are characterized with an upregulated expression of FAP (FAP-positive cancers) include, but are not limited to, bone cancer, brain cancer, breast cancer, colorectal cancer, esophageal cancer, gastric cancer, liver cancer, lung cancer, oral cancer, ovarian cancer, pancreatic cancer, parathyroid cancer, and renal cancer. In some instances, the FAP-positive cancers comprise a high level of fibrosis. In some instances, this level is compared to an equivalent cancer in which FAP is not upregulated. In additional instances, this level is compared to a fibrosis level of a normal subject.

In some cases, a dimert described herein binds to an extracellular portion of FAP-alpha. In some instances, the dimert binds to a human FAP-alpha (e.g., to an extracellular portion of FAP-alpha with the GenPept Accession Number I38593, or an equivalent thereof). In some cases, the dimert binds to FAP-alpha and an immune cell target, e.g., a cell surface polypeptide expressed on a T cell or a NK cell. In some cases, the dimert binds to FAP-alpha and optionally to an immune cell target (e.g., a cell surface polypeptide expressed on a T cell or a NK cell) for the treatment of a FAP-positive cancer, optionally a pancreatic cancer, further optionally a FAP-positive cancer characterized with a high level of fibrosis.

In some embodiments, the cancer is characterized with the expression and/or upregulation of B-cell maturation antigen (BCMA) (also known as tumor necrosis factor receptor superfamily member 17, TNFRSF17, and BCM), a cell surface receptor of the TNF receptor superfamily. In some instances, the amino acid sequence of the human BCMA is disclosed in GenPept Accession Number BAB60895.1 (accessed on February 10, 2020). In some cases, the cancer characterized with the expression and/or upregulation of BCMA is myeloma (or multiple myeloma).

In some cases, a dimert described herein binds to an extracellular portion of BCMA. In some instances, the dimert binds to a human BCMA (e.g., to an extracellular portion of BCMA with the GenPept Accession Number BAB60895.1, or an equivalent thereof). In some cases, the dimert binds to BCMA and an immune cell target, e.g., a cell surface polypeptide expressed on a T cell or a NK cell. In some cases, the dimert binds to BCMA and optionally to an immune cell target (e.g., a cell surface polypeptide expressed on a T cell or a NK cell) for the treatment of a myeloma.

In some embodiments, the cancer is characterized with an expression or upregulation of an epidermal growth factor receptor (EGFR) mutant, e.g., EGFR variant III (EGFRvIII). The EGFRvIII refers to a mutation of EGFR that comprises a deletion of exons 2-7 of the *EGFR* gene. In some instances, cancers that is characterized with an expression or upregulation of EGFRvIII (EGFRvIII-positive cancer) include, but are not limited to, glioblastoma, bladder cancer, breast cancer, colorectal cancer, esophageal cancer, head and neck squamous cell carcinoma (HNSCC), lung cancer, melanoma, ovarian cancer, a peripheral nerve sheath tumor (PNST),prostate cancer, sarcoma, and thyroid cancer.

In some cases, a dimert described herein binds to an extracellular portion of EGFRvIII. In some instances, the dimert binds to an extracellular portion of a human EGFRvIII. In some cases, the dimert binds to EGFRvIII and an immune cell target, e.g., a cell surface polypeptide expressed on a T cell or a NK cell. In some cases, the dimert binds to EGFRvIII and optionally to an immune cell target (e.g., a cell surface polypeptide expressed on a T cell or a NK cell) for the treatment of an EGFRvIII-positive cancer, optionally glioblastoma.

In some embodiments, the cancer is characterized with an upregulation of human epidermal growth factor receptor 2 (HER2), also known as HER2/neu, receptor tyrosine-protein kinase erbB-2, CD340, and ERBB2. In some instances, the amino acid sequence of the human HER2 is disclosed in GenPept Accession Number NP-004439.2 (accessed on February 10, 2020). In some instances, HER2-positive cancers include, but are not limited to, breast cancer, ovarian cancer, gastric cancer, colorectal cancer, pancreatic cancer, and endometrial cancer.

In some cases, a dimert described herein binds to an extracellular portion of HER2. In some instances, the dimert binds to an extracellular portion of a human HER2 (e.g., comprising an amino acid sequence as set forth in GenPept Accession Number NP-004439.2, or an equivalent thereof). In some cases, the dimert binds to HER2 and an immune cell target, e.g., a cell surface polypeptide expressed on a T cell or a NK cell. In some cases, the dimert binds to HER2 and optionally to an immune cell target (e.g., a cell surface polypeptide expressed on a T cell or a NK cell) for the treatment of a HER2-positive cancer, optionally breast cancer.

In some embodiments, the cancer is characterized with an upregulation of CD123, also known as interleukin-3receptor or IL-3RA. In some instances, the amino acid sequence of the human CD123 is disclosed in GenPept Accession Number NP_002174.1 (accessed on February 10, 2020). In some instances, CD123-positive cancers include, but are not limited to, acute myeloid leukemia (AML), acute lymphocytic leukemia (ALL), blastic plasmocytoid dendritic cell neoplasm, and hairy cell leukemia.

In some cases, a dimert described herein binds to an extracellular portion of CD123. In some instances, the dimert binds to an extracellular portion of a human CD123 (e.g., comprising an amino acid sequence as set forth in GenPept Accession Number NP_002174.1, or an equivalent thereof). In some cases, the dimert binds to CD123 and an immune cell target, e.g., a cell surface polypeptide expressed on a T cell or a NK cell. In some cases, the dimert binds to CD123 and optionally to an immune cell target (e.g., a cell surface polypeptide expressed on a T cell or a NK cell) for the treatment of a CD123-positive cancer, optionally AML.

In some embodiments, the cancer is characterized with an upregulation of CD38, also known as ADP-ribosyl cyclase 1 or ADPRC 1. In some instances, the amino acid sequence of the human CD38 is disclosed in GenPept Accession Number BAA18966.1 (accessed on February 10, 2020). In some instances, CD38-positive cancers include, but are not limited to, multiple myeloma, acute myeloid leukemia, prostate cancer, and lung cancer.

In some cases, a dimert described herein binds to an extracellular portion of CD38. In some instances, the dimert binds to an extracellular portion of a human CD38 (e.g., comprising an amino acid sequence as set forth in GenPept Accession Number BAA18966.1, or an equivalent thereof). In some cases, the dimert binds to CD38 and an immune cell target, e.g., a cell surface polypeptide expressed on a T cell or a NK cell. In some cases, the dimert binds to CD38 and optionally to an immune cell target (e.g., a cell surface polypeptide expressed on a T cell or a NK cell) for the treatment of a CD38-positive cancer, optionally AML.

In some embodiments, the cancer is characterized with an upregulation of mesothelin (also known as MSLN). In some instances, the amino acid sequence of the human mesothelin is disclosed in GenPept Accession Number AAV87530.1 (accessed on February 10, 2020). In some instances, mesothelin-positive cancers include, but are not limited to, mesothelioma, pancreatic cancer, ovarian cancer, endometrial cancer, biliary cancer, gastric cancer, lung adenocarcinoma, and pediatric acute myeloid leukemia.

In some cases, a dimert described herein binds to an extracellular portion of mesothelin. In some instances, the dimert binds to an extracellular portion of a human mesothelin (e.g., comprising an amino acid sequence as set forth in GenPept Accession Number AAV87530.1, or an equivalent thereof). In some cases, the dimert binds to mesothelin and an immune cell target, e.g., a cell surface polypeptide expressed on a T cell or a NK cell. In some cases, the dimert binds to mesothelin and optionally to an immune cell target (e.g., a cell surface polypeptide expressed on a T cell or a NK cell) for the treatment of a mesothelin-positive cancer, optionally mesothelioma.

In some embodiments, the cancer is characterized with an upregulation of interleukin-13 receptor alpha (IL13Ralpha). In some instances, the IL13Ralpha comprises IL13R alpha 1 (IL13R α1) and IL13R alpha 2 (IL13R α2). In some instances, the amino acid sequence of the human IL13R α1 is disclosed in GenPept Accession Number P78552.1 (accessed on February 10, 2020). In some instances, the amino acid sequence of the human IL13R α2 is disclosed in GenPept Accession Number Q14627.1 (accessed on February 10, 2020). In some instances, an IL13Ralpha-positive cancers include, but are not limited to, brain cancer (e.g., glioblastoma) and renal cell carcinoma (RCC).

In some cases, a dimert described herein binds to an extracellular portion of IL13Ralpha. In some instances, the dimert binds to an extracellular portion of a human IL13Ralpha (e.g., comprising an amino acid sequence as set forth in GenPept Accession Number P78552.1, Q14627.1, or an equivalent thereof). In some cases, the dimert binds to IL13Ralpha and an immune cell target, e.g., a cell surface polypeptide expressed on a T cell or a NK cell. In some cases, the dimert binds to IL13Ralpha and optionally to an immune cell target (e.g., a cell surface polypeptide expressed on a T cell or a NK cell) for the treatment of an IL13Ralpha-positive cancer, optionally a brain cancer.

In some embodiments, the cancer is characterized with an upregulation of B7-H3, also known as CD276, an immune checkpoint member. In some instances, the amino acid sequence of the human B7-H3 is disclosed in GenPept Accession Number CAE47548.1 (accessed on February 10, 2020). In some instances, a B7-H3-positive cancers include, but are not limited to, lung cancer (e.g., non-small-cell lung cancer), breast cancer, prostate cancer, renal cell carcinoma, brain cancer, pancreatic cancer, kidney cancer, gastric cancer, ovarian cancer, melanoma, and thyroid cancer.

In some cases, a dimert described herein binds to an extracellular portion of B7-H3. In some instances, the dimert binds to an extracellular portion of a human B7-H3 (e.g., comprising an amino acid sequence as set forth in GenPept Accession Number CAE47548.1, or an equivalent thereof). In some cases, the dimert binds to B7-H3 and a T-cell or NK cell target, e.g., a cell surface polypeptide expressed on a T cell or a NK cell. In some cases, the dimert binds to B7-H3 and optionally to a T-cell or NK cell target (e.g., a cell surface polypeptide expressed on a T cell or a NK cell) for the treatment of a B7-H3-positive cancer, optionally lung cancer (e.g., non-small-cell lung cancer), breast cancer, prostate cancer, renal cell carcinoma, brain cancer, pancreatic cancer, kidney cancer, gastric cancer, ovarian cancer, melanoma, or thyroid cancer.

In some embodiments, the cancer is characterized with an upregulation of receptor tyrosine kinase like orphan receptor 1 (ROR1), also known as neurotrophic tyrosine kinase, receptor-related 1 or NTRKR1. In some instances, the amino acid sequence of the human ROR1 is disclosed in GenPept Accession Number NP_005003 (accessed on February 10, 2020). In some instances, a ROR1-positive cancers include, but are not limited to, breast cancer, lung cancer, stomach cancer, ovarian cancer, chronic lymphocytic leukemia (CLL), and acute lymphocytic leukemia (ALL).

In some cases, a dimert described herein binds to an extracellular portion of ROR1. In some instances, the dimert binds to an extracellular portion of a human ROR1 (e.g., comprising an amino acid sequence as set forth in GenPept Accession Number NP_005003, or an equivalent thereof). In some cases, the dimert binds to ROR1 and an immune cell target, e.g., a cell surface polypeptide expressed on a T cell or a NK cell. In some cases, the dimert binds to ROR1 and optionally to an immune cell target (e.g., a cell surface polypeptide expressed on a T cell or a NK cell) for the treatment of a ROR1-positive cancer, optionally breast cancer, lung cancer, stomach cancer, ovarian cancer, CLL, or ALL.

In some embodiments, the cancer is characterized with an upregulation of ephrin type-A receptor 2 (EphA2), also known as EPH receptor A2, tyrosine-protein kinase receptor ECK, orepithelial cell receptor protein tyrosine kinase. In some instances, the amino acid sequence of the human EphA2 is disclosed in GenPept Accession Number NP_004422.2 (accessed on February 10, 2020). In some instances, an EphA2-positive cancers include, but are not limited to, breast cancer, bladder cancer, prostate cancer, skin cancer, lung cancer, ovarian cancer, brain cancer, mesothelioma, thyroid cancer, colorectal cancer, gastric cancer, esophageal cancer, endometrial cancer, cervical cancer, pancreatic, melanoma, renal cell cancer, and liver cancer.

In some cases, a dimert described herein binds to an extracellular portion of EphA2. In some instances, the dimert binds to an extracellular portion of a human EphA2 (e.g., comprising an amino acid sequence as set forth in GenPept Accession Number NP_004422.2, or an equivalent thereof). In some cases, the dimert binds to EphA2 and an immune cell target, e.g., a cell surface polypeptide expressed on a T cell or a NK cell. In some cases, the dimert binds to EphA2 and optionally to an immune cell target (e.g., a cell surface polypeptide expressed on a T cell or a NK cell) for the treatment of an EphA2-positive cancer, optionally breast cancer, bladder cancer, prostate cancer, skin cancer, lung cancer, ovarian cancer, brain cancer, mesothelioma, thyroid cancer, colorectal cancer, gastric cancer, esophageal cancer, endometrial cancer, cervical cancer, pancreatic, melanoma, renal cell cancer, or liver cancer.

In some embodiments, the cancer is a B-cell leukemia or a B-cell lymphoma. Exemplary B cell leukemia include B-cell chronic lymphocytic leukemia (or B-cell small lymphocytic lymphoma); acute lymphoblastic leukemia, mature B-cell type; B-cell prolymphocytic leukemia; precursor B lymphoblastic leukemia; and hairy cell leukemia. In some instances, the B-cell leukemia, the B-cell lymphoma, or a combination thereof is characterized with elevated expression of CD20 and/or CD22 on B cells. In some instances, a dimert described herein binds to CD20 or CD22. In some instances, the dimert binds to CD20 expressed on a B cell. In some instances, the dimert binds to CD22 expressed on a B cell. In some cases, the dimert further binds to another cell target, e.g., a cell surface polypeptide expressed on a cancer cell or a cell surface polypeptide expressed on a T cell or a NK cell. In some cases, the dimert binds to CD20 or CD22 and optionally to another cell target (e.g., a cell surface polypeptide expressed on a cancer cell or a cell surface polypeptide expressed on a T cell or a NK cell) for the treatment of B-cell leukemia or a B-cell lymphoma.

As used herein, "first-line therapy" comprises a primary treatment for a subject, optionally a subject with a cancer. In some instances under a cancer setting, the cancer is a primary cancer. In other instances, the cancer is a metastatic or recurrent cancer. In some cases, the first-line therapy comprises chemotherapy. In other cases, the first-line treatment comprises radiation therapy. A skilled artisan would readily understand that different first-line treatments may be applicable to different type of cancers.

As used herein, a second-line therapy encompasses treatments that are utilized after the primary or first-line treatment stops. A third-line therapy, a fourth-line therapy, or a fifth-line therapy encompass subsequent treatments. As indicated by the naming convention, a third-line therapy encompass a treatment course upon which a primary and second-line therapy have stopped.

A "subject" of diagnosis or treatment is a cell or an animal such as a mammal, or a human. A subject is not limited to a specific species and includes non-human animals subject to diagnosis or treatment and are those subject to infections or animal models, for example, simians, murines, such as, rats, mice, chinchilla, canine, such as dogs, leporids, such as rabbits, livestock, sport animals, and pets. Human patients are included within the term as well.

The term "tissue" is used herein to refer to tissue of a living or deceased organism or any tissue derived from or designed to mimic a living or deceased organism. The tissue may be healthy, diseased, and/or have genetic mutations. The biological tissue may include any single tissue (e.g., a collection of cells that may be interconnected) or a group of tissues making up an organ or part or region of the body of an organism. The tissue may comprise a homogeneous cellular material or it may be a composite structure such as that found in regions of the body including the thorax which for instance can include lung tissue, skeletal tissue, and/or muscle tissue. Exemplary tissues include, but are not limited to those derived from liver, lung, thyroid, skin, pancreas, blood vessels, bladder, kidneys, brain, biliary tree, duodenum, abdominal aorta, iliac vein, heart and intestines, including any combination thereof.

As used herein, the term "specifically binds" or "specifically binds to" or "specifically target" is meant a polypeptide or fragment thereof that recognizes and binds a biological molecule of interest (e.g., a polypeptide), but which does not substantially recognize and bind other molecules in a sample, for example, a biological sample, which includes or expresses a tumor antigen.

As used herein, "treating" or "treatment" of a disease in a subject refers to (1) preventing the symptoms or disease from occurring in a subject that is predisposed or does not yet display symptoms of the disease; (2) inhibiting the disease or arresting its development; or (3) ameliorating or causing regression of the disease or the symptoms of the disease. As understood in the art, "treatment" is an approach for obtaining beneficial or desired results, including clinical results. For the purposes of the present technology, beneficial or desired results can include one or more, but are not limited to, alleviation or amelioration of one or more symptoms, diminishment of extent of a condition (including a disease), stabilized (i.e., not worsening) state of a condition (including disease), delay or slowing of condition (including disease), progression, amelioration or palliation of the condition (including disease), states and remission (whether partial or total), whether detectable or undetectable. In one aspect, the term "treatment" excludes prevention.

As used herein the term "effective amount" intends to mean a quantity sufficient to achieve a desired effect. In the context of therapeutic or prophylactic applications, the effective amount will depend on the type and severity of the condition at issue and the characteristics of the individual subject, such as general health, age, sex, body weight, and tolerance to pharmaceutical compositions. In the context of gene therapy, in some embodiments the effective amount is the amount sufficient to result in regaining part or full function of a gene that is deficient in a subject. In other embodiments, the effective amount of a recombinant polynucleotide, vector or an AAV viral particle is the amount sufficient to result in expression of a gene in a subject. In some embodiments, the effective amount is the amount required to increase galactose metabolism in a subject in need thereof. The skilled artisan will be able to determine appropriate amounts depending on these and other factors.

In some embodiments the effective amount will depend on the size and nature of the application in question. It will also depend on the nature and sensitivity of the target subject and the methods in use. The skilled artisan will be able to determine the effective amount based on these and other considerations. The effective amount may comprise one or more administrations of a composition depending on the embodiment.

As used herein, the term "administer" or "administration" intends to mean delivery of a substance to a subject such as an animal or human. Administration can be effected in one dose, continuously or intermittently throughout the course of treatment. Methods of determining the most effective means and dosage of administration are known to those of skill in the art and will vary with the composition used for therapy, the purpose of the therapy, as well as the age, health or gender of the subject being treated. Single or multiple administrations can be carried out with the dose level and pattern being selected by the treating physician or in the case of pets and animals, treating veterinarian. Suitable dosage formulations and methods of administering the agents are known in the art. Route of administration can also be determined and method of determining the most effective route of administration are known to those of skill in the art and will vary with the composition used for treatment, the purpose of the treatment, the health condition or disease stage of the subject being treated and the target cell or tissue. Non-limiting examples of route of administration include intravenous, intra-arterial, intramuscular, intracardiac, intrathecal, subventricular, epidural, intracerebral, intracerebroventricular, sub-retinal, intravitreal, intraarticular, intraocular, intraperitoneal, intrauterine, intradermal, subcutaneous, transdermal, transmucosal, and inhalation.

### Modes for Carrying Out the Disclosure

Cancer is the second leading cause of death globally with an estimated 9.6 million deaths in 2018. There are many different types of cancer therapy in clinical development and on market, e.g., immunotherapy, hormone therapy, targeted drug therapy, adoptive cell therapy, and chemotherapy. Although advances are made in many therapeutic arenas, challenges still exist relating to factors such as half-life and toxicity that influence therapeutic efficacy. For example, oncolytic virus-based therapy provides targeted delivery of payloads to tumor microenvironment of interest. However, these oncolytic viruses express their payloads within the tumor cells and induce cell-killing effect, thereby limiting the expression of payloads to a few days at most, often limited to several hours. As such, to obtain a sustained and prolonged therapeutic effect, multiple administrations are needed, which increase toxicity, leading to adverse immune responses. Adoptive cell therapy such as chimeric antigen receptor (CAR)-T cell therapy provides a personalized treatment option to a subject suffering from cancer. However, some of the most common side effects for CAR T-cell therapy include cytokine release syndrome; neurologic events such as encephalopathy, aphasia, seizures, and lose of balance; neutropenia; and anemia. Furthermore, preparation of CAR-T cells involves several weeks of culturing and expansion prior to administration, time that can be critical in particular for a patient in an advanced stage of cancer.

Disclosed herein, in certain embodiments, are methods of delivering a therapeutic transgene (e.g., a dimert disclosed herein) with use of a gene therapy vector. In some embodiments, the gene therapy vector provides a stable sustained expression of the therapeutic transgene (e.g., the dimert). In additional embodiments, the gene therapy vector provides a constitutive expression. In further embodiments, the gene therapy vector provides a regulated expression. In some cases, the gene therapy vector is transduced in normal cells (e.g., in organ cells such as liver cells or in muscle cells). In some cases, a single administration of the gene therapy vector is sufficient to induce a stable sustained expression of the therapeutic transgene (e.g., the dimert). In additional cases, the gene therapy vector provides a continuous, long-term expression of the therapeutic transgene (e.g., the dimert) which provides long-term pressure on cancer cells.

In certain embodiments, disclosed herein is a method of delivering a therapeutic transgene (e.g., a dimert disclosed herein) with use of a TransJoin. In some instance, the TransJoin provides a constitutive expression of the therapeutic transgene (e.g., the dimert). In some instances, the TransJoin provides a sustained, stable, long-term expression of the therapeutic transgene (e.g., the dimert). In some cases, the long-term expression comprises about one week, two weeks, three weeks, four weeks, one month, 2 months, 3 months, 4 months, 5 months, 6 months, 8 months, 10 months, one year, or more. In some cases, the TransJoin is transduced in normal cells (e.g., in organ cells such as liver cells or in muscle cells). In some cases, a single administration of the TransJoin is sufficient to induce a stable sustained expression of the therapeutic transgene (e.g., the dimert). In additional cases, the TransJoin provides a continuous, long-term expression of the therapeutic transgene (e.g., the dimert) which provides long-term pressure on cancer cells.

In certain embodiments, disclosed herein is a novel method that activates transgene expression (e.g., a dimert disclosed herein) which can be used as a gene therapy platform for a regulated expression, e.g., short-term gene expression (e.g., weeks to months, further optionally one week, two weeks, three weeks, four weeks, one month, 2 months, 3 months, 4 months, 5 months, 6 months, 8 months, or more). In some instances, the method utilizes a TransSkip. In one aspect, transgene expression is placed under control of a drug or an external agent (e.g., an OncoSkip), such that administration of the drug or the agent modulates (activates or inactivates) gene expression. On the other hand, lack of administration or withdrawal of the drug or the agent reverts to the original status of gene expression before the administration. For example, when the drug or the agent can activate the gene expression, if there are side effects or if expression of the transgene is no longer needed, withdrawal of the administration can inactive the gene expression, thereby minimizing the side effects, if any. In some instances, the TransSkip is transduced in normal cells (e.g., in organ cells such as liver cells or in muscle cells). In some cases, a single administration of the TransSkip is sufficient to induce a stable sustained expression of the therapeutic transgene (e.g., the dimert). In additional cases, the TransSkip provides a regulated but continuous expression of the therapeutic transgene (e.g., the dimert) which provides long-term pressure on cancer cells.

Exon-skipping is a technology used to treat certain genetic diseases in which a short region of a gene is defective. The DNA mutations result in a damaged protein either because the wrong amino acids appear in the protein, or they generate a stop mutation resulting a truncated protein, or they change the reading frame generating both. Because mammalian genes are typically encoded in exons, meaning they are split into multiple gene segments (exons) that get spliced together during processing of mRNA, the DNA mutations (changes in or deletions of base pairs) that underlie many diseases are contained within a single exon. If that exon can be skipped over, and not included in the final spliced mRNA, then the mutated region will not be included in the final protein. While the protein will be shorter and may be missing some parts, it will be still be "in frame" and might retain some of its function.

For example, Duchenne muscular dystrophy (DMD) and Becker muscular dystrophy (BMD) are the most common childhood forms of muscular dystrophy, which are caused by genetic defects in the DMD gene encoding dystrophin, a muscle protein that is required for interactions between the cytoskeleton and the extracellular matrix to maintain muscle fiber stability during contraction. DMD mutations in the dystrophin gene are characterized by frame shifting insertions or deletions or nonsense point mutations, resulting in the absence of functional dystrophin. BMD mutations in general keep the reading frame intact, allowing synthesis of a partly functional dystrophin. An exon-skipping based therapy resulted in transforming the out-of-frame mutations present in the DMD patients to in-frame mutations that encodes partially functional dystrophin. In 2016 the FDA approved the first exon-skipping drug, Eteplirsen^{™} (Sarepta Therapeutics) for Duchenne muscular dystrophy with mutations in exon 51 of the dystrophin gene. When the drug (which is a modified short stretch of DNA also called an oligo) is administered, exon 51 is "skipped" to restore a near full-length and more functional protein. Similar technology is being developed to skip other dystrophin exons as well as exons in other disease-causing genes.

In contrast to exon-skipping, the present disclosure exploits transgene activation in gene therapy applications. Most if not all gene therapy approaches currently use the complimentary (cDNA) sequence of a gene; that is, the genetic sequence of the transgene is only the coding sequence (only the exons), and does not include any intervening (intron) sequences. Thus, the gene does not undergo any RNA splicing.

This disclosure and technology utilizes intervening sequences that contain splice donor and acceptor sites and RNA spliceosome binding sites to force a transgene to undergo the normal exon splicing. Thus, a "reverse engineered" (artificial) exon-intron-exon gene structure in a transgene is provided, which undergoes splicing when it is expressed in a target cell. However, the splicing is regulated based on the principles of exon-skipping. In one aspect, a purposefully inserted exon that contains a stop codon in the middle of a gene regulates gene expression, i.e., only when that exon is skipped will a normal, functional transgene be expressed by the artificial structure. In one embodiment, the functional transgene encodes an antibody. In another embodiment, the antibody is a bispecific or trispecific antibody (e.g., a dimert). In another embodiment, the antibody (e.g., the dimert) is a bispecific T cell engager (BiTE), a bispecific NK cell engager (BiKE), a trispecific T cell engager (TriTE), or a trispecific NK cell engager (TriKE).

In a further aspect, the extent and type of splicing is modified by the cell type, as many genes normally undergo alternative splicing that sometimes varies by cell type. In addition, splicing is sometimes altered in certain cancer cells (some exons of certain genes may be included or excluded in normal versus cancer cells). This technology can leverage these features to achieve transgene control that differs in normal versus cancer cells. Also, the antibodies, e.g., bispecific or trispecific antibodies, encoded by the functional transgenes can be used to treat cancer. Thus, in one aspect, modulation of splicing of the transgene in this present disclosure provides a method of treating cancer.

### Construct Embodiments

In certain embodiments, provided herein is a polynucleotide or a vector comprising, or alternatively consisting essentially of, or yet further consisting of: (a) a first polynucleotide sequence comprising a first portion of an open reading frame encoding a first polypeptide; (b) a second polynucleotide sequence comprising a second portion of the open reading frame encoding the first polypeptide; (c) a third polynucleotide sequence encoding a second polypeptide; and (d) a gene regulation polynucleotide sequence located between the first polynucleotide and the second polynucleotide. In some instances, the first polypeptide binds to a surface polypeptide (e.g., a surface receptor) of a first target cell and the second polypeptide binds to a surface polypeptide (e.g., a surface receptor) of a second target cell. In some instances, the first target cell and the second target cell are different. For example, the first target cell can be a tumor cell and the second target cell can be an immune cell. In a second example, the first target cell can be an immune cell and the second target cell can be a tumor cell. In a third example, the first target cell can be a first immune cell and the second target cell can be a second immune cell and the first immune cell is a different cell type than the second immune cell. In a fourth example, the first target cell is a first cancer cell and the second target cell is a second cancer cell, and the first cancer cell and the second cancer cell are from the same type of cancer, e.g., related to the same genetic defect or alternatively of the same tissue type.

In some embodiments, the first polypeptide is a first antibody or its binding fragment thereof and the second polypeptide is a second antibody or its binding fragment thereof. In some instances, provided herein is a polynucleotide or a vector comprising, or alternatively consisting essentially of, or yet further consisting of: (a) a first polynucleotide sequence comprising a first portion of an open reading frame encoding a first antibody or an antigen-binding fragment thereof; (b) a second polynucleotide sequence comprising a second portion of the open reading frame encoding the first antibody or an antigen-binding fragment thereof; (c) a third polynucleotide sequence encoding a second antibody or an antigen-binding fragment thereof; and (d) a gene regulation polynucleotide sequence located between the first polynucleotide and the second polynucleotide. Also provided is the compliment of the polynucleotide. In one aspect, the polynucleotide, its compliment, and/or the vector is detectably labeled. In some instances, the first antibody binds to a first target and the second antibody binds to a second target. In some cases, the first target is a surface polypeptide (e.g., a surface receptor) on a first cell and the second target is a surface polypeptide (e.g., a surface receptor) of a second cell. In some instances, the first target cell and the second target cell are different. For example, the first target cell can be a tumor cell and the second target cell can be an immune cell. In a second example, the first target cell can be an immune cell and the second target cell can be a tumor cell. In a third example, the first target cell can be a first immune cell and the second target cell can be a second immune cell and the first immune cell is a different cell type than the second immune cell. In a fourth example, the first target cell is a first cancer cell and the second target cell is a second cancer cell, and the first cancer cell and the second cancer cell are from the same type of cancer, e.g., related to the same genetic defect or alternatively of the same tissue type. In some cases, the first target is a first epitope and the second target is a second epitope and both epitopes are present on the same antigen. In some cases, the first and second antibodies have different amino acid sequences. In one aspect, the polynucleotide is contained within a gene expression vector, non-limiting examples of such include plasmids, DNA viral vectors, or gene delivery vehicles.

In some embodiments, also disclosed herein is a vector for use in a gene therapy comprising: a first polynucleotide sequence encoding a first antibody or its antigen-binding fragment thereof; and a second polynucleotide sequence encoding a second antibody or its antigen-binding fragment thereof. Also provided is the compliment of the polynucleotide. In one aspect, the polynucleotide, its compliment, and/or the vector is detectably labeled. In some instances, the first antibody binds to a first target and the second antibody binds to a second target. In some cases, the first target is a surface polypeptide (e.g., a surface receptor) on a first cell and the second target is a surface polypeptide (e.g., a surface receptor) of a second cell. In some instances, the first target cell and the second target cell are different. For example, the first target cell can be a tumor cell and the second target cell can be an immune cell. In a second example, the first target cell can be an immune cell and the second target cell can be a tumor cell. In a third example, the first target cell can be a first immune cell and the second target cell can be a second immune cell and the first immune cell is a different cell type than the second immune cell. In a fourth example, the first target cell is a first cancer cell and the second target cell is a second cancer cell, and the first cancer cell and the second cancer cell are from the same type of cancer, e.g., related to the same genetic defect or alternatively of the same tissue type. In some cases, the first target is a first epitope and the second target is a second epitope and both epitopes are present on the same antigen. In some cases, the first and second antibodies have different amino acid sequences.

In a further aspect, the gene regulation polynucleotide sequence comprises a splice donor site, an upstream intron, an exon containing stop codon sequences in all three reading frames, a downstream intron, and a splice acceptor site. In a further aspect, the gene-regulation polynucleotide sequence comprises one or more of a binding sequence for an antisense oligonucleotide. In a further aspect, the antisense oligonucleotide is morpholino. In a further aspect, the binding sequence for the morpholino oligonucleotide comprises a polynucleotide sequence at least 95% identical to SEQ ID NO: 24 (AATATGATCCAACAATAGAGGTAAATCTTG) or SEQ ID NO. 25 (GATCCAACAATAGAGGTAAATCTTGTTTTA), or alternatively with at least 96% or alternatively at least 97%, or alternatively 98%, or alternatively at least 99% identity thereto. In one embodiment, the morpholino oligonucleotide comprises a polynucleotide sequence at least 95% identical to SEQ ID NO. 27 (CAAGATTTACCTCTATTGTTGGATCATATT) or SEQ ID NO. 28 (TAAAACAAGATTTACCTCTATTGTTGGATC), or alternatively with at least 96% or alternatively at least 97%, or alternatively 98%, or alternatively at least 99% identity to each thereof. The splice donor site and the splice acceptor sites are well known in the art. A person with ordinary skill in the art would know the sequences, e.g., consensus sequences, for the splice donor site and the splice acceptor site. An exemplary splice site consensus sequence for U2 introns can include the 5' splice site MAG-GTRAGT, in which M is A or C; R is A or G, the underlined nucleotides denote that "GT" is invariant; and the dash "-" denotes the splice site. The 3' splice site for the U2 intron can be CAG-G, in which the underlined nucleotides denote that "AG" is invariant; and the dash "-" denotes the splice site. Additional examples of consensus splice site sequences include, but are not limited to the following:
p53 exon 10 5' splice site (donor): CAG-gtgagt, in which the dash "-" denotes the splice site;
Brd2 exon 3 5'ss: AAG-gtgagt, in which the dash "-" denotes the splice site;
BRCA1 exon 22 5' splice site: CAG-gtaagt, in which the dash "-" denotes the splice site;
SMN1 exon 1 5'ss: CAG-gtgagg, in which the dash "-" denotes the splice site;
BRD2 3' splice site Acceptor intron1 (lower case)/exon 2 (upper case): cccatctttacag-GCTCCC, in which the dash "-" denotes the splice site;
BCL-X 3' splice acceptor intron2 (lower case)/exon 3 (upper case): tctctccctgcag-GATACT, in which the dash "-" denotes the splice site;
fibronectin 3' splice acceptor intron28 (lower case)/exon29 (upper case): ctttttcatacag-GAGGAA, in which the dash "-" denotes the splice site; and
survivin 3' splice acceptor intron2 (lower case)/exon3 (upper case): tetttatttccagGCAAAG, in which the dash "-" denotes the splice site.

In some embodiments, a splice site consensus sequence is obtained from //science.umd.edu/labs/mount/RNAinfo/matrices.html.

In some embodiments, the stop codon comprises an oligonucleotide of the group of: TAA, TAG, or TGA. In a further aspect, the stop codon sequence comprises a polynucleotide sequence of TAAxTAGxTGAxTAGxTAAxTGAx (SEQ ID NO. 1), wherein x is any nucleotide or alternatively, the stop codon sequence comprises a polynucleotide sequence of TAATTAGTTGATTAGTTAATTGAT (SEQ ID NO. 2). In a yet further aspect, the gene regulation polynucleotide comprises a polynucleotide sequence at least 95% identical to SEQ ID NO. 2, or alternatively at least 96% or alternatively at least 97%, or alternatively 98%, or alternatively at least 99% identity to SEQ ID NO. 2.

In further embodiments, the first antibody or the antigen-binding fragment thereof specifically binds to an activating antigen on an immune effector cell and the second antibody or its antigen-binding fragment binds to a tumor antigen. In another aspect, the first antibody or its antigen-binding fragment specifically binds to a tumor antigen and the second antibody or its antigen-binding fragment binds to an activating antigen on an immune effector cell. In a further aspect, the vector also comprises a fourth polynucleotide sequence encoding a third antibody or an antigen-binding fragment thereof, wherein the third antibody or the antigen-binding fragment thereof binds to an activating antigen on an immune effector cell or a tumor antigen. In one aspect, the immune effector cell comprises a dendritic cell, a natural killer ("NK") cell, a macrophage, a T cell, a B cell, or combination thereof. Non-liming examples of immune effector cells include a T cell or an NK cell.

Non-limiting examples of the activating antigen on the immune effector cell comprises CD3, CD2, CD4, CD8, CD19, LFA1, CD45, NKG2D, NKp44, NKp46, NKp30, DNAM, or combination thereof.

Non-limiting examples of target antigen on an antigen-presenting cell include, but are not limited to, B7-H3 (CD276).

Non-limiting examples of target antigen on a B-cell include, but are not limited to, CD20 and CD22.

Non-limiting examples of tumor antigens comprise one or more of an ephrin type-A receptor 2 (EphA2), interleukin (IL)-13r alpha 2, an EGFR VIII, a PSMA, an EpCAM, a GD3, a fucosyl GM1, a PSCA, a PLAC1, a sarcoma breakpoint, a Wilms Tumor 1, alphafetoprotein (AFP), carcinoembryonic antigen (CEA), CA-125, MUC-1, epithelial tumor antigen (ETA), tyrosinase, melanoma-associated antigen (MAGE), a hematologic differentiation antigen, a surface glycoprotein, a gangliosides (GM2), a growth factor receptor, a stromal antigen, a vascular antigen, receptor tyrosine kinase like orphan receptor 1 (ROR1), mesothelin, CD38, CD123, human epidermal growth factor receptor 2 (HER2), B-cell maturation antigen (BCMA), fibroblast activation protein (FAP) alpha, or a combination thereof. Additional examples are found in the art, see, e.g., incorporated by reference herein. In another aspect, the recombinant vector expresses a pre-mRNA that encodes a dimert described herein. In some instances, the dimert is a bispecific antibody or a trispecific antibody, when the pre-mRNA is in contact with a morpholino oligonucleotide. Non-limiting examples of dimerts are of the group of: a bispecific T cell engager (BiTE) or a bispecific NK cell engager (BiKE); a trispecific antibody comprises a trispecific T cell engager (TriTE) or a trispecific NK cell engager (TriKE). In one aspect, the trispecific antibody comprises the first antibody or the antigen-binding fragment thereof, and the second antibody or its antigen-binding fragment.

In one aspect, the dimert (e.g., the bispecific or trispecific cell engager) comprises a polypeptide sequence at least 95% sequence identity to SEQ ID NO: 11, optionally at least 96%, at least 97%, at least 98%, or at least 99% sequence identity to SEQ ID NO: 11. In another embodiment, the polypeptide sequence encodes an antigen-binding fragment for CD3; CD2; CD4; CD8; CD19; lymphocyte function-associated antigen 1 (LFA1); CD45; interleukin 21 receptor (IL21R); natural-killer group 2, member D (NKG2D); a natural cytotoxicity receptor (NCR) such as NKp44, NKp46, or NKp30; or DNAX accessory molecule-1 (DNAM or DNAM-1; also referred to as CD226, or platelet and T cell activation antigen 1 (PTA1)). In another embodiment, the polypeptide sequence encodes an antigen-binding fragment for CD3, CD19, GD2, or NKG2D. In another embodiment, the polypeptide encodes a first antigen-binding fragment and a second antigen-binding fragment. In another embodiment, the first antigen-binding fragment binds to CD3 and the second antigen-binding fragment binds to CD19. In another embodiment, the first antigen-binding fragment binds to CD3 and the second antigen-binding fragment binds to GD2. In another embodiment, the first antigen-binding fragment binds to NKG2D and the second antigen-binding fragment binds to GD2.

In one embodiment, the trispecific engager or antibody comprises the first antigen-binding fragment, the second antigen-binding fragment, and the third antigen-binding fragment. In another embodiment, the trispecific engager or antibody comprises three antigen-binding fragments that binds to NKG2D, IL21R, and GD2, individually. In a further aspect, the trispecific engager or antibody comprises a polypeptide sequence at least at least 95% sequence identity to SEQ ID NO. 11, or alternatively with at least 96%, or alternatively at least 97%, or alternatively 98%, or alternatively at least 99% sequence identity to SEQ ID NO: 11.

In one embodiment, the antigen-binding fragment that binds to IL-21R is IL-21. The amino acid and cDNA sequences of IL-12 are shown in SEQ ID NO. 3 and SEQ ID NO. 4, respectively. In one embodiment, the antigen-binding fragment that binds to NKG2D comprises MICA, MICB, ULBP1, ULBP2, ULBP3, ULBP4, ULBP5, ULBP6, Rae-1α, Rae-1β, Rae-1γ, Rae-1δ, Rae-1ε, H60a, H60b, H60c, MULT1, or a fragment thereof. In one embodiment, the antigen-binding fragment that binds to NKG2D is MICA (SEQ ID NO. 5) or its fragment or an equivalent thereof.

The MICA sequence, in one embodiment, comprises a mutant of the wide-type. In one embodiment, the MICA mutant is a sequence variant of the wild-type MICA (e.g., the wild-type MICA sequence set forth in SEQ ID NO: 5). In another embodiment, the MICA mutant is a sequence variant of MUC-30 (SEQ ID NO. 7), which comprises a methionine mutation instead of alanine at position 129 of the wide-type MICA sequence (MICA-129Met). An equivalent of the MICA mutant (MICA-129Met) retains the methionine mutation at position 129 of the wild-type MICA. In another embodiment, the antigen-binding fragments of the bispecific or trispecific engager or antibody is separated by a linker sequence. One embodiment of the linker sequence comprises, or consists essentially of, or yet consists of GGGGSGGGGSGGGGS (SEQ ID NO. 9), or an equivalent thereof. The linker sequence is encoded by a polynucleotide sequence of GGCGGCGGCGGCAGCGGCGGCGGCGGCAGCGGCGGCGGCGGCAGC (SEQ ID NO. 10), or an equivalent thereof. In one embodiment, the two antigen-binding fragments separated by a linker sequence is IL21 and MICA (e.g., a wild-type MICA or a MICA mutant such as MUC-30 or MICA-129Met), or their fragments, or equivalents of each thereof. In one embodiment, the two antigen-binding fragments separated by a linker sequence is GD2 and MICA (e.g., a wild-type MICA or a MICA mutant such as MUC-30 or MICA-129Met) or their fragments, or equivalents of each thereof. In one embodiment, the two antigen-binding fragments separated by a linker sequence is IL21 and GD2, or their fragments or equivalents of each thereof. In another embodiment, the linker is inserted between the antigen-binding fragments of any of the following trispecific engagers:
IL21-MICA129-GD2
MICA129-IL21-GD2
GD2-IL21-MICA129
GD2-MICA129-IL21
IL21-MICA/V129M-GD2-HDD
MICA/V12M-IL21-GD2-HDD
GD2-IL21-MICA129-HDD
GD2-MICA129-IL21-HDD

In another embodiment, the dimert (e.g., the bispecific or trispecific engager) comprises a secretion consensus sequence (also referred to herein as sec0A). In some instances, the secretion consensus sequence (sec0A) comprises at least 90%, 95%, 96%, 97%, 98%, 99%, or 100% sequence identity to MWWRLWWLLLLLLLLWPMVWA (SEQ ID NO: 51) or consist of SEQ ID NO: 51. In some instances, the sec0A is encoded by a polynucleotide comprising ATGTGGTGGAGACTGTGGTGGCTGCTGCTGCTGCTGCTGCTGCTGTGGCCCATGGTG TGGGCC (SEQ ID NO: 52), or an equivalent thereof.

In some instances, the secretion consensus sequence further comprises one, two, three, four, or more residues at the C-terminus of the sequence. In some instances, the one, two, three, four, or more residues are residues with aliphatic side chains (e.g., Ala, Met, Ile, Val, or Leu). In some instances, the one, two, three, four, or more residues are Ala residues, Gly residues, Val residues, Ile residues, or a combination thereof. In some instances, the one, two, three, four, or more residues are Ala residues, Gly residues, Val residues, or a combination thereof. In some instances, the one, two, three, four, or more residues are Ala residues, Gly residues, or a combination thereof. In some instances, the secretory consensus sequence further comprises one, two, three, four, or more Ala residues at the C-terminus of the sequence. In some instances, the secretory consensus sequence further comprises one, two, three, four, or more Gly residues at the C-terminus of the sequence. In some instances, the secretory consensus sequence further comprises one, two, three, four, or more Val residues at the C-terminus of the sequence. In some instances, the secretory consensus sequence further comprises one, two, three, four, or more Ile residues at the C-terminus of the sequence.

In some instances, the secretion consensus sequence further comprises one, two, or three residues with aliphatic side chains (e.g., Ala, Met, Ile, Val, or Leu). In some instances, the one, two, or three residues are Ala residues, Gly residues, Val residues, Ile residues, or a combination thereof. In some instances, the one, two, or three residues are Ala residues, Gly residues, Val residues, or a combination thereof. In some instances, the one, two, or three residues are Ala residues, Gly residues, or a combination thereof. In some instances, the secretory consensus sequence further comprises one, two, or three Ala residues at the C-terminus of the sequence. In some instances, the secretory consensus sequence further comprises one, two, or three Gly residues at the C-terminus of the sequence. In some instances, the secretory consensus sequence further comprises one, two, or three Val residues at the C-terminus of the sequence. In some instances, the secretory consensus sequence further comprises one, two, or three Ile residues at the C-terminus of the sequence.

In some instances, the secretion consensus sequence further comprises one or two residues with aliphatic side chains (e.g., Ala, Met, Ile, Val, or Leu). In some instances, the one or two residues are Ala residues, Gly residues, Val residues, Ile residues, or a combination thereof. In some instances, the one or two residues are Ala residues, Gly residues, Val residues, or a combination thereof. In some instances, the one or two residues are Ala residues, Gly residues, or a combination thereof. In some instances, the secretory consensus sequence further comprises one or two Ala residues at the C-terminus of the sequence. In some instances, the secretory consensus sequence further comprises one or two Gly residues at the C-terminus of the sequence. In some instances, the secretory consensus sequence further comprises one or two Val residues at the C-terminus of the sequence. In some instances, the secretory consensus sequence further comprises one or two Ile residues at the C-terminus of the sequence.

In some instances, the secretion consensus sequence further comprises one residue with an aliphatic side chain (e.g., Ala, Met, Ile, Val, or Leu). In some instances, the one residue is Ala, Gly, Val, or Ile. In some instances, the secretory consensus sequence further comprises one Ala at the C-terminus of the sequence. In some instances, the secretory consensus sequence further comprises one Gly at the C-terminus of the sequence. In some instances, the secretory consensus sequence further comprises one Val at the C-terminus of the sequence. In some instances, the secretory consensus sequence further comprises one Ile at the C-terminus of the sequence.

In some instances, the secretion consensus sequence further comprises one or two Ala residues at the C-terminus of the consensus sequence and such sequences are termed as secrecon1A (or sec1A) with one Ala at the C-terminus and secrecon2A (or sec2A) with two Ala residues at the C-terminus. In some instances, sec1A comprises at least 90%, 95%, 96%, 97%, 98%, 99%, or 100% sequence identity to MWWRLWWLLLLLLLLWPMVWAA (SEQ ID NO: 53). In some instances, sec1A is encoded by a polynucleotide comprising ATGTGGTGGAGACTGTGGTGGCTGCTGCTGCTGCTGCTGCTGCTGTGGCCCATGGTG TGGGCCGCC (SEQ ID NO: 54), or an equivalent thereof. In some cases, sec2A comprises at least 90%, 95%, 96%, 97%, 98%, 99%, or 100% sequence identity to MWWRLWWLLLLLLLLWPMVWAAA (SEQ ID NO: 55). In some instances, sec2A is encoded by a polynucleotide comprising ATGTGGTGGAGACTGTGGTGGCTGCTGCTGCTGCTGCTGCTGCTGTGGCCCATGGTG TGGGCCGCCGCC (SEQ ID NO: 56), or an equivalent thereof.

In some embodiments, the secretory consensus sequence modulates the expression and/or secretion of the dimert. In some instances, the secretory consensus sequence (e.g., sec1A or sec2A) enhances the expression and/or secretion of the dimert. In some cases, the secretory consensus sequence (e.g., sec1A or sec2A) modulate (e.g., enhances) the expression of the dimert by about 1-fold, 2-fold, 3-fold, 4-fold, 5-fold, 6-fold, 7-fold, 8-fold, 9-fold, 10-fold, 20-fold, 50-fold, or more. In some cases, the secretory consensus sequence (e.g., sec1A or sec2A) modulate (e.g., enhances) the secretion of the dimert by about 1-fold, 2-fold, 3-fold, 4-fold, 5-fold, 6-fold, 7-fold, 8-fold, 9-fold, 10-fold, 20-fold, 50-fold, or more.

In some embodiments, a dimert described herein (e.g., a trispecific engager) comprises a secretion consensus sequence (e.g., sec0A, sec1A, or sec2A), an IL21 sequence (e.g., SEQ ID NO: 3), a MICA sequence (e.g., a wild-type sequence, MUC-30, or MICA-129Met), an anti-GD2 peptide sequence, or an equivalent of one or more thereof. In some instances, the dimert (e.g., the trispecific engager) comprises a secrecon1A sequence, an IL21 sequence, an MICA129 sequence, an antiGD2 peptide sequence, or an equivalent of one or more thereof. In one embodiment, the dimert (e.g., the trispecific engager) comprises a secrecon1A-linker-IL21-linker-MICA129-linker-antiGD2 peptide or an equivalent of one or more thereof. In one embodiment, the dimert (e.g., the trispecific engager) comprises a polypeptide sequence of SEQ ID NO: 11.
**SEQ ID NO. 11**
MWWRLWWLLLLLLLLWPMVWAARSSPGNMERIVICLMVIFLGTLVHKSSSQGQDRHMIRMRQLI DIVDQLKNYVNDLVPEFLPAPEDVETNCEWSAFSCFQKAQLKSANTGNNERIINVSIKKLKRKP PSTNAGRRQKHRLTCPSCDSYEKKPPKEFLERFKSLLQKMIHQHLSSRTHGSEDSGGGGSGGGG SGGGGSEPHSLRYNLTVLSWDGSVQSGFLAEVHLDGQPFLRCDRQKCRAKPQGQWAEDVLGNKT WDRETRDLTGNGKDLRMTLAHIKDQKEGLHSLQEIRVCEIHEDNSTRSSQHFYYDGELFLSQNL ETEEWTMPQSSRAQTLAMNIRNFLKEDAMKTKTHYHAMHADCLQELRRYLKSGVVLRRTVPPMV NVTRSEASEGNITVTCRASGFYPWNITLSWRQDGVSLSHDTQQWGDVLPDGNGTYQTWVATRIC QGEEQRFTCYMEHSGNHSTHPVPSGGGGSGGGGSGGGGSQVQLQQSGPELVKPGASVKISCKTS GYKFTEYTMHWVKQSHGKCLEWIGGINPNNGGTNYNQKFKGKATLTVDKSSSTAYMELRSLTSE DSAVYYCARDTTVPYAYWGQGTTVTVSSGGGGSGGGGSGGGGSDIELTQSPAIMSASPGEKVTM TCSASSSISYMHWYQQKPGTSPKRWIYDTSKLASSVPARFSGSGSGTSYSLTISSMEAEDAATY YCHQRSSYPLTFGCGTKLEIKRASTKGP, or an equivalent thereof.

**In one embodiment, the dimert (e.g., the trispecific engager) is encoded by a sequence of SEQ ID NO. 12.**
**SEQ ID NO. 12**
ATGTGGTGGAGACTGTGGTGGCTGCTGCTGCTGCTGCTGCTGCTGTGGCCCATGGTGTGGGCCG CCAGAAGCAGCCCCGGCAACATGGAGAGAATCGTGATCTGCCTGATGGTGATCTTCCTGGGCAC CCTGGTGCACAAGAGCAGCAGCCAGGGCCAGGACAGACACATGATCAGAATGAGACAGCTGATC GACATCGTGGACCAGCTGAAGAACTACGTGAACGACCTGGTGCCCGAGTTCCTGCCCGCCCCCG AGGACGTGGAGACCAACTGCGAGTGGAGCGCCTTCAGCTGCTTCCAGAAGGCCCAGCTGAAGAG CGCCAACACCGGCAACAACGAGAGAATCATCAACGTGAGCATCAAGAAGCTGAAGAGAAAGCCC CCCAGCACCAACGCCGGCAGAAGACAGAAGCACAGACTGACCTGCCCCAGCTGCGACAGCTACG AGAAGAAGCCCCCCAAGGAGTTCCTGGAGAGATTCAAGAGCCTGCTGCAGAAGATGATCCACCA GCACCTGAGCAGCAGAACCCACGGCAGCGAGGACAGCGGCGGCGGCGGCAGCGGCGGCGGCGGC AGCGGCGGCGGCGGCAGCGAGCCCCACAGTCTTCGTTATAACCTCACGGTGCTGTCCTGGGATG GATCTGTGCAGTCAGGGTTTCTCGCTGAGGTACATCTGGATGGTCAGCCCTTCCTGCGCTGTGA CAGGCAGAAATGCAGGGCAAAGCCCCAGGGACAGTGGGCAGAAGATGTCCTGGGAAATAAGACA TGGGACAGAGAGACCAGGGACTTGACAGGGAACGGAAAGGACCTCAGGATGACCCTGGCTCATA TCAAGGACCAGAAAGAAGGCTTGCATTCCCTCCAGGAGATTAGGGTCTGTGAGATCCATGAAGA CAACAGCACCAGGAGCTCCCAGCATTTCTACTACGATGGGGAGCTCTTCCTCTCCCAAAACCTG GAGACTGAGGAATGGACAATGCCCCAGTCCTCCAGAGCTCAGACCTTGGCCATGAACATCAGGA ATTTCTTGAAGGAAGATGCCATGAAGACCAAGACACACTATCACGCTATGCATGCAGACTGCCT GCAGGAACTACGGCGATATCTAAAATCCGGCGTAGTCCTGAGGAGAACAGTGCCCCCCATGGTG AATGTCACCCGCAGCGAGGCCTCAGAGGGCAACATTACCGTGACATGCAGGGCTTCTGGCTTCT ATCCCTGGAATATCACACTGAGCTGGCGTCAGGATGGGGTATCTTTGAGCCACGACACCCAGCA GTGGGGGGATGTCCTGCCTGATGGGAATGGAACCTACCAGACCTGGGTGGCCACCAGGATTTGC CAAGGAGAGGAGCAGAGGTTCACCTGCTACATGGAACACAGCGGGAATCACAGCACTCACCCTG TGCCCTCTGGCGGCGGCGGCAGCGGCGGCGGCGGCAGCGGCGGCGGCGGCAGCCAGGTGCAGCT GCAGCAGAGCGGCCCCGAGCTGGTGAAGCCCGGCGCCAGCGTGAAGATCAGCTGCAAGACCAGC GGCTACAAGTTCACCGAGTACACCATGCACTGGGTGAAGCAGAGCCACGGCAAGTGCCTGGAGT GGATCGGCGGCATCAACCCCAACAACGGCGGCACCAACTACAACCAGAAGTTCAAGGGCAAGGC CACCCTGACCGTGGACAAGAGCAGCAGCACCGCCTACATGGAGCTGAGAAGCCTGACCAGCGAG GACAGCGCCGTGTACTACTGCGCCAGAGACACCACCGTGCCCTACGCCTACTGGGGCCAGGGCA CCACCGTGACCGTGAGCAGCGGCGGCGGCGGCAGCGGCGGCGGCGGCAGCGGCGGCGGCGGCAG CGACATCGAGCTGACCCAGAGCCCCGCCATCATGAGCGCCAGCCCCGGCGAGAAGGTGACCATG ACCTGCAGCGCCAGCAGCAGCATCAGCTACATGCACTGGTACCAGCAGAAGCCCGGCACCAGCC CCAAGAGATGGATCTACGACACCAGCAAGCTGGCCAGCAGCGTGCCCGCCAGATTCAGCGGCAG CGGCAGCGGCACCAGCTACAGCCTGACCATCAGCAGCATGGAGGCCGAGGACGCCGCCACCTAC TACTGCCACCAGAGAAGCAGCTACCCCCTGACCTTCGGCTGCGGCACCAAGCTGGAGATCAAGA GAGCCAGCACCAAGGGCCCCTAG, or an equivalent thereof.

**In** one embodiment, the dimert (e.g., the bispecific engager) comprises a secrecon1A sequence (sec1A), a CD3 sequence, and an anti-GD2 peptide sequence, or an equivalent of one or more thereof. In one embodiment, the bispecific engager comprises a polypeptide sequence of SEQ ID NO. 13, arranged as sec1A-anti-CD3-linker-anti-GD2-HDD. The sec1A portion is underlined. The anti-CD3 portion is bold. The anti-GD2 portion is underlined and italicized. The shaded region in gray denotes the HDD portion, which comprises the HDD peptide in bold, a hinge region in small letter upstream of the HDD peptide, and a spacer region in small letter downstream of the HDD peptide.
**SEQ ID NO. 13**
MWWRLWWLLLLLLLLWPMVWAA**QVQLQQSGPELVKPGASVKISCKTSGYKFTEYTMHWVKQSHG KCLEWIGGINPNNGGTNYNQKFKGKATLTVDKSSSTAYMELRSLTSEDSAVYYCARDTTVPYAY WGQGTTVTVSSGGGGSGGGGSGGGGSDIELTQSPAIMSASPGEKVTMTCSASSSISYMHWYQQK PGTSPKRWIYDTSKLASSVPARFSGSGSGTSYSLTISSMEAEDAATYYCHQRSSYPLTFGCGTK LEIKRASTKGP**GGGGSGGGGSGGGGS*QVQLVQSGGGVVQPGRSLRLSCKASGYTFTRYTMHWVR QAPGKGLEWIGYINPSRGYTNYNQKFKDRFTISRDNSKNTAFLQMDSLRPEDTGVYFCARYYDD HYCLDYWGQGTPVTVSSGGGGSGGGGSGGGGSDIQMTQSPSSLSASVGDRVTITCSASSSVSYM NWYQQTPGKAPKRWIYDTSKLASGVPSRFSGSGSGTDYTFTISSLQPEDIATYYCQQWSSNPFT FGQGTKLQITR*tplgdtthtsg**MVSKLSQLQTELLAALLESGLSKEALIQALGE**gsggap, or an equivalent thereof.

**In another embodiment, the dimert (e.g., the bispecific engager) comprising a sec1A sequence, a anti-CD3 sequence, and an anti-GD2 peptide sequence, or an equivalent of one or more** thereof and **in one aspect is encoded by a sequence of SEQ ID NO. 14.**
SEQ ID NO. 14
ATGTGGTGGAGACTGTGGTGGCTGCTGCTGCTGCTGCTGCTGCTGTGGCCCATGGTGTGGGCCG CCCAGGTGCAGCTGCAGCAGAGCGGCCCCGAGCTGGTGAAGCCCGGCGCCAGCGTGAAGATCAG CTGCAAGACCAGCGGCTACAAGTTCACCGAGTACACCATGCACTGGGTGAAGCAGAGCCACGGC AAGTGCCTGGAGTGGATCGGCGGCATCAACCCCAACAACGGCGGCACCAACTACAACCAGAAGT TCAAGGGCAAGGCCACCCTGACCGTGGACAAGAGCAGCAGCACCGCCTACATGGAGCTGAGAAG CCTGACCAGCGAGGACAGCGCCGTGTACTACTGCGCCAGAGACACCACCGTGCCCTACGCCTAC TGGGGCCAGGGCACCACCGTGACCGTGAGCAGCGGCGGCGGCGGCAGCGGCGGCGGCGGCAGCG GCGGCGGCGGCAGCGACATCGAGCTGACCCAGAGCCCCGCCATCATGAGCGCCAGCCCCGGCGA GAAGGTGACCATGACCTGCAGCGCCAGCAGCAGCATCAGCTACATGCACTGGTACCAGCAGAAG CCCGGCACCAGCCCCAAGAGATGGATCTACGACACCAGCAAGCTGGCCAGCAGCGTGCCCGCCA GATTCAGCGGCAGCGGCAGCGGCACCAGCTACAGCCTGACCATCAGCAGCATGGAGGCCGAGGA CGCCGCCACCTACTACTGCCACCAGAGAAGCAGCTACCCCCTGACCTTCGGCTGCGGCACCAAG CTGGAGATCAAGAGAGCCAGCACCAAGGGCCCCGGCGGCGGCGGCAGCGGCGGCGGCGGCAGCG GCGGCGGCGGCAGCCAGGTGCAGCTGGTGCAGAGCGGCGGCGGCGTGGTGCAGCCCGGCAGAAG CCTGAGACTGAGCTGCAAGGCCAGCGGCTACACCTTCACCAGATACACCATGCACTGGGTGAGA CAGGCCCCCGGCAAGGGCCTGGAGTGGATCGGCTACATCAACCCCAGCAGAGGCTACACCAACT ACAACCAGAAGTTCAAGGACAGATTCACCATCAGCAGAGACAACAGCAAGAACACCGCCTTCCT GCAGATGGACAGCCTGAGACCCGAGGACACCGGCGTGTACTTCTGCGCCAGATACTACGACGAC CACTACTGCCTGGACTACTGGGGCCAGGGCACCCCCGTGACCGTGAGCAGCGGCGGCGGCGGCA GCGGCGGCGGCGGCAGCGGCGGCGGCGGCAGCGACATCCAGATGACCCAGAGCCCCAGCAGCCT GAGCGCCAGCGTGGGCGACAGAGTGACCATCACCTGCAGCGCCAGCAGCAGCGTGAGCTACATG AACTGGTACCAGCAGACCCCCGGCAAGGCCCCCAAGAGATGGATCTACGACACCAGCAAGCTGG CCAGCGGCGTGCCCAGCAGATTCAGCGGCAGCGGCAGCGGCACCGACTACACCTTCACCATCAG CAGCCTGCAGCCCGAGGACATCGCCACCTACTACTGCCAGCAGTGGAGCAGCAACCCCTTCACC TTCGGCCAGGGCACCAAGCTGCAGATCACCAGAACCCCCCTGGGCGACACCACCCACACCAGCG GCATGGTGAGCAAGCTGAGCCAGCTGCAGACCGAGCTGCTGGCCGCCCTGCTGGAGAGCGGCCT GAGCAAGGAGGCCCTGATCCAGGCCCTGGGCGAGGGCAGCGGCGGCGCCCCCTAG, or an equivalent thereof.

In another embodiment, the dimert (e.g., the bispecific engager) comprises a secrecon1A sequence, an anti-CD19 sequence and an anti-CD3 sequence, arranged as sec1A-anti-CD19-linker-anti-CD3. The bispecific engager comprises a polypeptide sequence of SEQ ID NO. 15. The sec1A portion is underlined.
**SEQ ID NO. 15** MWWRLWWLLLLLLLLWPMVWAADIQLTQSPASLAVSLGQRATISCKASQSVDYDGDSYLNWYQQ IPGQPPKLLIYDASNLVSGIPPRFSGSGSGTDFTLNIHPVEKVDAATYHCQQSTEDPWTFGGGT KLEIKGGGGSGGGGSGGGGSQVQLQQSGAELVRPGSSVKISCKASGYAFSSYWMNWVKQRPGQG LEWIGQIWPGDGDTNYNGKFKGKATLTADESSSTAYMQLSSLASEDSAVYFCARRETTTVGRYY YAMDYWGQGTTVTVSSGGGGSDIKLQQSGAELARPGASVKMSCKTSGYTFTRYTMHWVKQRPGQ GLEWIGYINPSRGYTNYNQKFKDKATLTTDKSSSTAYMQLSSLTSEDSAVYYCARYYDDHYCLD YWGQGTTLTVSSVEGGSGGSGGSGGSGGVDDIQLTQSPAIMSASPGEKVTMTCRASSSVSYMNW YQQKSGTSPKRWIYDTSKVASGVPYRFSGSGSGTSYSLTISSMEAEDAATYYCQQWSSNPLTFG AGTKLELKHHHHHHTPLGDTTHTSGMVSKLSQLQTELLAALLESGLSKEALIQALGEGSGGAP, or an equivalent thereof.

In another embodiment, the dimert (e.g., the bispecific engager) comprising a secrecon1A sequence, an anti-CD19 sequence and an anti-CD3 sequence arranged as sec1A-anti-CD19-linker-anti-CD3, or an equivalent of one or more thereof. In one aspect, it is encoded by a polynucleotide sequence of SEQ ID NO. 16. 
SEQ ID NO. 16
ATGTGGTGGAGACTGTGGTGGCTGCTGCTGCTGCTGCTGCTGTGGCCCATGGTGTGGGCCG
CCGACATCCAGCTGACCCAGAGCCCCGCCAGCCTGGCCGTGAGCCTGGGCCAGAGAGCCACCAT
CAGCTGCAAGGCCAGCCAGAGCGTGGACTACGACGGCGACAGCTACCTGAACTGGTACCAGCAG
ATCCCCGGCCAGCCCCCCAAGCTGCTGATCTACGACGCCAGCAACCTGGTGAGCGGCATCCCCC
CCAGATTCAGCGGCAGCGGCAGCGGCACCGACTTCACCCTGAACATCCACCCCGTGGAGAAGGT
GGACGCCGCCACCTACCACTGCCAGCAGAGCACCGAGGACCCCTGGACCTTCGGCGGCGGCACC
AAGCTGGAGATCAAGGGCGGCGGCGGCAGCGGCGGCGGCGGCAGCGGCGGCGGCGGCAGCCAGG
TGCAGCTGCAGCAGAGCGGCGCCGAGCTGGTGAGACCCGGCAGCAGCGTGAAGATCAGCTGCAA
GGCCAGCGGCTACGCCTTCAGCAGCTACTGGATGAACTGGGTGAAGCAGAGACCCGGCCAGGGC
CTGGAGTGGATCGGCCAGATCTGGCCCGGCGACGGCGACACCAACTACAACGGCAAGTTCAAGG
GCAAGGCCACCCTGACCGCCGACGAGAGCAGCAGCACCGCCTACATGCAGCTGAGCAGCCTGGC
CAGCGAGGACAGCGCCGTGTACTTCTGCGCCAGAAGAGAGACCACCACCGTGGGCAGATACTAC
 
TACGCCATGGACTACTGGGGCCAGGGCACCACCGTGACCGTGAGCAGCGGCGGCGGCAGCG
ACATCAAGCTGCAGCAGAGCGGCGCCGAGCTGGCCAGACCCGGCGCCAGCGTGAAGATGAGCTG
CAAGACCAGCGGCTACACCTTCACCAGATACACCATGCACTGGGTGAAGCAGAGACCCGGCCAG
GGCCTGGAGTGGATCGGCTACATCAACCCCAGCAGAGGCTACACCAACTACAACCAGAAGTTCA
AGGACAAGGCCACCCTGACCACCGACAAGAGCAGCAGCACCGCCTACATGCAGCTGAGCAGCCT
GACCAGCGAGGACAGCGCCGTGTACTACTGCGCCAGATACTACGACGACCACTACTGCCTGGAC
TACTGGGGCCAGGGCACCACCCTGACCGTGAGCAGCGTGGAGGGCGGCAGCGGCGGCAGCGGCG
GCAGCGGCGGCAGCGGCGGCGTGGACGACATCCAGCTGACCCAGAGCCCCGCCATCATGAGCGC
CAGCCCCGGCGAGAAGGTGACCATGACCTGCAGAGCCAGCAGCAGCGTGAGCTACATGAACTGG
TACCAGCAGAAGAGCGGCACCAGCCCCAAGAGATGGATCTACGACACCAGCAAGGTGGCCAGCG
GCGTGCCCTACAGATTCAGCGGCAGCGGCAGCGGCACCAGCTACAGCCTGACCATCAGCAGCAT
GGAGGCCGAGGACGCCGCCACCTACTACTGCCAGCAGTGGAGCAGCAACCCCCTGACCTTCGGC
GCCGGCACCAAGCTGGAGCTGAAGCACCACCACCACCACCACACCCCCCTGGGCGACACCACCC
ACACCAGCGGCATGGTGAGCAAGCTGAGCCAGCTGCAGACCGAGCTGCTGGCCGCCCTGCTGGA
GAGCGGCCTGAGCAAGGAGGCCCTGATCCAGGCCCTGGGCGAGGGCAGCGGCGGCGCCCCCTA,
 or an equivalent thereof.

In some embodiments, the dimert comprises a secretion signal termed secreconAA (sec2A). In some instances, the dimert comprises secreconAA linked in tandem with an amino acid sequence from Blinatumomab (which targets CD3 and CD19). In some instances, the secreconAA-Blinatumomab (or sec2A-CD19xCD3) comprises a polypeptide sequence of SEQ ID NO: 29 in which the secreconAA portion is underlined.
**SEQ ID NO: 29**
**MWWRLWWLLLLLLLLWPMVWAAA**DIQLTQSPASLAVSLGQRATISCKASQSVDYDGDSYLNWYQ QIPGQPPKLLIYDASNLVSGIPPRFSGSGSGTDFTLNIHPVEKVDAATYHCQQSTEDPWTFGGG TKLEIKGGGGSGGGGSGGGGSQVQLQQSGAELVRPGSSVKISCKASGYAFSSYWMNWVKQRPGQ GLEWIGQIWPGDGDTNYNGKFKGKATLTADESSSTAYMQLSSLASEDSAVYFCARRETTTVGRY YYAMDYWGQGTTVTVSSGGGGSDIKLQQSGAELARPGASVKMSCKTSGYTFTRYTMHWVKQRPG QGLEWIGYINPSRGYTNYNQKFKDKATLTTDKSSSTAYMQLSSLTSEDSAVYYCARYYDDHYCL DYWGQGTTLTVSSVEGGSGGSGGSGGSGGVDDIQLTQSPATMSASPGEKVTMTCRASSSVSYMN WYQQKSGTSPKRWIYDTSKVASGVPYRFSGSGSGTSYSLTISSMEAEDAATYYCQQWSSNPLTF GAGTKLELKHHHHHH, or an equivalent thereof.

In some embodiments, the dimert comprising sec2A-CD19xCD3 is encoded by a polynucleotide sequence of SEQ ID NO: 30.
**SEQ ID NO: 30**
ATGTGGTGGAGACTGTGGTGGCTGCTGCTGCTGCTGCTGCTGCTGTGGCCCATGGTGTGGGCCG CCGCCGACATCCAGCTGACCCAGAGCCCCGCCAGCCTGGCCGTGAGCCTGGGCCAGAGAGCCAC CATCAGCTGCAAGGCCAGCCAGAGCGTGGACTACGACGGCGACAGCTACCTGAACTGGTACCAG CAGATCCCCGGCCAGCCCCCCAAGCTGCTGATCTACGACGCCAGCAACCTGGTGAGCGGCATCC CCCCCAGATTCAGCGGCAGCGGCAGCGGCACCGACTTCACCCTGAACATCCACCCCGTGGAGAA GGTGGACGCCGCCACCTACCACTGCCAGCAGAGCACCGAGGACCCCTGGACCTTCGGCGGCGGC ACCAAGCTGGAGATCAAGGGCGGCGGCGGCAGCGGCGGCGGCGGCAGCGGCGGCGGCGGCAGCC AGGTGCAGCTGCAGCAGAGCGGCGCCGAGCTGGTGAGACCCGGCAGCAGCGTGAAGATCAGCTG CAAGGCCAGCGGCTACGCCTTCAGCAGCTACTGGATGAACTGGGTGAAGCAGAGACCCGGCCAG GGCCTGGAGTGGATCGGCCAGATCTGGCCCGGCGACGGCGACACCAACTACAACGGCAAGTTCA AGGGCAAGGCCACCCTGACCGCCGACGAGAGCAGCAGCACCGCCTACATGCAGCTGAGCAGCCT GGCCAGCGAGGACAGCGCCGTGTACTTCTGCGCCAGAAGAGAGACCACCACCGTGGGCAGATAC TACTACGCCATGGACTACTGGGGCCAGGGCACCACCGTGACCGTGAGCAGCGGCGGCGGCGGCA GCGACATCAAGCTGCAGCAGAGCGGCGCCGAGCTGGCCAGACCCGGCGCCAGCGTGAAGATGAG CTGCAAGACCAGCGGCTACACCTTCACCAGATACACCATGCACTGGGTGAAGCAGAGACCCGGC CAGGGCCTGGAGTGGATCGGCTACATCAACCCCAGCAGAGGCTACACCAACTACAACCAGAAGT TCAAGGACAAGGCCACCCTGACCACCGACAAGAGCAGCAGCACCGCCTACATGCAGCTGAGCAG CCTGACCAGCGAGGACAGCGCCGTGTACTACTGCGCCAGATACTACGACGACCACTACTGCCTG GACTACTGGGGCCAGGGCACCACCCTGACCGTGAGCAGCGTGGAGGGCGGCAGCGGCGGCAGCG GCGGCAGCGGCGGCAGCGGCGGCGTGGACGACATCCAGCTGACCCAGAGCCCCGCCATCATGAG CGCCAGCCCCGGCGAGAAGGTGACCATGACCTGCAGAGCCAGCAGCAGCGTGAGCTACATGAAC TGGTACCAGCAGAAGAGCGGCACCAGCCCCAAGAGATGGATCTACGACACCAGCAAGGTGGCCA GCGGCGTGCCCTACAGATTCAGCGGCAGCGGCAGCGGCACCAGCTACAGCCTGACCATCAGCAG CATGGAGGCCGAGGACGCCGCCACCTACTACTGCCAGCAGTGGAGCAGCAACCCCCTGACCTTC GGCGCCGGCACCAAGCTGGAGCTGAAGCACCACCACCACCACCACTAG, or an equivalent thereof.

In some embodiments, the dimert comprising a secreconA (sec1A) sequence, CD19, and CD3 arranged in sec1A-CD19xCD3, or an equivalent of one or more thereof. In one aspect, it is encoded by a polynucleotide sequence of SEQ ID NO: 31.
**SEQ ID NO: 31**
ATGTGGTGGAGACTGTGGTGGCTGCTGCTGCTGCTGCTGCTGCTGTGGCCCATGGTGTGGGCCG CCGACATCCAGCTGACCCAGAGCCCCGCCAGCCTGGCCGTGAGCCTGGGCCAGAGAGCCACCAT CAGCTGCAAGGCCAGCCAGAGCGTGGACTACGACGGCGACAGCTACCTGAACTGGTACCAGCAG ATCCCCGGCCAGCCCCCCAAGCTGCTGATCTACGACGCCAGCAACCTGGTGAGCGGCATCCCCC CCAGATTCAGCGGCAGCGGCAGCGGCACCGACTTCACCCTGAACATCCACCCCGTGGAGAAGGT GGACGCCGCCACCTACCACTGCCAGCAGAGCACCGAGGACCCCTGGACCTTCGGCGGCGGCACC AAGCTGGAGATCAAGGGCGGCGGCGGCAGCGGCGGCGGCGGCAGCGGCGGCGGCGGCAGCCAGG TGCAGCTGCAGCAGAGCGGCGCCGAGCTGGTGAGACCCGGCAGCAGCGTGAAGATCAGCTGCAA GGCCAGCGGCTACGCCTTCAGCAGCTACTGGATGAACTGGGTGAAGCAGAGACCCGGCCAGGGC CTGGAGTGGATCGGCCAGATCTGGCCCGGCGACGGCGACACCAACTACAACGGCAAGTTCAAGG GCAAGGCCACCCTGACCGCCGACGAGAGCAGCAGCACCGCCTACATGCAGCTGAGCAGCCTGGC CAGCGAGGACAGCGCCGTGTACTTCTGCGCCAGAAGAGAGACCACCACCGTGGGCAGATACTAC TACGCCATGGACTACTGGGGCCAGGGCACCACCGTGACCGTGAGCAGCGGCGGCGGCGGCAGCG ACATCAAGCTGCAGCAGAGCGGCGCCGAGCTGGCCAGACCCGGCGCCAGCGTGAAGATGAGCTG CAAGACCAGCGGCTACACCTTCACCAGATACACCATGCACTGGGTGAAGCAGAGACCCGGCCAG GGCCTGGAGTGGATCGGCTACATCAACCCCAGCAGAGGCTACACCAACTACAACCAGAAGTTCA AGGACAAGGCCACCCTGACCACCGACAAGAGCAGCAGCACCGCCTACATGCAGCTGAGCAGCCT GACCAGCGAGGACAGCGCCGTGTACTACTGCGCCAGATACTACGACGACCACTACTGCCTGGAC TACTGGGGCCAGGGCACCACCCTGACCGTGAGCAGCGTGGAGGGCGGCAGCGGCGGCAGCGGCG GCAGCGGCGGCAGCGGCGGCGTGGACGACATCCAGCTGACCCAGAGCCCCGCCATCATGAGCGC CAGCCCCGGCGAGAAGGTGACCATGACCTGCAGAGCCAGCAGCAGCGTGAGCTACATGAACTGG TACCAGCAGAAGAGCGGCACCAGCCCCAAGAGATGGATCTACGACACCAGCAAGGTGGCCAGCG GCGTGCCCTACAGATTCAGCGGCAGCGGCAGCGGCACCAGCTACAGCCTGACCATCAGCAGCAT GGAGGCCGAGGACGCCGCCACCTACTACTGCCAGCAGTGGAGCAGCAACCCCCTGACCTTCGGC GCCGGCACCAAGCTGGAGCTGAAGCACCACCACCACCACCACTAG, or an equivalent thereof.

In some embodiments, the dimert comprising a secrecon (sec0A) sequence, CD19, and CD3, or an equivalent of one or more thereof, which is one aspect is arranged as sec0A-CD19xCD3 is encoded by a polynucleotide sequence of SEQ ID NO: 32.
**SEQ ID NO: 32**
ATGTGGTGGAGACTGTGGTGGCTGCTGCTGCTGCTGCTGCTGCTGTGGCCCATGGTGTGGGCCG ACATCCAGCTGACCCAGAGCCCCGCCAGCCTGGCCGTGAGCCTGGGCCAGAGAGCCACCATCAG CTGCAAGGCCAGCCAGAGCGTGGACTACGACGGCGACAGCTACCTGAACTGGTACCAGCAGATC CCCGGCCAGCCCCCCAAGCTGCTGATCTACGACGCCAGCAACCTGGTGAGCGGCATCCCCCCCA GATTCAGCGGCAGCGGCAGCGGCACCGACTTCACCCTGAACATCCACCCCGTGGAGAAGGTGGA CGCCGCCACCTACCACTGCCAGCAGAGCACCGAGGACCCCTGGACCTTCGGCGGCGGCACCAAG CTGGAGATCAAGGGCGGCGGCGGCAGCGGCGGCGGCGGCAGCGGCGGCGGCGGCAGCCAGGTGC AGCTGCAGCAGAGCGGCGCCGAGCTGGTGAGACCCGGCAGCAGCGTGAAGATCAGCTGCAAGGC CAGCGGCTACGCCTTCAGCAGCTACTGGATGAACTGGGTGAAGCAGAGACCCGGCCAGGGCCTG GAGTGGATCGGCCAGATCTGGCCCGGCGACGGCGACACCAACTACAACGGCAAGTTCAAGGGCA AGGCCACCCTGACCGCCGACGAGAGCAGCAGCACCGCCTACATGCAGCTGAGCAGCCTGGCCAG CGAGGACAGCGCCGTGTACTTCTGCGCCAGAAGAGAGACCACCACCGTGGGCAGATACTACTAC GCCATGGACTACTGGGGCCAGGGCACCACCGTGACCGTGAGCAGCGGCGGCGGCGGCAGCGACA TCAAGCTGCAGCAGAGCGGCGCCGAGCTGGCCAGACCCGGCGCCAGCGTGAAGATGAGCTGCAA GACCAGCGGCTACACCTTCACCAGATACACCATGCACTGGGTGAAGCAGAGACCCGGCCAGGGC CTGGAGTGGATCGGCTACATCAACCCCAGCAGAGGCTACACCAACTACAACCAGAAGTTCAAGG ACAAGGCCACCCTGACCACCGACAAGAGCAGCAGCACCGCCTACATGCAGCTGAGCAGCCTGAC CAGCGAGGACAGCGCCGTGTACTACTGCGCCAGATACTACGACGACCACTACTGCCTGGACTAC TGGGGCCAGGGCACCACCCTGACCGTGAGCAGCGTGGAGGGCGGCAGCGGCGGCAGCGGCGGCA GCGGCGGCAGCGGCGGCGTGGACGACATCCAGCTGACCCAGAGCCCCGCCATCATGAGCGCCAG CCCCGGCGAGAAGGTGACCATGACCTGCAGAGCCAGCAGCAGCGTGAGCTACATGAACTGGTAC CAGCAGAAGAGCGGCACCAGCCCCAAGAGATGGATCTACGACACCAGCAAGGTGGCCAGCGGCG TGCCCTACAGATTCAGCGGCAGCGGCAGCGGCACCAGCTACAGCCTGACCATCAGCAGCATGGA GGCCGAGGACGCCGCCACCTACTACTGCCAGCAGTGGAGCAGCAACCCCCTGACCTTCGGCGCC GGCACCAAGCTGGAGCTGAAGCACCACCACCACCACCACTAG, or an equivalent thereof.

In some embodiments, the dimert comprising a CD19 and CD3 sequence without a secrecon sequence arranged as CD19xCD3, or an equivalent of one or both thereof which in one aspect is encoded by a polynucleotide sequence of SEQ ID NO: 33.
**SEQ ID NO: 33**
ATGGACATCCAGCTGACCCAGAGCCCCGCCAGCCTGGCCGTGAGCCTGGGCCAGAGAGCCACCA TCAGCTGCAAGGCCAGCCAGAGCGTGGACTACGACGGCGACAGCTACCTGAACTGGTACCAGCA GATCCCCGGCCAGCCCCCCAAGCTGCTGATCTACGACGCCAGCAACCTGGTGAGCGGCATCCCC CCCAGATTCAGCGGCAGCGGCAGCGGCACCGACTTCACCCTGAACATCCACCCCGTGGAGAAGG TGGACGCCGCCACCTACCACTGCCAGCAGAGCACCGAGGACCCCTGGACCTTCGGCGGCGGCAC CAAGCTGGAGATCAAGGGCGGCGGCGGCAGCGGCGGCGGCGGCAGCGGCGGCGGCGGCAGCCAG GTGCAGCTGCAGCAGAGCGGCGCCGAGCTGGTGAGACCCGGCAGCAGCGTGAAGATCAGCTGCA AGGCCAGCGGCTACGCCTTCAGCAGCTACTGGATGAACTGGGTGAAGCAGAGACCCGGCCAGGG CCTGGAGTGGATCGGCCAGATCTGGCCCGGCGACGGCGACACCAACTACAACGGCAAGTTCAAG GGCAAGGCCACCCTGACCGCCGACGAGAGCAGCAGCACCGCCTACATGCAGCTGAGCAGCCTGG CCAGCGAGGACAGCGCCGTGTACTTCTGCGCCAGAAGAGAGACCACCACCGTGGGCAGATACTA CTACGCCATGGACTACTGGGGCCAGGGCACCACCGTGACCGTGAGCAGCGGCGGCGGCGGCAGC GACATCAAGCTGCAGCAGAGCGGCGCCGAGCTGGCCAGACCCGGCGCCAGCGTGAAGATGAGCT GCAAGACCAGCGGCTACACCTTCACCAGATACACCATGCACTGGGTGAAGCAGAGACCCGGCCA GGGCCTGGAGTGGATCGGCTACATCAACCCCAGCAGAGGCTACACCAACTACAACCAGAAGTTC AAGGACAAGGCCACCCTGACCACCGACAAGAGCAGCAGCACCGCCTACATGCAGCTGAGCAGCC TGACCAGCGAGGACAGCGCCGTGTACTACTGCGCCAGATACTACGACGACCACTACTGCCTGGA CTACTGGGGCCAGGGCACCACCCTGACCGTGAGCAGCGTGGAGGGCGGCAGCGGCGGCAGCGGC GGCAGCGGCGGCAGCGGCGGCGTGGACGACATCCAGCTGACCCAGAGCCCCGCCATCATGAGCG CCAGCCCCGGCGAGAAGGTGACCATGACCTGCAGAGCCAGCAGCAGCGTGAGCTACATGAACTG GTACCAGCAGAAGAGCGGCACCAGCCCCAAGAGATGGATCTACGACACCAGCAAGGTGGCCAGC GGCGTGCCCTACAGATTCAGCGGCAGCGGCAGCGGCACCAGCTACAGCCTGACCATCAGCAGCA TGGAGGCCGAGGACGCCGCCACCTACTACTGCCAGCAGTGGAGCAGCAACCCCCTGACCTTCGG CGCCGGCACCAAGCTGGAGCTGAAGCACCACCACCACCACCACTAG, or an equivalent thereof.

In one aspect, the bispecific T cell engager comprises a polypeptide sequence at least 95% identical to any one of SEQ ID NOs. 12 and 13, or alternatively at least 96% or alternatively at least 97%, or alternatively 98%, or alternatively at least 99% identity. In one embodiment, the trispecific antibody comprises the first antibody or its antigen-binding fragment, the second antibody its antigen-binding fragment, and the third antibody or its antigen-binding fragment. In a further aspect, the trispecific antibody comprises a polypeptide sequence at least at least 95% sequence identity to SEQ ID NO. 11, or alternatively at least 96% or alternatively at least 97%, or alternatively 98%, or alternatively at least 99% sequence identity to SEQ ID NO: 11.

In a further aspect, the recombinant polynucleotide expresses a pre-mRNA that encodes a trispecific antibody, when the pre-mRNA is in contact with the morpholino oligonucleotide.

In one aspect, the antigen-binding domain is a single-chain variable fragment of an antibody.

In a further aspect, the recombinant polynucleotide or vector further comprises a polynucleotide sequence encoding a secretory peptide. In another aspect, the vector further comprises a polynucleotide sequence encoding a dimerization domain. In another aspect, the vector comprises a 5' inverted terminal repeat (ITR) and a 3' ITR. In another aspect, the vector comprises a sequence of SEQ ID Nos. 4, 6, 8, 12, 15, 16, 30-33, or an equivalent thereof, or a polynucleotide, or alternatively at least 95%, or alternatively at least 96% or alternatively at least 97%, or alternatively 98%, or alternatively at least 99% identity. Non-limiting examples of the vector include: a recombinant viral vector that comprises a backbone vector selected from the group of a retroviral vector, a lentiviral vector, a murine leukemia viral ("MLV") vector, an Epstein-Barr viral ("EBV") vector, an adenoviral vector, a herpes viral ("HSV") vector, an Adeno-associated viral ("AAV") vector, an AAV vector, or optionally a self-complementary AAV vector. These are optionally detectably labeled. Also provided are the compliments of the polynucleotides that are optionally detectably labeled.

The recombinant polynucleotides vectors can be contained within host cells, e.g., prokaryotic or eukaryotic cells. The cells can be used to recombinantly express or replicate the polynucleotides by culturing the cells containing the polynucleotides under conditions that allow for replication of the polynucleotides and optionally expression of the polynucleotides. The polynucleotides and/or expression products are optionally isolated from the cell culture.

The invention also provides a viral packaging system comprising: the vector as described above, wherein the backbone is derived from a plasmid, a virus; a packaging plasmid; and an envelope plasmid. The packaging plasmid contains the nucleoside, capsid and matrix proteins. Examples of packaging plasmids are also described in the patent literature, e.g., U.S. Patent Nos. 7,262,049; 6,995,258; 7,252,991 and 5,710,037. The system also contains a plasmid encoding an envelope protein provided by an envelope plasmid.

This disclosure also provides the suitable packaging cell line. In one aspect, the packaging cell line is the HEK-293 cell line. Other suitable cell lines are known in the art, for example, described in the patent literature within U.S. Patent Nos. 7,070,994; 6,995,919; 6,475,786; 6,372,502; 6,365,150; and 5,591,624.

This invention further provides a method for producing an AAV particle, comprising, or alternatively consisting essentially of, or yet further consisting of, transducing a packaging cell line with the viral system as described above, under conditions suitable to package the viral vector. Such conditions are known in the art and briefly described herein. The viral particle can be isolated from the cell supernatant, using methods known to those of skill in the art, e.g., centrifugation. Such isolated particles are further provided by this invention.

This invention further provides the isolated AAV viral particles produced by this method. The viral particle comprises, or alternatively consists essentially of, or yet further consists of a polynucleotide as described herein.

### Host Cells

Yet further provided is an isolated cell or population of cells, comprising, or alternatively consisting essentially of, or yet further consisting of, isolated polynucleotides, viral particles, vectors and packaging systems as described above and incorporated herein by reference. In one aspect, the isolated cell is a packaging cell line.

Also provided is an isolated cell or population of cells, comprising, or alternatively consisting essentially of, or yet further consisting of, a polynucleotide sequence as described herein.

The isolated cells described herein can be any of a cell of a species of the group of: murine, rats, rabbit, simians, bovines, ovine, porcine, canines, feline, farm animals, sport animals, pets, equine, and primate, and in particular a human cell.

The vectors and cells can be contained within a composition that contains the vector and/or host cell and a carrier, e.g., a pharmaceutically acceptable carrier. They can be formulated for various modes of administration and contain an effective amount of the vector and/or host cell that is effective for the patient, the disorder or disease, the vector and mode of administration. In one aspect, the mode of administration is systemic or intravenous. In another aspect, administration is local by direct injection. In one aspect, the morpholino oligonucleotide is contacted as the same time or subsequent to the vector. Alternatively, the morpholino oligonucleotide is contacted prior to the vector.

### Methods of Use

The polynucleotides and vectors are useful to treat a variety of diseases or disorder. In one aspect, a method for delivering a transgene is provided. The method comprises administering an effective amount of the polynucleotide or vector comprising the transgene to the cell, tissue or patient to be treated. In one aspect, an effective amount of an antisense oligonucleotide (e.g., a morpholino oligonucleotide) is administered to the cell, tissue or patient to be treated. Non-limiting examples of transgenes are provided and are selected based on the purpose of the method. The cells or tissue can be mammalian, e.g. human. In one aspect, the antisense oligonucleotide (e.g., the morpholino oligonucleotide) is contacted as the same time or subsequent to the vector. Alternatively, it is prior to the vector. In another aspect, the vector is introduced to the cell by transfection, infection, transformation, electroporation, injection, microinjection, or the combination thereof.

Also provided herein is a method for treating a cancer in a subject in need thereof. The method comprises, or alternatively consists essentially of, or yet further consists of, administering to the subject an effective amount of the recombinant viral vector or cell as described herein. In a further aspect, the method further comprises administering to the subject an effective amount of an antisense oligonucleotide (e.g., a morpholino oligonucleotide). In one aspect, an effective amount of an anti-cancer agent is administered to the subject. Non-limiting examples of anti-cancer agents include anti-cancer peptides, polypeptides, nucleic acid molecules, small molecules, viral particles, or combinations thereof. In another aspect, the vector is introduced to the cell by transfection, infection, transformation, electroporation, injection, microinjection, or the combination thereof. The therapy can be administered as a first line, a second line, a third line, a fourth line, or a fifth line therapy. The therapy can be adjuvant or combined with other cancer therapies.

In one aspect of the disclosed methods, the viral particle is an oncolytic HSV particle.

### Administration

Administration of the recombinant polynucleotide and/or vector (e.g., AAV), viral particle or compositions of this disclosure can be effected in one dose, continuously or intermittently throughout the course of treatment. Administration may be through any suitable mode of administration, including but not limited to: intravenous, intra-arterial, intramuscular, intracardiac, intrathecal, subventricular, epidural, intracerebral, intracerebroventricular, sub-retinal, intravitreal, intraarticular, intraocular, intraperitoneal, intrauterine, intradermal, subcutaneous, transdermal, transmuccosal, and inhalation. In some instances, the mode of administration comprises parenteral administration. In one embodiment, the recombinant polynucleotide or vector or the composition is administered by intramuscular injection or intravenous injection. In another embodiment, the recombinant polynucleotide or vector or the composition is administered systemically. In another embodiment, the recombinant polynucleotide or vector or the composition is parentally administration by injection, infusion or implantation.

Methods of determining the most effective means and dosage of administration are known to those of skill in the art and will vary with the composition used for therapy, the purpose of the therapy and the subject being treated. Single or multiple administrations can be carried out with the dose level and pattern being selected by the treating physician. It is noted that dosage may be impacted by the route of administration. Suitable dosage formulations and methods of administering the agents are known in the art. Non-limiting examples of such suitable dosages may be as low as 1E+9 vector genomes to as much as 1E+17 vector genomes per administration.

In some embodiments of the methods described herein, the number of viral particles (e.g., AAV) administered to the subject ranges administered to the subject ranges from about 10⁹ to about 10¹⁷. In particular embodiments, about 10¹⁰ to about 10¹⁶, about 10¹⁰ to about 10¹⁵ about 10¹⁰ to about 10¹², about 10¹¹ to about 10¹³, about 10¹¹ to about 10¹², about 10¹¹ to about 10¹⁴, about 10¹¹ to about 10¹⁵, about 10¹¹ to about 10¹⁶, about 5x10¹¹ to about 5x10¹², or about 10¹² to about 10¹³ viral particles are administered to the subject. In some instances, about 10¹¹ to about 10¹² viral particles are administered to the subject. In some instances, about 10¹³ to about 10¹⁵ viral particles are administered to the subject. In some instances, about 10⁹ to about 10¹² viral particles are administered to the subject. In some instances, about 10⁹ to about 10¹¹ viral particles are administered to the subject. In some instances, the amount of the viral particle administered is based on the weight of the subject. One skilled in the art would understand how to modulate the amount of viral particles delivered so that the total amount delivered to a subject ranges from about 10⁹ to about 10¹⁷, optionally about 10¹⁰ to about 10¹⁶, about 10¹⁰ to about 10¹⁵ , about 10¹⁰ to about 10¹², about 10¹¹ to about 10¹³, about 10¹¹ to about 10¹², about 10¹¹ to about 10¹⁴, about 10¹¹ to about 10¹⁵, about 10¹¹ to about 10¹⁶, about 5x10¹¹ to about 5x10¹², or about 10¹² to about 10¹³ viral particles. In some cases, the subject is a pediatric subject (e.g., a subject less than 18 years of age). In some cases, about 10⁹ to about 10¹², about 10¹⁰ to about 10¹², about 10¹¹ to about 10¹², or about 10⁹ to about 10¹⁰ viral particles are administered to a pediatric subject.

In a further aspect, the viral particle and compositions of the disclosure can be administered in combination with other treatments, e.g., those approved treatments suitable for cancer and its associated disorders or conditions.

Successful treatment and/or repair is determined when one or more of the following is detected: alleviation or amelioration of one or more of symptoms of the treated subject's disease, disorder, or condition, diminishment of extent of the subject's disease, disorder, or condition, stabilized (i.e., not worsening) state of a disease, disorder, or condition, delay or slowing of the progression of the disease, disorder, or condition, and amelioration or palliation of the disease, disorder, or condition. In some embodiments, success of treatment is determined by detecting the presence repaired target polynucleotide in one or more cells, tissues, or organs isolated from the subject. In some embodiments, success of treatment is determined by detecting the presence polypeptide encoded by the repaired target polynucleotide in one or more cells, tissues, or organs isolated from the subject.

### Kits

The agents, vectors, or compositions described herein may, in some embodiments, be assembled into pharmaceutical or diagnostic or research kits to facilitate their use in therapeutic, diagnostic or research applications. In some embodiments, the kits of the present disclosure include one or more of: modified viral capsid proteins, isolated polynucleotides, vectors, host cells, recombinant viral particles, recombinant expression systems, modified AAV, modified cells, isolated tissues, compositions, or pharmaceutical compositions as described herein.

In some embodiments, a kit further comprises instructions for use. Specifically, such kits may include one or more agents described herein, along with instructions describing the intended application and the proper use of these agents. As an example, in one embodiment, the kit may include instructions for mixing one or more components of the kit and/or isolating and mixing a sample and applying to a subject. In certain embodiments, agents in a kit are in a pharmaceutical formulation and dosage suitable for a particular application and for a method of administration of the agents. Kits for research purposes may contain the components in appropriate concentrations or quantities for running various experiments.

The kit may be designed to facilitate use of the methods described herein and can take many forms. Each of the compositions of the kit, where applicable, may be provided in liquid form (e.g., in solution), or in solid form, (e.g., a dry powder). In certain cases, some of the compositions may be constitutable or otherwise processable (e.g., to an active form), for example, by the addition of a suitable solvent or other species (for example, water or a cell culture medium), which may or may not be provided with the kit. In some embodiments, the compositions may be provided in a preservation solution (e.g., cryopreservation solution). Non-limiting examples of preservation solutions include DMSO, paraformaldehyde, and CryoStor^{®} (Stem Cell Technologies, Vancouver, Canada). In some embodiments, the preservation solution contains an amount of metalloprotease inhibitors.

As used herein, "instructions" can define a component of instruction and/or promotion, and typically involve written instructions on or associated with packaging of the claimed methods, recombinant vectors, or compositions. Instructions also can include any oral or electronic instructions provided in any manner such that a user will clearly recognize that the instructions are to be associated with the kit, for example, audiovisual (e.g., videotape, DVD, etc.), internet, and/or web-based communications, etc. In some embodiments, the written instructions are in a form prescribed by a governmental agency regulating the manufacture, use or sale of pharmaceuticals or biological products, which instructions can also reflect approval by the agency of manufacture, use or sale for animal administration.

In some embodiments, the kit contains any one or more of the components described herein in one or more containers. Thus, in some embodiments, the kit may include a container housing agents described herein. The agents may be in the form of a liquid, gel or solid (powder). The agents may be prepared sterilely, packaged in syringe and shipped refrigerated. Alternatively, it may be housed in a vial or other container for storage. A second container may have other agents prepared sterilely. Alternatively, the kit may include the active agents premixed and shipped in a syringe, vial, tube, or other container. The kit may have one or more or all of the components required to administer the agents to a subject, such as a syringe, topical application devices, or IV needle tubing and bag.

The therapies as described herein can be combined with appropriate diagnostic techniques to identify and select patients for the therapy.

### Method of Production

In additional method provided by this disclosure is a method of producing a bispecific antibody or a trispecific antibody in a cell, comprising contacting a cell comprising a vector as described herein, with an effective amount of a morpholino oligonucleotide. In one embodiment, the morpholino oligonucleotide comprises a polynucleotide sequence at least 95% identical to SEQ ID NO. 27 (CAAGATTTACCTCTATTGTTGGATCATATT) or SEQ ID NO. 28 (TAAAACAAGATTTACCTCTATTGTTGGATC), or an equivalent of each thereof. In one aspect, the morpholino oligonucleotide is contacted as the same time or subsequent to the vector. Alternatively, it is prior to the vector. A non-limiting example of the bispecific antibody comprises a polypeptide sequence at least 95% identical to any one of SEQ ID NOs: 13 and 15, or alternatively at least 96%, or at least 97%, or at least 98%, or at least 99% sequence identity to any one of SEQ ID NOs: 13 and 15. In another aspect, trispecific antibody comprises a polypeptide sequence at least 95% identical to SEQ ID NO: 11, or alternatively at least 96%, or at least 97%, or at least 98%, or at least 99% sequence identity to SEQ ID NO: 11.

In another aspect, the vector is introduced to the cell by transfection, infection, transformation, electroporation, injection, microinjection, or the combination thereof. Non-limiting examples of cells include a fibroblast, a skeletal cell, an epithelial cell, a muscle cell, a neural cell, an endocrine cell, a melanocyte, a blood cell, or combination thereof.

Further provided are kits comprising one or more of the vector, cell or composition as described herein, optionally with instructional documents or material.

### Specified Embodiments

There are several ways to modulate the gene expression through the principles of exon-skipping.

In one aspect, at least three exons are needed in order to be able to skip an exon in this strategy. With a three-exon structure, there are two splice donor and two splice acceptor sites as illustrated in **FIG. 1****.** The first donor site is spliced to the first acceptor site ("a" in **FIG. 1****)** or, alternatively, to the second acceptor site ("c" in **FIG. 1****).** The second donor site can only splice to the second acceptor site ("b" in **FIG. 1****).** There are thus 5 possible RNA structures that can result from such a three exon / two intron structure, depending on whether no splicing occurs, only one splice event occurs (a, b, or c) or two splices occur (a+b).

Sequence-specific oligonucleotides ("oligos") are constructed that interfere specifically with each of the splicing events, typically at the site of the splice donor or acceptor sites or at a binding site for one of the spliceosome components (sometimes in the middle of an intron). For the purposes of this embodiment and as shown in **FIG. 1****,** each oligo is classified based on whether it interferes with a donor or an acceptor as 1-donor (1D), 2-acceptor (1A), 2-donor (2D), or 3-acceptor (3D).

For example, due to alternative splicing or interference by oligos, exon 2 of a gene is skipped in a cancer cell but remains in a normal cell. In this embodiment, a normal gene that contains an exon that is skipped does not express in cancer cells, but express a protein in normal cells. The sequences of the introns that flank that exon 2 in this construct can used to recapitulate the same splicing pattern in the transgene. It follows that a construct comprising a transgene in a typical intron-exon-intron structure that is spliced both in normal and tumor cells maintains a relatively efficient splicing and results in high levels of a fully spliced transcript (exons 1+2+3, utilizing splices a+b). Furthermore, when oligos block splicing of intron a or intron b (or both), exon 2 is skipped to generate transcripts that fuse exon 1 to exon 3. The relative expected abundance of the "theoretically possible" transcripts shown in **FIG. 1** are thus modified in **FIG. 2** to show the transcripts that are likely to be abundant.

In one embodiment, based on the above splicing concepts, the oligonucleotides and "exon skipping" technology are used to switch a transgene from a transcript containing exon 1, exon 2 and exon 6 to a transcript containing exon 1 and exon 3. Thus, a construct or a vector creates a functional polypeptide that is encoded by a transcript with exon 1 fused to exon 3. In one embodiment, a stop codon is engineered to be operably linked to exon 2 such that without interference by an oligonucleotide to skin exon 2, the transcription stops at the stop codon and before translation of exon 3. The premature stop codon leads to a non-functional transcript that contains exon 1, exon 2 and exon 3. In one embodiment, a stop codon is placed in all three reading frames to ensure a full stop. In that case, a baseline of a non-function transcript (exons 1+2+3) is switched to a functional transcript (exon 1+2) as illustrated in **FIG. 3****,** because ribosome stops translation of the transcript after the stop codon, which includes exon 3.

The construction of such a transgene is fairly straightforward then, but an intron-exonSTOP-intron structure that is appropriately "spliced in" under normal circumstances is selected. The intron-exonSTOP-intron can be engineered using sequences that flank a normally spliced exon, and cloned into a specific site in the transgene that results in splice donor and acceptor consensus sequences in the flanking bases. Because the exon in the normal gene from which the sequence was taken will also be skipped, the exon should be chosen carefully. For example, the intron-exon-intron could be from a spliced DNA virus gene, in which case no normal cellular genes would be expected to be affected. Alternatively, if the gene therapy application is for cancer, one might consider utilizing an intron-exon-intron boundary from an oncogene that might result in a defective oncogene. Such a strategy will leverage the off-target skipping of a cellular gene as potentially a "bonus" therapeutic effect.

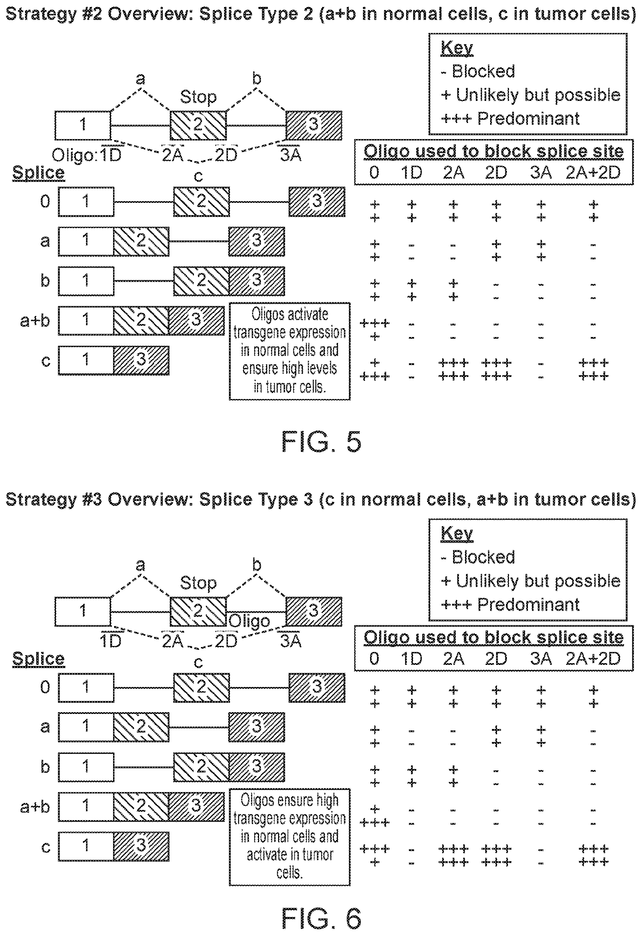

Some exons are differentially regulated in cancer cells compared with normal cells. For example, a splice type 2 exon is "spliced in" in normal cells, but excluded from the same gene in certain cancer cells. In that case, if one uses the intron-exon-intron boundary of a splice type 2 exon in the construction (see **FIG. 4****),** the baseline and skipped expression of the transgene will differ in normal versus cancer cells as shown in **FIG. 5****.** Because the engineered exonSTOP will be normally absent from cancer cells, the transgene will be activated in those cells but not normal cells **(****FIG. 5****).** Such an exon could be used to selectively express a transgene in cancer cells but not normal cells even in the absence of any exon skipping. Such a scenario might be desirable for a toxin gene or a pro-drug enzyme, for example. Skipping the exon 2 using an olio will activate the transgene in normal cells and may increase expression in cancer cells if the baseline skipping in those cells is less than 100%.

Conversely, a splice type 3 exon is excluded in normal cells, but "spliced in" in certain cancer cells. Leveraging the regulated splicing of such exon will thus give the opposite result as the splice type 2, as illustrated in **FIG. 6****.**

In summary, a strategy has been disclosed to achieve the following with respect to controlling transgene expression in the context of gene therapy:
Strategy #1: Activation of transgene expression is dependent on administration of an exon-skipping oligonucleotide. The effect is seen in both normal and cancer cells.
Strategy #2: Activation of transgene expression is normally high in certain cancer cells (only in certain cancers) and low in normal cells in the absence of any drug or externally added agent. This application could be used for cancer-selective expression for those cancers that have the appropriately regulated splicing. Administration of an exon-skipping oligonucleotide will activate expression in normal cells in addition to the specific cancer cells.
Strategy #3: Activation of transgene expression is high in normal cells, but low in certain cancer cells. Administration of an exon-skipping oligonucleotide activates expression in those cancer cells.

Although the principles of controllable gene expression can be applied to any gene therapy application, gene therapy for cancer treatment is a specific application. In this embodiment, a therapeutic drug that is expressed in and secreted from normal cells can be activated when needed via administration of the appropriate exon-skipping oligonucleotide.

For these purposes, Applicant has designed an intron-exonSTOP-intron cassette from exon 1 of the *KRAS* gene as an example of a construct of Strategy #1 to insert into a transgene of interest to achieve controllable gene expression. Oligos that induce exon skipping are selected for therapeutic application.

In one aspect, Applicant exploits the structure of the *KRAS* gene, which is among the most commonly mutated genes in cancer. KRAS has not been targetable by drugs because it is not an enzyme and has no obviously drug-binding pocket on its surface. Thus, to the best of Applicant's knowledge, this skipping technology is the first method to target KRAS in cancer. For these purposes, Applicant's construct utilizes the first exon of *KRAS* in the 5' untranslated region, and the ATG start codon for protein translation is inside of the 2^{nd} exon. (Thus, the 2^{nd} exon is often called exon 1 and the first exon is exon 0). An oligo that induces exon skip of the exon containing the ATG will result in a transcript that isn't translated normally because the ATG start codon is missing.

### KRAS-based intron-exonSTOP-intron

From the Genbank sequence (NCBI Reference Sequence: NG_007524.1, last accessed on February 20, 2019), the exons of KRAS are located at: 4990..5170,10526..10647,28509..28687,30148..30307, 46010..51132, whereas the cDNA joins: 10537..10647,28509..28687,30148..30307,46010..46126.

The first ATG of the normal *KRAS* gene is at position 10537
The exon to skip is between10526-10647 (122 bp).

The construct contains a fake version with STOP codons in each reading frame.

In one embodiment, for a *KRAS* 1 exon, the shaded and underlined nucleotides (including ATG of *KRAS*) is substituted with stop codons:

GCCTGCTGAAAATGACTGAATATAAACTTGTGGTAGTTGGAGCTGGTGGCGTAGGC
AAGAGTGCCTTGACGATACAGCTAATTCAGAATCATTTTGTGGACGAATATGATCC
AACAATAGAG (SEQ ID NO: 17).

Exemplary stop codons are: TAA, TAG, and TGA. In one aspect, all three reading frames are: TAAxTAGxTGA, wherein x is any nucleotide.

To design all 3 stop codons twice in a row, a stop codon can be inserted. In addition, one can avoid an 11 base pair repeat by using the sequence: TAAxTAGxTGAxTAGxTAAxTGAx (24 bp) (SEQ ID No: 1), wherein x is any nucleotide. A specific embodiment is: TAATTAGCTGAGTAGATAAGTGAT (SEQ ID No: 2). As a result, one embodiment containing the exonKras1STOP (the stop codons are underlined) is:

GCCTGCTGAAATAATTAGCTGAGTAGATAAGTGATGTTGGAGCTGGTGGCGTAGGC

AAGAGTGCCTTGACGATACAGCTAATTCAGAATCATTTTGTGGACGAATATGATCC
AACAATAGAG (SEQ ID NO. 18).

The normal upstream intron is 5171-10525 (5,355 bp) of NCBI Reference Sequence NG_007524.1: "gtacg... ataag", wherein the remainder portion of the upstream intron is shown as "...".

The normal downstream intron is 10648-28508 (17,861 bp) of NCBI Reference Sequence NG_007524.1: "gtaaa... ctcag", wherein the remainder portion of the downstream intron is shown as "...".

In one aspect, about 75 base pairs ("bps") on the upstream and downstream ends of both introns are included. In one embodiment, the upstream intron sequence is as follows:

GTACGGAGCGGACCACCCCTCCTGGGCCCCTGCCCGGGTCCCGACCCTCTTTGCCGG
CGCCGGGCGGGGCCGGCGGAGTATTTGATAGTGTATTAACCTTATGTGTGACATGTT
CTAATATAGTCACATTTTCATTATTTTTATTATAAG (SEQ ID NO. 19).

In one embodiment, the downstream intron sequence is as follows:

GTAAATCTTGTTTTAATATGCATATTACTGGTGCAGGACCATTCTTTGATACAGATA
AAGGTTTCTCTGACCATTTTGAGTTGTATATAACACCTTTTTTGAAGTAAAAGGTGC
ACTGTAATAATCCAGACTGTGTTTCTCCCTTCTCAG (SEQ ID NO. 20).

Therefore, in one embodiment, the following sequence is an intron-exonKras1STOP-intron sequence (the sequence with no highlight is upstream intron, the sequence in grey is intron containing STOP sequences, and the underlined sequence is downstream intron):

GTACGGAGCGGACCACCCCTCCTGGGCCCCTGCCCGGGTCCCGACCCTCTTTGCCGG
CGCCGGGCGGGGCCGGCGGAGTATTTGATAGTGTATTAACCTTATGTGTGACATGTT
CTAATATAGTCACATTTTCATTATTTTTATTATAAGGCCTGCTGAAATAATTAGCTGA
GTAGATAAGTGATGTTGGAGCTGGTGGCGTAGGCAAGAGTGCCTTGACGATACAGC
TAATTCAGAATCATTTTGTGGACGAATATGATCCAACAATAGAGGTAAATCTTGTTT
TAATATGCATATTACTGGTGCAGGACCATTCTTTGATACAGATAAAGGTTTCTCTGA
CCATTTTGAGTTGTATATAACACCTTTTTTGAAGTAAAAGGTGCACTGTAATAATCC
AGACTGTGTTTCTCCCTTCTCAG (SEQ ID NO. 21).

In one aspect, a series of oligos that span the junction of the exon-downstream intron boundary for their ability to cause exon skipping can be added. Exon skipping oligos tend to be 20-30 base pairs, antisense to the DNA.

In one embodiment, the morpholino binding site is a Kras1-derived intron-exonSTOP-intron morpholino site (SEQ ID NO. 26). The sequence of KlExonStopIntron 3' junction sequence listed below (exon is labelled as gray and intron is underlined): AATATGATCCAACAATAGAGGTAAATCTTGTTTTAATATGCATATTACTGG (SEQ ID NO. 26).

Applicant created and tested two morpholino binding sites, called KTS1 (SEQ ID NO. 24) & KTS2 (SEQ ID NO. 25). The KTS1 morpholino sequence comprises a sequence of SEQ ID NO. 27. And the KTS2 morpholino sequence comprises a sequence of SEQ ID NO. 28. Applicant used a KTS2 morpholino-inverted sequence (SEQ ID NO. 29) as a negative control.
SEQ ID NO. 24-KTS1 (KRAS TransSkip 1) (Exon labelled as gray and intron underlined)
   AATATGATCCAACAATAGAGGTAAATCTTG
SEQ ID NO. 27-KTS1 morpholino
   CAAGATTTACCTCTATTGTTGGATCATATT
SEQ ID NO. 25-KTS2 (KRAS TransSkip 2)
   GATCCAACAATAGAGGTAAATCTTGTTTTA
SEQ ID NO. 28-KTS2 morpholino
   TAAAACAAGATTTACCTCTATTGTTGGATC
SEQ ID NO. 29-KTS2 morpholino-inverted sequence
   CTAGGTTGTTATCTCCATTTAGAACAAAAT

In some instances, a splice donor site comprises A/C AG ***GT A/G AGT (SEQ ID NO: 34), in which "***" indicates the exon-intron boundary and also the site of insertion of an intron-exonSTOP-intron sequence.

In some instances, a splice acceptor comprises (Py)XCAG ***G G/T (SEQ ID NO: 35), in which "***" indicates the intron-exon boundary.

In some cases, exemplary splice donor sites for insertion of an intron-exonSTOP-intron sequence include AAG-GG (SEQ ID NO: 36), CAG-GG (SEQ ID NO: 37), AAG-GT (SEQ ID NO: 38), or CAG-GT (SEQ ID NO: 39), in which the "-" indicates the site of insertion.

### CATAAVERT CONSTRUCT

To create a Cancer Targeted **AAV** expressed, regulated T (CATAAVERT) linker (also being called a "TransSkip"), the intron-exonKras1STOP-intron sequence is inserted into sites of the coding region that generate consensus splice donor and acceptor sites. In one embodiment, the vector comprising the regulated CATAAVERT expresses CD3 and GD2.

In another embodiment, the vector comprises a sequence encoding a secrecon-AA-CD3xGD2-HDD dimert. For this construct, the sequence of secrecon-AA-CD3xGD2-HDD dimert comprises all of the potential sites (highlighted in grey) for insertion of an intron-exon Kras1 stop-intron to generate a consensus splice donor/acceptor (insert sequence after 3rd of 5 bp of one or more of them), is provided:

ATGTGGTGGAGACTGTGGTGGCTGCTGCTGCTGCTGCTGCTGTGGCCCATGGTG
TGGGCCGCCGCCCAGGTGCAGCTGCAGCAGAGCGGCCCCGAGCTGGTGAAGCCCGG
CGCCAGCGTGAAGATCAGCTGCAAGACCAGCGGCTACAAGTTCACCGAGTACACCA
TGCACTGGGTGAAGCAGAGCCACGGCAAGTGCCTGGAGTGGATCGGCGGCATCAAC
CCCAACAACGGCGGCACCAACTACAACCAGAAGTTCAAGGGCAAGGCCACCCTGA
CCGTGGACAAGAGCAGCAGCACCGCCTACATGGAGCTGAGAAGCCTGACCAGCGA
GGACAGCGCCGTGTACTACTGCGCCAGAGACACCACCGTGCCCTACGCCTACTGGG
GCCAGGGCACCACCGTGACCGTGAGCAGCGGCGGCGGCAGCGGCGGCGGCGG
CAGCGGCGGCGGCGGCAGCGACATCGAGCTGACCCAGAGCCCCGCCATCATGAGC
GCCAGCCCCGGCGAGAAGGTGACCATGACCTGCAGCGCCAGCAGCAGCATCAGCTA
CATGCACTGGTACCAGCAGAAGCCCGGCACCAGCCCCAAGAGATGGATCTACGACA
CCAGCAAGCTGGCCAGCAGCGTGCCCGCCAGATTCAGCGGCAGCGGCAGCGGCACC
AGCTACAGCCTGACCATCAGCAGCATGGAGGCCGAGGACGCCGCCACCTACTACTG
CCACCAGAGAAGCAGCTACCCCCTGACCTTCGGCTGCGGCACCAAGCTGGAGATCA
AGAGAGCCAGCACCAAGGGCCCCGGCGGCGGCGGCAGCGGCGGCGGCGGCAGCGG
CGGCGGCGGCAGCCAGGTGCAGCTGGTGCAGAGCGGCGGCGGCGTGGTGCAGCCC
GGCAGAAGCCTGAGACTGAGCTGCAAGGCCAGCGGCTACACCTTCACCAGATACAC
CATGCACTGGGTGAGACAGGCCCCCGGCAAGGGCCTGGAGTGGATCGGCTACATCA
ACCCCAGCAGAGGCTACACCAACTACAACCAGAAGTTCAAGGACAGATTCACCATC
AGCAGAGACAACAGCAAGAACACCGCCTTCCTGCAGATGGACAGCCTGAGACCCG
AGGACACCGGCGTGTACTTCTGCGCCAGATACTACGACGACCACTACTGCCTGGAC
TACTGGGGCCAGGGCACCCCCGTGACCGTGAGCAGCGGCGGCGGCGGCAGCGGCG
GCGGCGGCAGCGGCGGCGGCGGCAGCGACATCCAGATGACCCAGAGCCCCAGCAG
CCTGAGCGCCAGCGTGGGCGACAGAGTGACCATCACCTGCAGCGCCAGCAGCAGCG

TGAGCTACATGAACTGGTACCAGCAGACCCCCGGCAAGGCCCCCAAGAGATGGATC
TACGACACCAGCAAGCTGGCCAGCGGCGTGCCCAGCAGATTCAGCGGCAGCGGCA
GCGGCACCGACTACACCTTCACCATCAGCAGCCTGCAGCCCGAGGACATCGCCACC
TACTACTGCCAGCAGTGGAGCAGCAACCCCTTCACCTTCGGCCAGGGCACCAAGCT
GCAGATCACCAGAACCCCCCTGGGCGACACCACCCACACCAGCGGCATGGTGAGCA
AGCTGAGCCAGCTGCAGACCGAGCTGCTGGCCGCCCTGCTGGAGAGCGGCCTGAGC
AAGGAGGCCCTGATCCAGGCCCTGGGCGAGGGCAGCGGCGGCGCCCCCTAG (SEQ
ID NO. 22)

In one aspect, an insertion is made to the most upstream site to minimize the length of CD3xGD2 dimert that is translated prior to stop codon (the underlined sequence is a splice junction that is retained in mRNA when intron is spliced, the double underlined sequence is the splice donor site at beginning of an upstream intron sequence, the sequence in grey is an exon containing STOP sequences, and the sequence in bold is a downstream intron). In some instances, this sequence is termed CD3xGD2 K1 dimert:

ATGTGGTGGAGACTGTGGTGGCTGCTGCTGCTGCTGCTGCTGTGGCCCATGGTG
TGGGCCGCCGCCCAGGTGCAGCTGCAGCAGAGCGGCCCCGAGCTGGTGAAGCCCGG
CGCCAGCGTGAAGATCAGCTGCAAGACCAGCGGCTACAAGTTCACCGAGTACACCA
TGCACTGGGTGAAGCAGAGCCACGGCAAGTGCCTGGAGTGGATCGGCGGCATCAAC
CCCAACAACGGCGGCACCAACTACAACCAGAAGTTCAAGGTACGGAGCGGACCAC
CCCTCCTGGGCCCCTGCCCGGGTCCCGACCCTCTTTGCCGGCGCCGGGCGGGGCCGG
CGGAGTATTTGATAGTGTATTAACCTTATGTGTGACATGTTCTAATATAGTCACATT
TTCATTATTTTTATTATAAGGCCTGCTGAAATAATTAGCTGAGTAGATAAGTGATGT
TGGAGCTGGTGGCGTAGGCAAGAGTGCCTTGACGATACAGCTAATTCAGAATCATT
TTGTGGACGAATATGATCCAACAATAGAGGTAAATCTTGTTTTAATATGCATATT
ACTGGTGCAGGACCATTCTTTGATACAGATAAAGGTTTCTCTGACCATTTTGAG
TTGTATATAACACCTTTTTTGAAGTAAAAGGTGCACTGTAATAATCCAGACTGT
GTTTCTCCCTTCTCAGGGCAAGGCCACCCTGACCGTGGACAAGAGCAGCAGCACC
GCCTACATGGAGCTGAGAAGCCTGACCAGCGAGGACAGCGCCGTGTACTACTGCGC
CAGAGACACCACCGTGCCCTACGCCTACTGGGGCCAGGGCACCACCGTGACCGTGA
GCAGCGGCGGCGGCGGCAGCGGCGGCGGCGGCAGCGGCGGCGGCGGCAGCGACAT
CGAGCTGACCCAGAGCCCCGCCATCATGAGCGCCAGCCCCGGCGAGAAGGTGACCA
TGACCTGCAGCGCCAGCAGCAGCATCAGCTACATGCACTGGTACCAGCAGAAGCCC
GGCACCAGCCCCAAGAGATGGATCTACGACACCAGCAAGCTGGCCAGCAGCGTGCC

CGCCAGATTCAGCGGCAGCGGCAGCGGCACCAGCTACAGCCTGACCATCAGCAGCA
TGGAGGCCGAGGACGCCGCCACCTACTACTGCCACCAGAGAAGCAGCTACCCCCTG
ACCTTCGGCTGCGGCACCAAGCTGGAGATCAAGAGAGCCAGCACCAAGGGCCCCG
GCGGCGGCGGCAGCGGCGGCGGCAGCGGCGGCGGCAGCCAGGTGCAGCT
GGTGCAGAGCGGCGGCGGCGTGGTGCAGCCCGGCAGAAGCCTGAGACTGAGCTGC
AAGGCCAGCGGCTACACCTTCACCAGATACACCATGCACTGGGTGAGACAGGCCCC
CGGCAAGGGCCTGGAGTGGATCGGCTACATCAACCCCAGCAGAGGCTACACCAACT
ACAACCAGAAGTTCAAGGACAGATTCACCATCAGCAGAGACAACAGCAAGAACAC
CGCCTTCCTGCAGATGGACAGCCTGAGACCCGAGGACACCGGCGTGTACTTCTGCG
CCAGATACTACGACGACCACTACTGCCTGGACTACTGGGGCCAGGGCACCCCCGTG
ACCGTGAGCAGCGGCGGCGGCGGCAGCGGCGGCGGCGGCAGCGGCGGCGGCGGCA
GCGACATCCAGATGACCCAGAGCCCCAGCAGCCTGAGCGCCAGCGTGGGCGACAG
AGTGACCATCACCTGCAGCGCCAGCAGCAGCGTGAGCTACATGAACTGGTACCAGC
AGACCCCCGGCAAGGCCCCCAAGAGATGGATCTACGACACCAGCAAGCTGGCCAG
CGGCGTGCCCAGCAGATTCAGCGGCAGCGGCAGCGGCACCGACTACACCTTCACCA
TCAGCAGCCTGCAGCCCGAGGACATCGCCACCTACTACTGCCAGCAGTGGAGCAGC
AACCCCTTCACCTTCGGCCAGGGCACCAAGCTGCAGATCACCAGAACCCCCCTGGG
CGACACCACCCACACCAGCGGCATGGTGAGCAAGCTGAGCCAGCTGCAGACCGAG
CTGCTGGCCGCCCTGCTGGAGAGCGGCCTGAGCAAGGAGGCCCTGATCCAGGCCCT
GGGCGAGGGCAGCGGCGGCGCCCCCTAG (SEQ ID NO. 23).

In some instances, the upstream splicing factor binding site is modified (region modified is shown as italicized and bold). The underlined sequence is a splice junction that is retained in mRNA when intron is spliced, the double underlined sequence is the splice donor site at beginning of an upstream intron sequence, the sequence in grey is an exon containing STOP sequences, and the sequence in bold is a downstream intron. In some instances, this sequence is termed CD3xGD2 K2 dimert:

ATGTGGTGGAGACTGTGGTGGCTGCTGCTGCTGCTGCTGCTGTGGCCCATGGTG
TGGGCCGCCGCCCAGGTGCAGCTGCAGCAGAGCGGCCCCGAGCTGGTGAAGCCCGG
CGCCAGCGTGAAGATCAGCTGCAAGACCAGCGGCTACAAGTTCACCGAGTACACCA
TGCACTGGGTGAAGCAGAGCCACGGCAAGTGCCTGGAGTGGATCGGCGGCATCAAC
CCCAACAACGGCGGCACCAACTACAACCAGAAGTTCAAGGTACGGAGCGGACCAC
CCCTCCTGGGCCCCTGCCCGGGTCCCGACCCTCTTTGCCGGCGCCGGGCGGGGCCGG

CGGAGTATTTGATAGTGTATTAACCTTATGTGTGACATGTTCTAATATAGTCACATT
TTCCTTCTTTTTTTTTTCAGGCCTGCTGAAATAATTAGCTGAGTAGATAAGTGATGTT
GGAGCTGGTGGCGTAGGCAAGAGTGCCTTGACGATACAGCTAATTCAGAATCATTT
TGTGGACGAATATGATCCAACAATAGAGGTAAATCTTGTTTTAATATGCATATTA
CTGGTGCAGGACCATTCTTTGATACAGATAAAGGTTTCTCTGACCATTTTGAGT
TGTATATAACACCTTTTTTGAAGTAAAAGGTGCACTGTAATAATCCAGACTGTG
TTTCTCCCTTCTCAGGGCAAGGCCACCCTGACCGTGGACAAGAGCAGCAGCACCG
CCTACATGGAGCTGAGAAGCCTGACCAGCGAGGACAGCGCCGTGTACTACTGCGCC
AGAGACACCACCGTGCCCTACGCCTACTGGGGCCAGGGCACCACCGTGACCGTGAG
CAGCGGCGGCGGCGGCAGCGGCGGCGGCGGCAGCGGCGGCGGCGGCAGCGACATC
GAGCTGACCCAGAGCCCCGCCATCATGAGCGCCAGCCCCGGCGAGAAGGTGACCAT
GACCTGCAGCGCCAGCAGCAGCATCAGCTACATGCACTGGTACCAGCAGAAGCCCG
GCACCAGCCCCAAGAGATGGATCTACGACACCAGCAAGCTGGCCAGCAGCGTGCCC
GCCAGATTCAGCGGCAGCGGCAGCGGCACCAGCTACAGCCTGACCATCAGCAGCAT
GGAGGCCGAGGACGCCGCCACCTACTACTGCCACCAGAGAAGCAGCTACCCCCTGA
CCTTCGGCTGCGGCACCAAGCTGGAGATCAAGAGAGCCAGCACCAAGGGCCCCGGC
GGCGGCGGCAGCGGCGGCGGCGGCAGCGGCGGCGGCGGCAGCCAGGTGCAGCTGG
TGCAGAGCGGCGGCGGCGTGGTGCAGCCCGGCAGAAGCCTGAGACTGAGCTGCAA
GGCCAGCGGCTACACCTTCACCAGATACACCATGCACTGGGTGAGACAGGCCCCCG
GCAAGGGCCTGGAGTGGATCGGCTACATCAACCCCAGCAGAGGCTACACCAACTAC
AACCAGAAGTTCAAGGACAGATTCACCATCAGCAGAGACAACAGCAAGAACACCG
CCTTCCTGCAGATGGACAGCCTGAGACCCGAGGACACCGGCGTGTACTTCTGCGCC
AGATACTACGACGACCACTACTGCCTGGACTACTGGGGCCAGGGCACCCCCGTGAC
CGTGAGCAGCGGCGGCGGCGGCAGCGGCGGCGGCGGCAGCGGCGGCGGCGGCAGC
GACATCCAGATGACCCAGAGCCCCAGCAGCCTGAGCGCCAGCGTGGGCGACAGAG
TGACCATCACCTGCAGCGCCAGCAGCAGCGTGAGCTACATGAACTGGTACCAGCAG
ACCCCCGGCAAGGCCCCCAAGAGATGGATCTACGACACCAGCAAGCTGGCCAGCG
GCGTGCCCAGCAGATTCAGCGGCAGCGGCAGCGGCACCGACTACACCTTCACCATC
AGCAGCCTGCAGCCCGAGGACATCGCCACCTACTACTGCCAGCAGTGGAGCAGCAA
CCCCTTCACCTTCGGCCAGGGCACCAAGCTGCAGATCACCAGAACCCCCCTGGGCG
ACACCACCCACACCAGCGGCATGGTGAGCAAGCTGAGCCAGCTGCAGACCGAGCTG
CTGGCCGCCCTGCTGGAGAGCGGCCTGAGCAAGGAGGCCCTGATCCAGGCCCTGGG
CGAGGGCAGCGGCGGCGCCCCCTAG (SEQ ID NO: 40).

In some instances, the CD3xGD2 dimert construct comprises one or more additional modifications in the polynucleotide sequence. In some instances, the CD3xGD2 dimert construct is sec2A-CD3xGD2-HDD-K3 and the sequence of sec2A-CD3xGD2-HDD-K3 is set forth as SEQ ID NO: 41. The underlined sequence is a splice junction that is retained in mRNA when intron is spliced, the double underlined sequence is the splice donor site at beginning of an upstream intron sequence, the sequence in grey is an exon containing STOP sequences, and the sequence in bold is a downstream intron.

SEQ ID NO: 41
ATGTGGTGGAGACTGTGGTGGCTGCTGCTGCTGCTGCTGCTGTGGCCCATGGTG
TGGGCCGCCGCCCAGGTGCAGCTGCAGCAGAGCGGCCCCGAGCTGGTGAAGCCCGG
CGCCAGCGTGAAGATCAGCTGCAAGACCAGCGGCTACAAGTTCACCGAGTACACCA
TGCACTGGGTGAAGCAGAGCCACGGCAAGTGCCTGGAGTGGATCGGCGGCATCAAC
CCCAACAACGGCGGCACCAACTACAACCAGAAGTTCAAGGTACGGAGCGGACCAC
CCCTCCTGGGCCCCTGCCCGGGTCCCGACCCTCTTTGCCGGCGCCGGGCGGGGCCGG
CGGAGTATTTGATAGTGTATTAACCTTATGTGTGACATGTTCTAATATAGTCACATT
TTCCTTCTTTTTTTTTTAAGGCCTGCTGAAATAATTAGCTGAGTAGATAAGTGATGTT
GGAGCTGGTGGCGTAGGCAAGAGTGCCTTGACGATACAGCTAATTCAGAATCATTT
TGTGGACGAATATGATCCAACAATAGAGGTAAATCTTGTTTTAATATGCATATTA
CTGGTGCAGGACCATTCTTTGATACAGATAAAGGTTTCTCTGACCATTTTGAGT
TGTATATAACACCTTTTTTGAAGTAAAAGGTGCACTGTAATAATCCAGACTGTG
TTTCTCCCTTCTCAGGGCAAGGCCACCCTGACCGTGGACAAGAGCAGCAGCACCG
CCTACATGGAGCTGAGAAGCCTGACCAGCGAGGACAGCGCCGTGTACTACTGCGCC
AGAGACACCACCGTGCCCTACGCCTACTGGGGCCAGGGCACCACCGTGACCGTGAG
CAGCGGCGGCGGCGGCAGCGGCGGCGGCGGCAGCGGCGGCGGCGGCAGCGACATC
GAGCTGACCCAGAGCCCCGCCATCATGAGCGCCAGCCCCGGCGAGAAGGTGACCAT
GACCTGCAGCGCCAGCAGCAGCATCAGCTACATGCACTGGTACCAGCAGAAGCCCG
GCACCAGCCCCAAGAGATGGATCTACGACACCAGCAAGCTGGCCAGCAGCGTGCCC
GCCAGATTCAGCGGCAGCGGCAGCGGCACCAGCTACAGCCTGACCATCAGCAGCAT
GGAGGCCGAGGACGCCGCCACCTACTACTGCCACCAGAGAAGCAGCTACCCCCTGA
CCTTCGGCTGCGGCACCAAGCTGGAGATCAAGAGAGCCAGCACCAAGGGCCCCGGC
GGCGGCGGCAGCGGCGGCGGCGGCAGCGGCGGCGGCGGCAGCCAGGTGCAGCTGG
TGCAGAGCGGCGGCGGCGTGGTGCAGCCCGGCAGAAGCCTGAGACTGAGCTGCAA

GGCCAGCGGCTACACCTTCACCAGATACACCATGCACTGGGTGAGACAGGCCCCCG
GCAAGGGCCTGGAGTGGATCGGCTACATCAACCCCAGCAGAGGCTACACCAACTAC
AACCAGAAGTTCAAGGACAGATTCACCATCAGCAGAGACAACAGCAAGAACACCG
CCTTCCTGCAGATGGACAGCCTGAGACCCGAGGACACCGGCGTGTACTTCTGCGCC
AGATACTACGACGACCACTACTGCCTGGACTACTGGGGCCAGGGCACCCCCGTGAC
CGTGAGCAGCGGCGGCGGCAGCGGCGGCGGCAGCGGCGGCGGCAGC
GACATCCAGATGACCCAGAGCCCCAGCAGCCTGAGCGCCAGCGTGGGCGACAGAG
TGACCATCACCTGCAGCGCCAGCAGCAGCGTGAGCTACATGAACTGGTACCAGCAG
ACCCCCGGCAAGGCCCCCAAGAGATGGATCTACGACACCAGCAAGCTGGCCAGCG
GCGTGCCCAGCAGATTCAGCGGCAGCGGCAGCGGCACCGACTACACCTTCACCATC
AGCAGCCTGCAGCCCGAGGACATCGCCACCTACTACTGCCAGCAGTGGAGCAGCAA
CCCCTTCACCTTCGGCCAGGGCACCAAGCTGCAGATCACCAGAACCCCCCTGGGCG
ACACCACCCACACCAGCGGCATGGTGAGCAAGCTGAGCCAGCTGCAGACCGAGCTG
CTGGCCGCCCTGCTGGAGAGCGGCCTGAGCAAGGAGGCCCTGATCCAGGCCCTGGG
CGAGGGCAGCGGCGGCGCCCCCTAG

In some instances, the CD3xGD2 dimert construct is sec2A-CD3xGD2-HDD-K4 and the sequence of sec2A-CD3xGD2-HDD-K4 is set forth as SEQ ID NO: 42. The underlined sequence is a splice junction that is retained in mRNA when intron is spliced, the double underlined sequence is the splice donor site at beginning of an upstream intron sequence, the sequence in grey is an exon containing STOP sequences, and the sequence in bold is a downstream intron.

SEQ ID NO: 42

ATGTGGTGGAGACTGTGGTGGCTGCTGCTGCTGCTGCTGCTGTGGCCCATGGTG

TGGGCCGCCGCCCAGGTGCAGCTGCAGCAGAGCGGCCCCGAGCTGGTGAAGCCCGG

CGCCAGCGTGAAGATCAGCTGCAAGACCAGCGGCTACAAGTTCACCGAGTACACCA

TGCACTGGGTGAAGCAGAGCCACGGCAAGTGCCTGGAGTGGATCGGCGGCATCAAC

CCCAACAACGGCGGCACCAACTACAACCAGAAGTTCAAGGTACGGAGCGGACCAC

CCCTCCTGGGCCCCTGCCCGGGTCCCGACCCTCTTTGCCGGCGCCGGGCGGGGCCGG

CGGAGTATTTGATAGTGTATTAACCTTATGTGTGACATGTTCTAATATAGTCACATT

TTCCTTCTTTTTATTATAAGCCTGCTGAAATAATTAGCTGAGTAGATAAGTGATGTT

GGAGCTGGTGGCGTAGGCAAGAGTGCCTTGACGATACAGCTAATTCAGAATCATTT

TGTGGACGAATATGATCCAACAATAGAGGTAAATCTTGTTTTAATATGCATATTA

CTGGTGCAGGACCATTCTTTGATACAGATAAAGGTTTCTCTGACCATTTTGAGT
TGTATATAACACCTTTTTTGAAGTAAAAGGTGCACTGTAATAATCCAGACTGTG
TTTCTCCCTTCTCAGGGCAAGGCCACCCTGACCGTGGACAAGAGCAGCAGCACCG
CCTACATGGAGCTGAGAAGCCTGACCAGCGAGGACAGCGCCGTGTACTACTGCGCC
AGAGACACCACCGTGCCCTACGCCTACTGGGGCCAGGGCACCACCGTGACCGTGAG
CAGCGGCGGCGGCGGCAGCGGCGGCGGCGGCAGCGGCGGCGGCGGCAGCGACATC
GAGCTGACCCAGAGCCCCGCCATCATGAGCGCCAGCCCCGGCGAGAAGGTGACCAT
GACCTGCAGCGCCAGCAGCAGCATCAGCTACATGCACTGGTACCAGCAGAAGCCCG
GCACCAGCCCCAAGAGATGGATCTACGACACCAGCAAGCTGGCCAGCAGCGTGCCC
GCCAGATTCAGCGGCAGCGGCAGCGGCACCAGCTACAGCCTGACCATCAGCAGCAT
GGAGGCCGAGGACGCCGCCACCTACTACTGCCACCAGAGAAGCAGCTACCCCCTGA
CCTTCGGCTGCGGCACCAAGCTGGAGATCAAGAGAGCCAGCACCAAGGGCCCCGGC
GGCGGCGGCAGCGGCGGCGGCGGCAGCGGCGGCGGCGGCAGCCAGGTGCAGCTGG
TGCAGAGCGGCGGCGGCGTGGTGCAGCCCGGCAGAAGCCTGAGACTGAGCTGCAA
GGCCAGCGGCTACACCTTCACCAGATACACCATGCACTGGGTGAGACAGGCCCCCG
GCAAGGGCCTGGAGTGGATCGGCTACATCAACCCCAGCAGAGGCTACACCAACTAC
AACCAGAAGTTCAAGGACAGATTCACCATCAGCAGAGACAACAGCAAGAACACCG
CCTTCCTGCAGATGGACAGCCTGAGACCCGAGGACACCGGCGTGTACTTCTGCGCC
AGATACTACGACGACCACTACTGCCTGGACTACTGGGGCCAGGGCACCCCCGTGAC
CGTGAGCAGCGGCGGCGGCGGCAGCGGCGGCGGCGGCAGCGGCGGCGGCGGCAGC
GACATCCAGATGACCCAGAGCCCCAGCAGCCTGAGCGCCAGCGTGGGCGACAGAG
TGACCATCACCTGCAGCGCCAGCAGCAGCGTGAGCTACATGAACTGGTACCAGCAG
ACCCCCGGCAAGGCCCCCAAGAGATGGATCTACGACACCAGCAAGCTGGCCAGCG
GCGTGCCCAGCAGATTCAGCGGCAGCGGCAGCGGCACCGACTACACCTTCACCATC
AGCAGCCTGCAGCCCGAGGACATCGCCACCTACTACTGCCAGCAGTGGAGCAGCAA
CCCCTTCACCTTCGGCCAGGGCACCAAGCTGCAGATCACCAGAACCCCCCTGGGCG
ACACCACCCACACCAGCGGCATGGTGAGCAAGCTGAGCCAGCTGCAGACCGAGCTG
CTGGCCGCCCTGCTGGAGAGCGGCCTGAGCAAGGAGGCCCTGATCCAGGCCCTGGG
CGAGGGCAGCGGCGGCGCCCCCTAG

In some instances, the CD3xGD2 dimert construct is sec2A-CD3xGD2-HDD-K5 and the sequence of sec2A-CD3xGD2-HDD-K5 is set forth as SEQ ID NO: 43. The underlined sequence is a splice junction that is retained in mRNA when intron is spliced, the double underlined sequence is the splice donor site at beginning of an upstream intron sequence, the sequence in grey is an exon containing STOP sequences, and the sequence in bold is a downstream intron.

SEQ ID NO: 43
ATGTGGTGGAGACTGTGGTGGCTGCTGCTGCTGCTGCTGCTGTGGCCCATGGTG
TGGGCCGCCGCCCAGGTGCAGCTGCAGCAGAGCGGCCCCGAGCTGGTGAAGCCCGG
CGCCAGCGTGAAGATCAGCTGCAAGACCAGCGGCTACAAGTTCACCGAGTACACCA
TGCACTGGGTGAAGCAGAGCCACGGCAAGTGCCTGGAGTGGATCGGCGGCATCAAC
CCCAACAACGGCGGCACCAACTACAACCAGAAGTTCAAGGTACGGAGCGGACCAC
CCCTCCTGGGCCCCTGCCCGGGTCCCGACCCTCTTTGCCGGCGCCGGGCGGGGCCGG
CGGAGTATTTGATAGTGTATTAACCTTATGTGTGACATGTTCTAATATAGTCACATT
TTCATTATTTTTTTTTTCAGGCCTGCTGAAATAATTAGCTGAGTAGATAAGTGATGTT
GGAGCTGGTGGCGTAGGCAAGAGTGCCTTGACGATACAGCTAATTCAGAATCATTT
TGTGGACGAATATGATCCAACAATAGAGGTAAATCTTGTTTTAATATGCATATTA
CTGGTGCAGGACCATTCTTTGATACAGATAAAGGTTTCTCTGACCATTTTGAGT
TGTATATAACACCTTTTTTGAAGTAAAAGGTGCACTGTAATAATCCAGACTGTG
TTTCTCCCTTCTCAGGGCAAGGCCACCCTGACCGTGGACAAGAGCAGCAGCACCG
CCTACATGGAGCTGAGAAGCCTGACCAGCGAGGACAGCGCCGTGTACTACTGCGCC
AGAGACACCACCGTGCCCTACGCCTACTGGGGCCAGGGCACCACCGTGACCGTGAG
CAGCGGCGGCGGCAGCGGCGGCGGCAGCGGCGGCGGCAGCGACATC
GAGCTGACCCAGAGCCCCGCCATCATGAGCGCCAGCCCCGGCGAGAAGGTGACCAT
GACCTGCAGCGCCAGCAGCAGCATCAGCTACATGCACTGGTACCAGCAGAAGCCCG
GCACCAGCCCCAAGAGATGGATCTACGACACCAGCAAGCTGGCCAGCAGCGTGCCC
GCCAGATTCAGCGGCAGCGGCAGCGGCACCAGCTACAGCCTGACCATCAGCAGCAT
GGAGGCCGAGGACGCCGCCACCTACTACTGCCACCAGAGAAGCAGCTACCCCCTGA
CCTTCGGCTGCGGCACCAAGCTGGAGATCAAGAGAGCCAGCACCAAGGGCCCCGGC
GGCGGCGGCAGCGGCGGCGGCGGCAGCGGCGGCGGCGGCAGCCAGGTGCAGCTGG
TGCAGAGCGGCGGCGGCGTGGTGCAGCCCGGCAGAAGCCTGAGACTGAGCTGCAA
GGCCAGCGGCTACACCTTCACCAGATACACCATGCACTGGGTGAGACAGGCCCCCG
GCAAGGGCCTGGAGTGGATCGGCTACATCAACCCCAGCAGAGGCTACACCAACTAC
AACCAGAAGTTCAAGGACAGATTCACCATCAGCAGAGACAACAGCAAGAACACCG
CCTTCCTGCAGATGGACAGCCTGAGACCCGAGGACACCGGCGTGTACTTCTGCGCC
AGATACTACGACGACCACTACTGCCTGGACTACTGGGGCCAGGGCACCCCCGTGAC

CGTGAGCAGCGGCGGCGGCGGCAGCGGCGGCGGCGGCAGCGGCGGCGGCGGCAGC
GACATCCAGATGACCCAGAGCCCCAGCAGCCTGAGCGCCAGCGTGGGCGACAGAG
TGACCATCACCTGCAGCGCCAGCAGCAGCGTGAGCTACATGAACTGGTACCAGCAG
ACCCCCGGCAAGGCCCCCAAGAGATGGATCTACGACACCAGCAAGCTGGCCAGCG
GCGTGCCCAGCAGATTCAGCGGCAGCGGCAGCGGCACCGACTACACCTTCACCATC
AGCAGCCTGCAGCCCGAGGACATCGCCACCTACTACTGCCAGCAGTGGAGCAGCAA
CCCCTTCACCTTCGGCCAGGGCACCAAGCTGCAGATCACCAGAACCCCCCTGGGCG
ACACCACCCACACCAGCGGCATGGTGAGCAAGCTGAGCCAGCTGCAGACCGAGCTG
CTGGCCGCCCTGCTGGAGAGCGGCCTGAGCAAGGAGGCCCTGATCCAGGCCCTGGG
CGAGGGCAGCGGCGGCGCCCCCTAG

In some embodiments, a dimert described herein comprises a CD3 sequence, a CD19 sequence, and optionally a secrecon sequence. In some instances, the dimert comprises a CD3xCD19 construct as set forth in SEQ ID NO: 44 highlighting all of the potential sites for insertion of an intron-exon-Kras1 stop-intron sequence to generate a consensus splice donor/acceptor site. Each potential insertion site is highlighted in gray.

SEQ ID NO: 44

ATGTGGTGGAGACTGTGGTGGCTGCTGCTGCTGCTGCTGCTGTGGCCCATGGTG

TGGGCCGCCGACATCCAGCTGACCCAGAGCCCCGCCAGCCTGGCCGTGAGCCTGGG

CCAGAGAGCCACCATCAGCTGCAAGGCCAGCCAGAGCGTGGACTACGACGGCGAC

AGCTACCTGAACTGGTACCAGCAGATCCCCGGCCAGCCCCCCAAGCTGCTGATCTA

CGACGCCAGCAACCTGGTGAGCGGCATCCCCCCCAGATTCAGCGGCAGCGGCAGCG

GCACCGACTTCACCCTGAACATCCACCCCGTGGAGAAGGTGGACGCCGCCACCTAC

CACTGCCAGCAGAGCACCGAGGACCCCTGGACCTTCGGCGGCGGCACCAAGCTGGA

GATCAAGGGCGGCGGCGGCAGCGGCGGCGGCGGCAGCGGCGGCGGCGGCAGCCAG

GTGCAGCTGCAGCAGAGCGGCGCCGAGCTGGTGAGACCCGGCAGCAGCGTGAAGA

TCAGCTGCAAGGCCAGCGGCTACGCCTTCAGCAGCTACTGGATGAACTGGGTGAAG

CAGAGACCCGGCCAGGGCCTGGAGTGGATCGGCCAGATCTGGCCCGGCGACGGCG

ACACCAACTACAACGGCAAGTTCAAGGGCAAGGCCACCCTGACCGCCGACGAGAG

CAGCAGCACCGCCTACATGCAGCTGAGCAGCCTGGCCAGCGAGGACAGCGCCGTGT

ACTTCTGCGCCAGAAGAGAGACCACCACCGTGGGCAGATACTACTACGCCATGGAC

TACTGGGGCCAGGGCACCACCGTGACCGTGAGCAGCGGCGGCGGCGGCAGCGACA

TCAAGCTGCAGCAGAGCGGCGCCGAGCTGGCCAGACCCGGCGCCAGCGTGAAGAT

GAGCTGCAAGACCAGCGGCTACACCTTCACCAGATACACCATGCACTGGGTGAAGC
AGAGACCCGGCCAGGGCCTGGAGTGGATCGGCTACATCAACCCCAGCAGAGGCTAC
ACCAACTACAACCAGAAGTTCAAGGACAAGGCCACCCTGACCACCGACAAGAGCA
GCAGCACCGCCTACATGCAGCTGAGCAGCCTGACCAGCGAGGACAGCGCCGTGTAC
TACTGCGCCAGATACTACGACGACCACTACTGCCTGGACTACTGGGGCCAGGGCAC
CACCCTGACCGTGAGCAGCGTGGAGGGCGGCAGCGGCGGCAGCGGCGGCAGCGGC
GGCAGCGGCGGCGTGGACGACATCCAGCTGACCCAGAGCCCCGCCATCATGAGCGC
CAGCCCCGGCGAGAAGGTGACCATGACCTGCAGAGCCAGCAGCAGCGTGAGCTAC
ATGAACTGGTACCAGCAGAAGAGCGGCACCAGCCCCAAGAGATGGATCTACGACA
CCAGCAAGGTGGCCAGCGGCGTGCCCTACAGATTCAGCGGCAGCGGCAGCGGCACC
AGCTACAGCCTGACCATCAGCAGCATGGAGGCCGAGGACGCCGCCACCTACTACTG
CCAGCAGTGGAGCAGCAACCCCCTGACCTTCGGCGCCGGCACCAAGCTGGAGCTGA
AGCACCACCACCACCACCACTAG

In some instances, an insertion is made to the most upstream site. In some instances, the dimert further comprises a secrecon sequence. In some cases, the CD3xCD19 construct is sec1A-CD3xCD19-K1 and its sequence is set forth as SEQ ID NO: 45. As shown below, the underlined sequence is a splice junction that is retained in mRNA when intron is spliced, the italicized and bold sequence is the upstream intron sequence, the sequence in grey is an exon containing STOP sequences, and the sequence in bold is a downstream intron.

SEQ ID NO: 45
ATGTGGTGGAGACTGTGGTGGCTGCTGCTGCTGCTGCTGCTGTGGCCCATGGTG
TGGGCCGCCGACATCCAGCTGACCCAGAGCCCCGCCAGCCTGGCCGTGAGCCTGGG
CCAGAGAGCCACCATCAGCTGCAAGGCCAGCCAGAGCGTGGACTACGACGGCGAC
AGCTACCTGAACTGGTACCAGCAGATCCCCGGCCAGCCCCCCAAGCTGCTGATCTA
CGACGCCAGCAACCTGGTGAGCGGCATCCCCCCCAGATTCAGCGGCAGCGGCAGCG
GCACCGACTTCACCCTGAACATCCACCCCGTGGAGAAGGTACGGAGCGGACCACCC
CTCCTGGGCCCCTGCCCGGGTCCCGACCCTCTTTGCCGGCGCCGGGCGGGGCCGGCG
GAGTATTTGATAGTGTATTAACCTTATGTGTGACATGTTCTAATATAGTCACATTTTCA
TTATTTTTATTATAAGGCCTGCTGAAATAATTAGCTGAGTAGATAAGTGATGTTGGA
GCTGGTGGCGTAGGCAAGAGTGCCTTGACGATACAGCTAATTCAGAATCATTTTGT
GGACGAATATGATCCAACAATAGAGGTAAATCTTGTTTTAATATGCATATTACTG
GTGCAGGACCATTCTTTGATACAGATAAAGGTTTCTCTGACCATTTTGAGTTGT

ATATAACACCTTTTTTGAAGTAAAAGGTGCACTGTAATAATCCAGACTGTGTTT
CTCCCTTCTCAGGTGGACGCCGCCACCTACCACTGCCAGCAGAGCACCGAGGACC
CCTGGACCTTCGGCGGCGGCACCAAGCTGGAGATCAAGGGCGGCGGCAGCGG
CGGCGGCGGCAGCGGCGGCGGCGGCAGCCAGGTGCAGCTGCAGCAGAGCGGCGCC
GAGCTGGTGAGACCCGGCAGCAGCGTGAAGATCAGCTGCAAGGCCAGCGGCTACG
CCTTCAGCAGCTACTGGATGAACTGGGTGAAGCAGAGACCCGGCCAGGGCCTGGAG
TGGATCGGCCAGATCTGGCCCGGCGACGGCGACACCAACTACAACGGCAAGTTCAA
GGGCAAGGCCACCCTGACCGCCGACGAGAGCAGCAGCACCGCCTACATGCAGCTG
AGCAGCCTGGCCAGCGAGGACAGCGCCGTGTACTTCTGCGCCAGAAGAGAGACCAC
CACCGTGGGCAGATACTACTACGCCATGGACTACTGGGGCCAGGGCACCACCGTGA
CCGTGAGCAGCGGCGGCGGCAGCGACATCAAGCTGCAGCAGAGCGGCGCCGA
GCTGGCCAGACCCGGCGCCAGCGTGAAGATGAGCTGCAAGACCAGCGGCTACACCT
TCACCAGATACACCATGCACTGGGTGAAGCAGAGACCCGGCCAGGGCCTGGAGTGG
ATCGGCTACATCAACCCCAGCAGAGGCTACACCAACTACAACCAGAAGTTCAAGGA
CAAGGCCACCCTGACCACCGACAAGAGCAGCAGCACCGCCTACATGCAGCTGAGCA
GCCTGACCAGCGAGGACAGCGCCGTGTACTACTGCGCCAGATACTACGACGACCAC
TACTGCCTGGACTACTGGGGCCAGGGCACCACCCTGACCGTGAGCAGCGTGGAGGG
CGGCAGCGGCGGCAGCGGCGGCAGCGGCGGCAGCGGCGGCGTGGACGACATCCAG
CTGACCCAGAGCCCCGCCATCATGAGCGCCAGCCCCGGCGAGAAGGTGACCATGAC
CTGCAGAGCCAGCAGCAGCGTGAGCTACATGAACTGGTACCAGCAGAAGAGCGGC
ACCAGCCCCAAGAGATGGATCTACGACACCAGCAAGGTGGCCAGCGGCGTGCCCTA
CAGATTCAGCGGCAGCGGCAGCGGCACCAGCTACAGCCTGACCATCAGCAGCATGG
AGGCCGAGGACGCCGCCACCTACTACTGCCAGCAGTGGAGCAGCAACCCCCTGACC
TTCGGCGCCGGCACCAAGCTGGAGCTGAAGCACCACCACCACCACCACTAG

In some instances, the CD3xCD19 construct is sec1A-CD3xCD19-K3 and its sequence is set forth as SEQ ID NO: 46. As shown below, the underlined sequence is a splice junction that is retained in mRNA when intron is spliced, the italicized and bold region is a modified region of the upstream splicing factor binding site, the sequence in grey is an exon containing STOP sequences, and the sequence in bold is a downstream intron.

SEQ ID NO: 46
ATGTGGTGGAGACTGTGGTGGCTGCTGCTGCTGCTGCTGCTGTGGCCCATGGTG
TGGGCCGCCGACATCCAGCTGACCCAGAGCCCCGCCAGCCTGGCCGTGAGCCTGGG

CCAGAGAGCCACCATCAGCTGCAAGGCCAGCCAGAGCGTGGACTACGACGGCGAC
AGCTACCTGAACTGGTACCAGCAGATCCCCGGCCAGCCCCCCAAGCTGCTGATCTA
CGACGCCAGCAACCTGGTGAGCGGCATCCCCCCCAGATTCAGCGGCAGCGGCAGCG
GCACCGACTTCACCCTGAACATCCACCCCGTGGAGAAGGTACGGAGCGGACCACCC
CTCCTGGGCCCCTGCCCGGGTCCCGACCCTCTTTGCCGGCGCCGGGCGGGGCCGGC
GGAGTATTTGATAGTGTATTAACCTTATGTGTGACATGTTCTAATATAGTCACATTTT
CCTTCTTTTTTTTTTAAGGCCTGCTGAAATAATTAGCTGAGTAGATAAGTGATGTTGGA
GCTGGTGGCGTAGGCAAGAGTGCCTTGACGATACAGCTAATTCAGAATCATTTTGT
GGACGAATATGATCCAACAATAGAGGTAAATCTTGTTTTAATATGCATATTACTG
GTGCAGGACCATTCTTTGATACAGATAAAGGTTTCTCTGACCATTTTGAGTTGT
ATATAACACCTTTTTTGAAGTAAAAGGTGCACTGTAATAATCCAGACTGTGTTT
CTCCCTTCTCAGGTGGACGCCGCCACCTACCACTGCCAGCAGAGCACCGAGGACC
CCTGGACCTTCGGCGGCGGCACCAAGCTGGAGATCAAGGGCGGCGGCGGCAGCGG
CGGCGGCGGCAGCGGCGGCGGCGGCAGCCAGGTGCAGCTGCAGCAGAGCGGCGCC
GAGCTGGTGAGACCCGGCAGCAGCGTGAAGATCAGCTGCAAGGCCAGCGGCTACG
CCTTCAGCAGCTACTGGATGAACTGGGTGAAGCAGAGACCCGGCCAGGGCCTGGAG
TGGATCGGCCAGATCTGGCCCGGCGACGGCGACACCAACTACAACGGCAAGTTCAA
GGGCAAGGCCACCCTGACCGCCGACGAGAGCAGCAGCACCGCCTACATGCAGCTG
AGCAGCCTGGCCAGCGAGGACAGCGCCGTGTACTTCTGCGCCAGAAGAGAGACCAC
CACCGTGGGCAGATACTACTACGCCATGGACTACTGGGGCCAGGGCACCACCGTGA
CCGTGAGCAGCGGCGGCGGCGGCAGCGACATCAAGCTGCAGCAGAGCGGCGCCGA
GCTGGCCAGACCCGGCGCCAGCGTGAAGATGAGCTGCAAGACCAGCGGCTACACCT
TCACCAGATACACCATGCACTGGGTGAAGCAGAGACCCGGCCAGGGCCTGGAGTGG
ATCGGCTACATCAACCCCAGCAGAGGCTACACCAACTACAACCAGAAGTTCAAGGA
CAAGGCCACCCTGACCACCGACAAGAGCAGCAGCACCGCCTACATGCAGCTGAGCA
GCCTGACCAGCGAGGACAGCGCCGTGTACTACTGCGCCAGATACTACGACGACCAC
TACTGCCTGGACTACTGGGGCCAGGGCACCACCCTGACCGTGAGCAGCGTGGAGGG
CGGCAGCGGCGGCAGCGGCGGCAGCGGCGGCAGCGGCGGCGTGGACGACATCCAG
CTGACCCAGAGCCCCGCCATCATGAGCGCCAGCCCCGGCGAGAAGGTGACCATGAC
CTGCAGAGCCAGCAGCAGCGTGAGCTACATGAACTGGTACCAGCAGAAGAGCGGC
ACCAGCCCCAAGAGATGGATCTACGACACCAGCAAGGTGGCCAGCGGCGTGCCCTA
CAGATTCAGCGGCAGCGGCAGCGGCACCAGCTACAGCCTGACCATCAGCAGCATGG

AGGCCGAGGACGCCGCCACCTACTACTGCCAGCAGTGGAGCAGCAACCCCCTGACC
TTCGGCGCCGGCACCAAGCTGGAGCTGAAGCACCACCACCACCACCACTAG

### EXAMPLES

These examples are provided for illustrative purposes only and not to limit the scope of the claims provided herein.

### Example 1 - Generation of CD3xGD2-HDD Dimert utilizing exemplary CD3xGD2-HDD TransJoins

### Maps of exemplary AAV constructs for testing CD3xGD2-HDD expression.

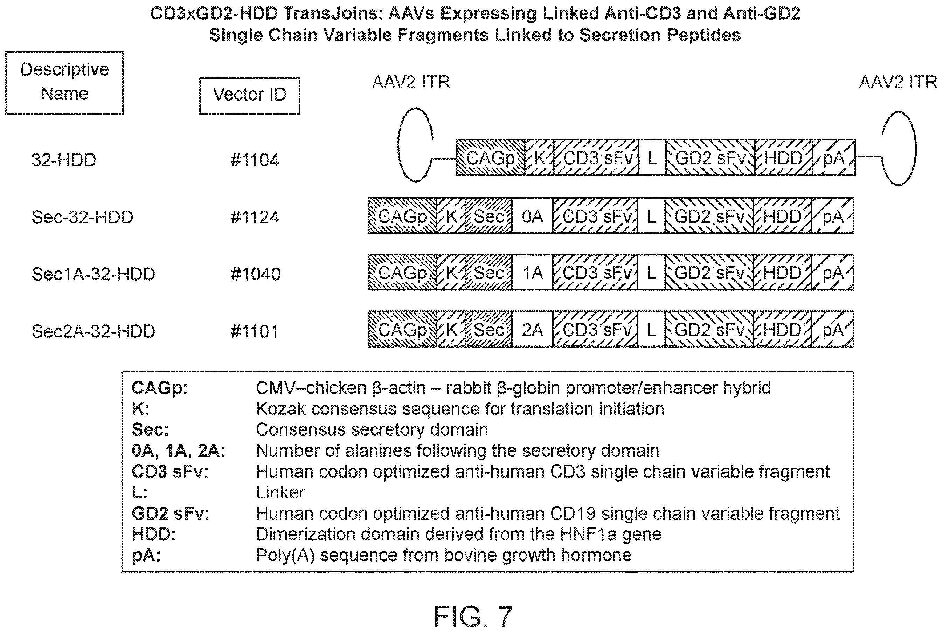

As a proof of principle, a previously described bispecific molecule that targets human CD3 on T cells and the disialoganglioside GD2 on neuroblastoma and other types of cancer cells was expressed. This bispecific protein has the amino acid sequence for the heavy and light variable region against human CD3 (using the format for a single chain variable fragment, scFv) derived from clone OKT3 fused with a short linker (L) to the scFv construct against GD2 derived from clone 5F11, linked as a dimer by the HNF1a dimerization domain (HDD), as reported in Ahmed et al., OncoImmunology, 4:4, e989776, DOI: 10.4161/2162402X.2014.989776. The vectorbuilder.com codon optimization tool (//en.vectorbuilder.com/tool/codon-optimization.html) was used to reverse engineer an optimal human DNA coding sequence for CD3xGD2-HDD. The resulting DNA sequence beginning with an ATG start codon was synthesized and it was cloned downstream of the chicken-actin-b-globin promoter (CAGp) in an adenovirus-associated virus expression cassette with inverted terminal repeats derived from AAV2. Three other versions that contain a consensus secretory signal domain ("secrecon," based on Barash et al., Biochem Biophys Res Commun. 2002; 294: 835-842) downstream of the ATG start site and ending with 0, 1, or 2 alanines were made, based on the finding that alanines enhance secretion depending on the protein (Güler-Gane et al., PLoS ONE 11(5): e0155340. doi:10.1371/journal.pone.0155340). The proteins derived from these constructs were referred to as heterodimeric scFvs or Dimerts. **FIG. 7** illustrates the 4 exemplary CD3xGD2-HDD dimert constructs.

### Assays to determine structure and function of Dimerts.

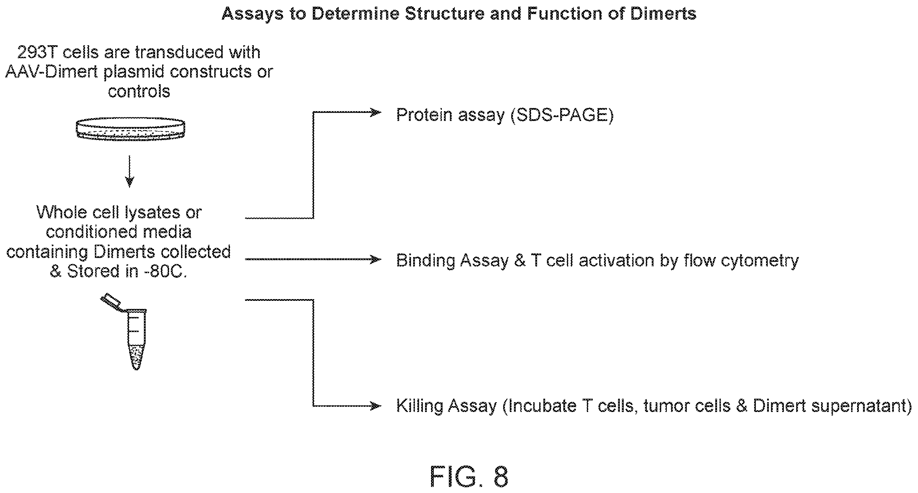

293T cells were transduced with AAV expression vectors (or controls) and collected and stored supernatants. The supernatants were tested for the presence of the correct size protein on electrophoresis (SDS-PAGE), for binding to CD3 via a binding competition assay using flow cytometry, for activating T cells by flow cytometry, and for killing tumor cells when co-incubated with T cells. *See* **FIG. 8** which illustrates a cartoon representation of assays for determining the structure and function of dimerts disclosed herein.

**The three constructs containing a secretion peptide show less CD3xGD2-HDD Dimert retained in cells.**

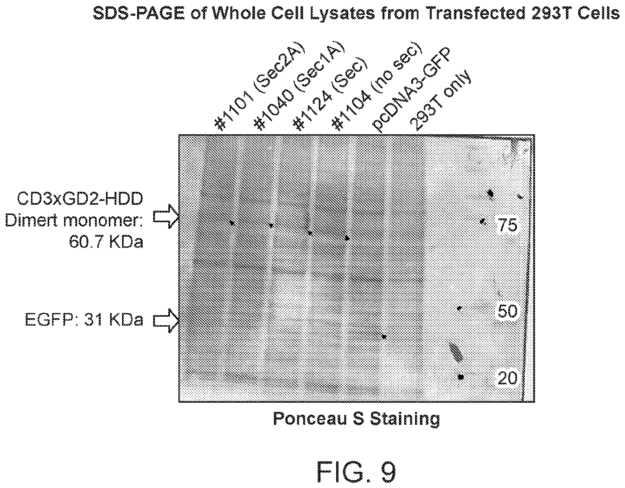

Whole cell lysates of 293T cells transfected with different AAV CD3xGD2-HDD expression plasmids were collected 48hrs post transfection. Polyacrylamide electrophoresis (PAGE) was performed using 50ug of total protein per lane and stained the gel with Ponceau S. The results showed more protein using construct #1104 that lacks a secretion sequence, whereas all 3 constructs with a secretion sequence showed less protein. Controls included cells alone or cells transfected with a control plasmid (pcDNA3-GFP). KDa, kilodalton; Sec, secretory domain; GFP, green fluorescent protein; MW, molecular weight. *See* **FIG. 9****.**

**Only supernatants from cells transfected with AAV CD3xGD2-HDD constructs comprising a secretion sequence bind and activate T cells.**

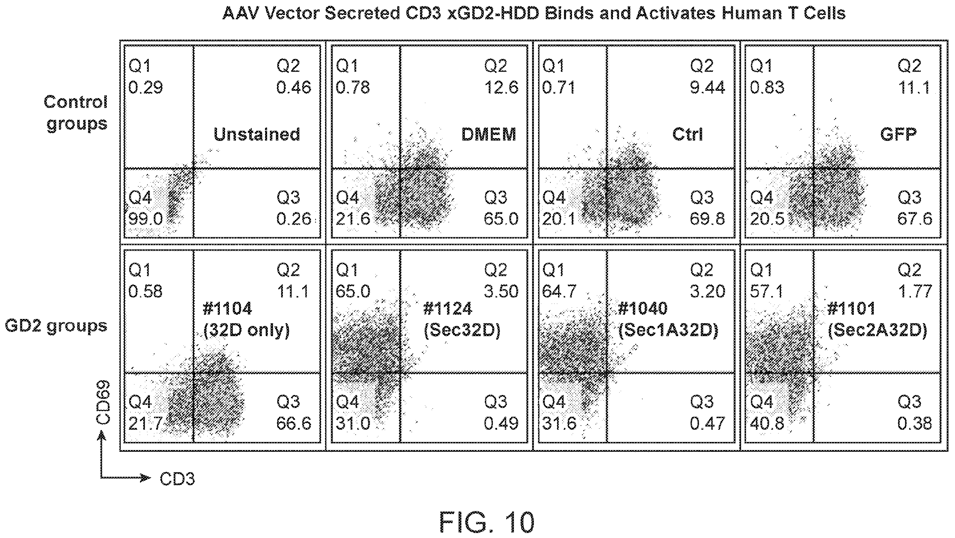

Supernatants were tested for binding to and activating human T cells. Binding was determined by competition with a fluorescently-labeled anti-CD3 antibody pre-bound to T (Jurkat) cells. Stained cells showed 77.6%, 79.24% and 78.7% (Q2+Q3) CD3 positive in control groups (DMEM, Ctrl, GFP), respectively, and 77.7% from AAV vector #1104 that lacks a secretion peptide. In contrast, binding of the fluorescently labeled anti-CD3 antibody was reduced to 2.15%, 3.67%, and 3.99% (Q2+Q3) by supernatants from cells transfected with each of the vectors containing a secretion peptide. Furthermore, cells were co-stained with anti-CD69 as a marker of T cell activation. Control groups and AAV vector #1104 all showed less than 13.4% CD69 positive (Q1+Q2), whereas the three different secretion containing AAV vectors showed 58.87-68.5% CD69 positive. *See* **FIG. 10****.**

Method details: Jurkat 2e5 cells/well in 400ul of RPMI+10%FBS were seeded in 24-well plates, to each well was then added 100 ul supernatants from 293T transfected cells (20% of total culture). After 48hrs incubation, Jurkat cells were spun down, washed 1X with PBS, then stained with PE-Anti-hCD69 (1:100) and PerCP-anti-hCD3e (1:300 #OKT3) for 30mins on ice. After being washed with FACS buffer, each sample was fixed in 1%PFA and analyzed by flow cytometry. DMEM is media alone added to Jurkat, Ctrl is supernatant added from untransfected 293T cells, and GFP is supernatant added from 293T cells transfected with an AAV plasmid expressing GFP.

**Only supernatants from cells transfected with AAV vectors containing a secretion peptide activate human T cells.**

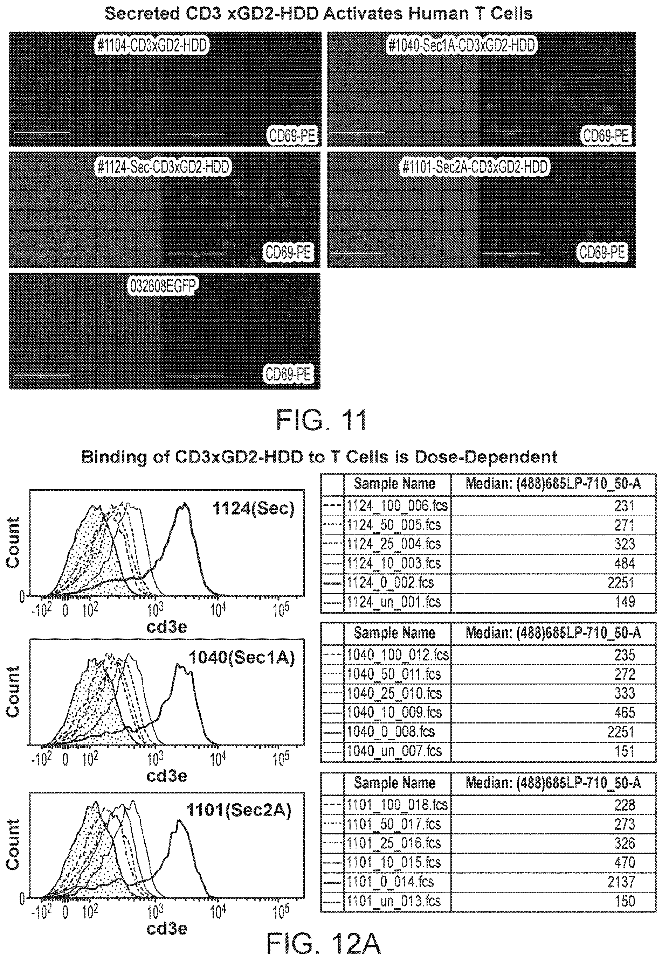

Human T (Jurkat) cells were incubated with supernatants from 293T cells transfected with various AAV vectors, stained for CD69 with a PE-labeled antibody, and examined under fluorescence microscopy. *See* **FIG. 11****,** in which left panels are phase contrast, and right panels are fluorescence. Neither the EGFP vector control nor the vector lacking a secretion peptide #1104 showed positive staining, whereas the other three vectors showed high levels of staining (red). Method details: Jurkat 2e5 cells/well in 400ul of RPMI+10%FBS were seeded in 24-well plates, to each well was then added 100 ul supernatants from 293T transfected cells (20% of total culture). After 48hrs incubation, Jurkat cells were spun down, washed 1X with PBS, then stained with phycoerythrin (PE)-Anti-hCD69 (1:100) for 30mins on ice. After being washed with FACS buffer, each sample was fixed in 1%PFA for 5 mins. Scale bar: 100um.

**Binding of CD3xGD2-HDD to T cells is dose-dependent.**

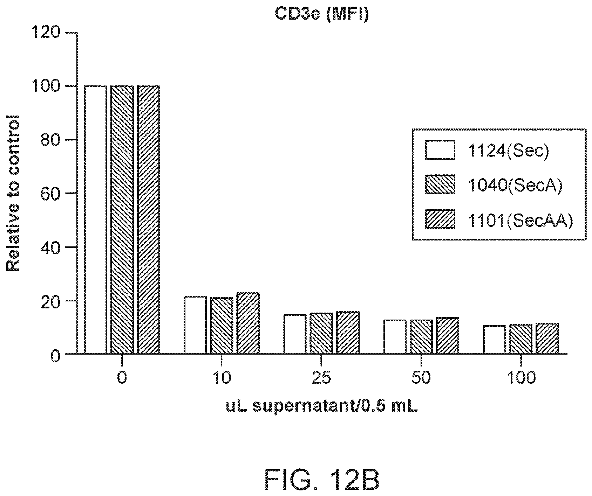

Various amounts of supernatant from 293T cells transfected with the different secretion peptide containing AAV plasmids were incubated and tested for competition with Peridinin Chlorophyll Protein Complex (PerCP)-conjugated anti-hCD3 on Jurkat T cells. *See* **FIG. 12A****,** in which left panels show FACS plots, and right panels show median staining level numbers. Gray shading in left panels is unstained, and dark gray line in each panel indicates maximum stained cells without added supernatants. **FIG. 12B** shows bar graph of median staining levels normalized to unstained controls.

Method details: Jurkat 2e5 cells/well in 400ul of RPMI+10%FBS were seeded in 24-well plates, then to each well was added 10ul, 25ul, 50ul or 100ul of supernatant from 293T transfected cells (2~20% of total culture). After 24hrs incubation, Jurkat cells were spun down, washed 1X with PBS, then stained with PerCP-anti-hCD3e (1:300 #OKT3) for 30mins on ice. After wash with FACS buffer, each samples were fixed in 1%PFA for 5 mins before being analyzed by flow cytometry. Sec = secretory peptide, A = alanine.

**Binding assay for the anti-GD2 arm of a CD3xGD2-HDD Dimert and confirmation that GD2 and CD3 binding both reside on a single molecule.**

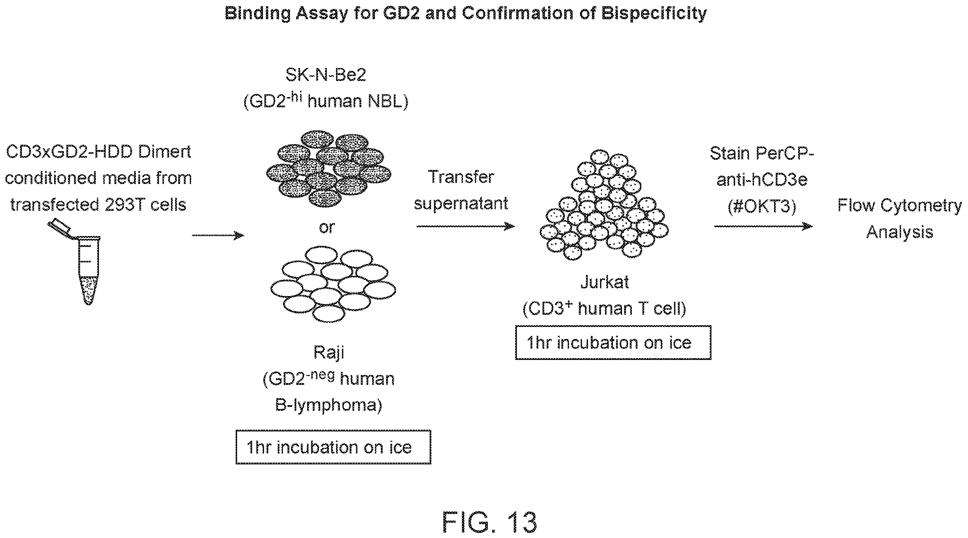

The binding of CD3xGD2-HDD to GD2 was measured indirectly by first incubating supernatants from 293T transfected cells with either GD2-positive or GD2-negative cells, then repeating the CD3 T cell binding competition assay. Proteins binding GD2 should be absorbed by the GD2-positive cells but not by the GD2-negative cells, resulting in loss of competition for T cell binding. This assay was designed as such to confirm that the GD2 binding is co-linked to CD3 binding, in other words that a single molecule is bispecific. *See* **FIG. 13** for a cartoon representation of the binding assay.

**CD3xGD2-HDD binds both CD3 and GD2.**

As illustrated in **FIG. 13****,** supernatants derived from 293T cells transfected with #1101 CD3xGD2-HDD AAV vector were collected and pre-incubated with GD2-positive SK-N-Be(2) or GD2-negative Raji cells. After a spin and wash, the binding competition assay was performed for CD3 T (Jurkat) cells.

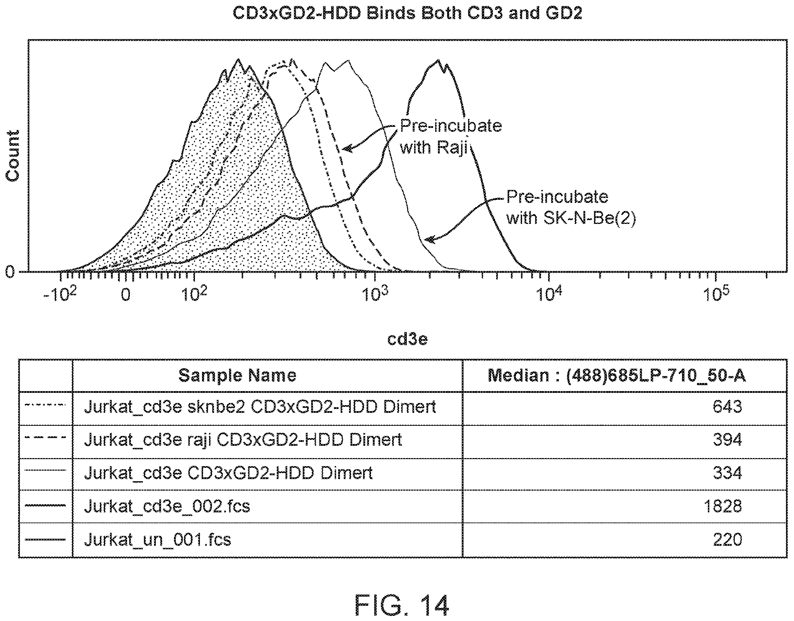

**FIG. 14** illustrates an exemplary CD3xGD2-HDD Dimert which binds to both CD3 and GD2. Gray shading in the top panel with black outline is unstained Jurkat T cells, dark gray line is Jurkat T cells fully stained for CD3 without added supernatant. Sample Jurkat_cd3e showed fully competed CD3 binding using supernatant without pre-incubation, nearly overlapping with supernatant pre-incubated with GD2-negative Raji cells. In contrast, Sample Jurkat_cd3e sknbe2 showed a loss of competition when supernatant was pre-incubated with GD2-positive SK-N-Be(2) neuroblastoma cells, confirming that the same molecules binds both CD3 and GD2.

**Assay to determine if CD3xGD2-HDD induces GD2+ target cell killing by T cells.**

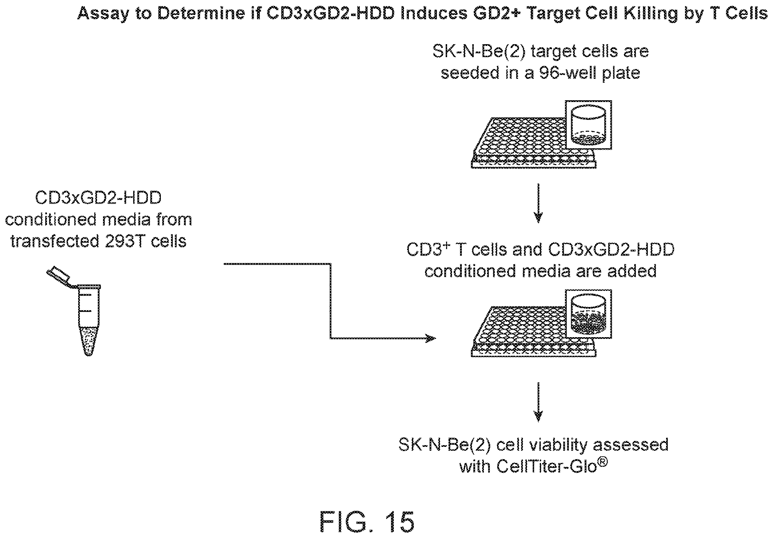

**FIG. 15** illustrates an assay flow-chart to determine if CD3xGD2-HDD induces GD2+ target cell killing by T cells. As shown in FIG. 15, GD2+ neuroblastoma (SK-N-Be(2)) cells are seeded into wells followed by primary human T cells (purchased from StemExpress) and supernatant from AAV vector-transfected 293T cells. Cell viability is assessed with CellTiter-Glo from Promega (Madison, WI).

**Secreted CD3xGD2-HDD induces T cell killing of neuroblastoma cells.**

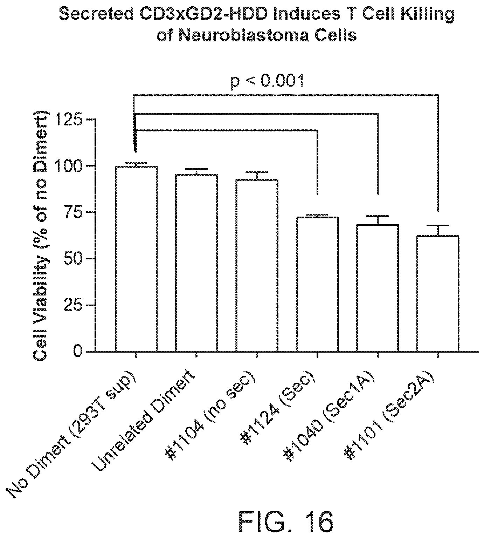

Using the assay illustrated in **FIG. 15****,** cytotoxicity of human T cells plus supernatants (from 293T cells transfected with various AAV vector plasmids) were tested on GD2+ SK-N-Be(2) neuroblastoma cells. A T cell to target cell ratio of 10:1 was used. Cells were assayed for viability after 48 hours in co-culture. Compared with supernatants from untransfected cells ("no Dimert"), there was no appreciable cytotoxicity using supernatants from cells transfected with a construct expression an unrelated dimert (CD19xCD3) or the vector lacking a secretion peptide (#1104) **(****FIG. 16****).** In contrast, supernatants from cells transfected with each of the vectors containing a secretion peptide induced a statistically significant cytotoxicity (p<0.001), killing 25-30% of the cells **(****FIG. 16****).**

**T cell mediated cytotoxicity by CD3xGD2-HDD Dimert is related to GD2 expression.**

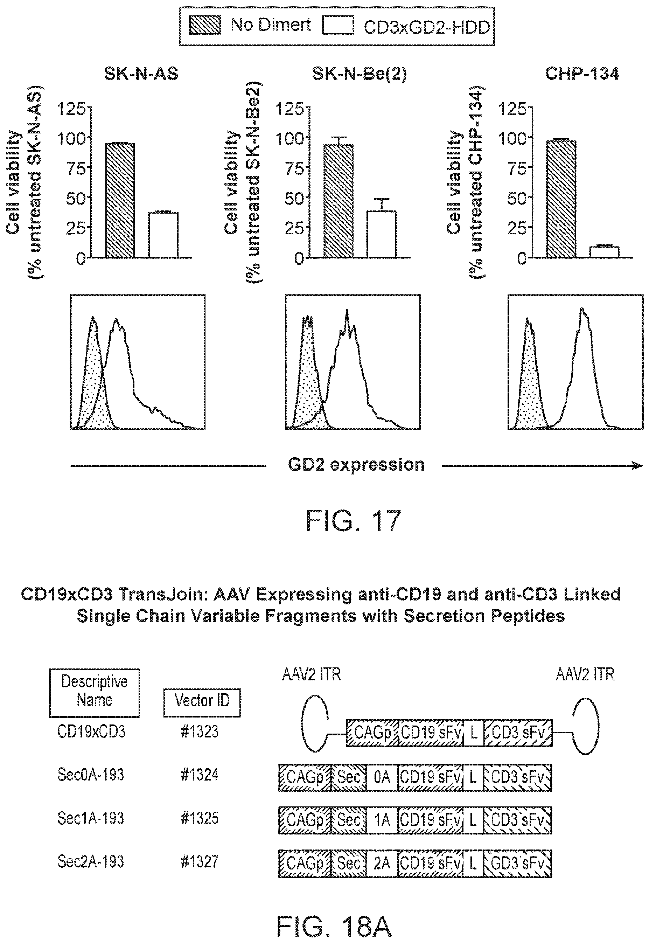

A panel of neuroblastoma cell lines were tested for their sensitivity to killing by human T cells combined with supernatants from AAV vector #1101 (upper graphs of **FIG. 17****)** and measured their GD2 expression by flow cytometry (lower graphs of **FIG. 17****,** shaded curves are isotype controls). CHP-134 cells showed the most cytotoxicity and the highest GD2 expression.

### Example 2 - Generation of CD19xCD3 Dimert utilizing exemplary CD19xCD3 TransJoins

### Maps of exemplary AAV constructs for testing CD19xCD3 Dimert expression.

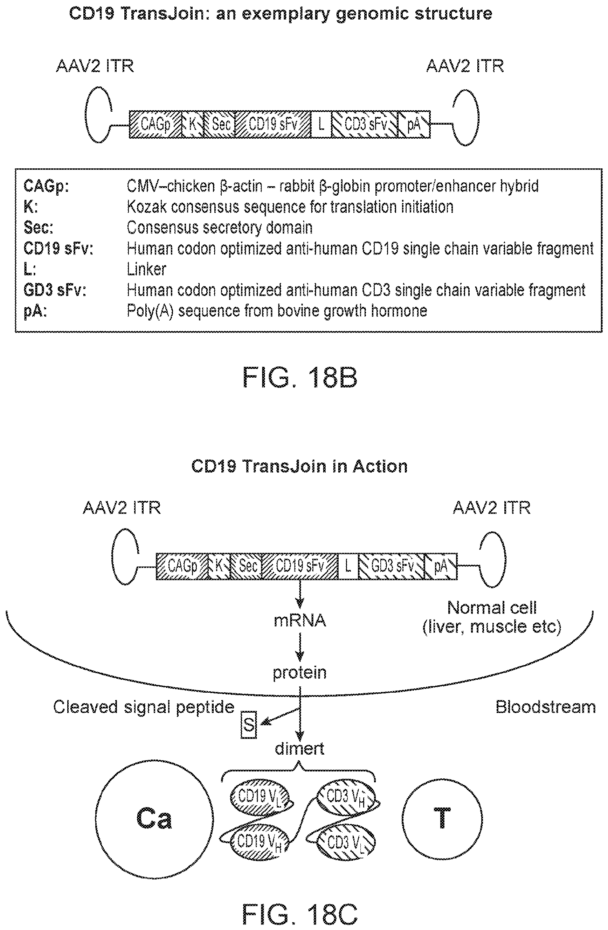

The FDA-approved protein therapeutic known as blinatumomab, a so-called bispecific T cell engager (BiTE), was generated to target human CD19 on B cells / B cell malignancies and human CD3 on T cells. The publicly available amino acid sequence for blinatumomab (//www.drugbank.ca/drugs/DB09052) was used and the vectorbuilder.com codon optimization tool (//en.vectorbuilder.com/tool/codon-optimization.html) was further used to reverse engineer an optimal human DNA coding sequence for CD19xCD3. The resulting DNA sequence beginning with an ATG start codon was synthesized and cloned downstream of the chicken-actin-b-globin promoter (CAGp) in an adenovirus-associated virus expression cassette with inverted terminal repeats derived from AAV2. Three other versions that contain a consensus secretory signal domain ("secrecon," based on Barash et al., Biochem Biophys Res Commun. 2002; 294: 835-842) downstream of the ATG start site and ending with 0, 1, or 2 alanines were made, based on the finding that alanines enhance secretion depending on the protein (Güler-Gane et al., PLoS ONE 11(5): e0155340. doi:10.1371/journal.pone.0155340). The proteins derived from these constructs were referred to as heterodimeric scFvs or Dimerts. **FIG. 18A** and **FIG. 18B** illustrates 5 exemplary CD19xCD3 constructs.

**FIG. 18C** illustrates a cartoon representation of a CD19 dimert interacting with a cancer cell and a T cell. The CD19 dimert is produced by a cell comprising a CD19 TransJoin. When administered to a subject, e.g., intravenously, the AAV TransJoin (e.g., the AAV CD19 TransJoin as illustrated in this figure) enters normal cells such as liver or muscle and expresses the polypeptide it encodes. The secretion signal peptide is cleaved during secretion, leaving the active dimert that binds a cancer cell (Ca) on one end and a T immune cell on the other end.

**Only supernatants from cells transfected with AAV CD19xCD2 constructs containing a secretion sequence bind and activate T cells.**

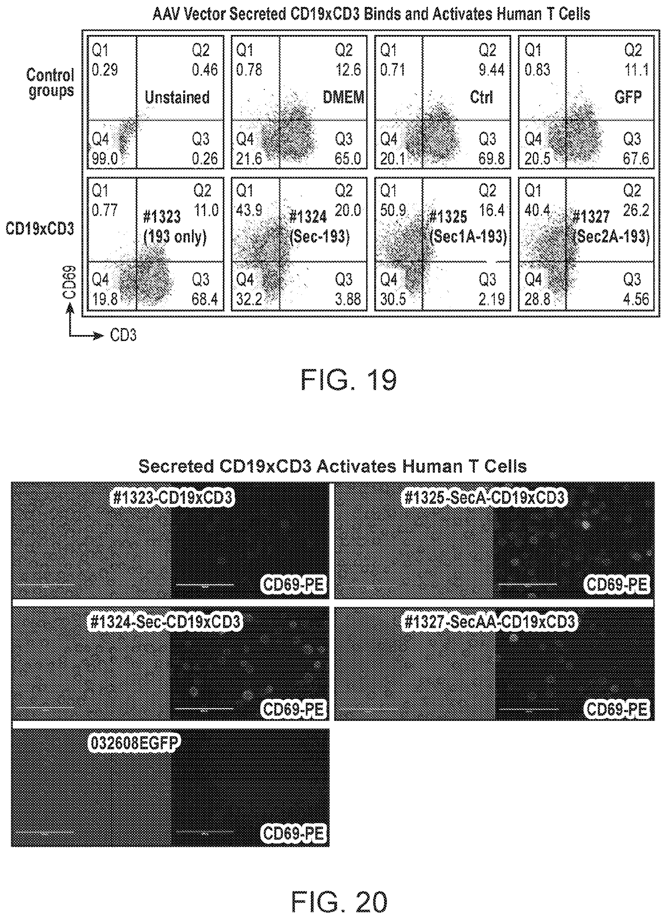

Supernatants were tested for binding to and activating human T cells. Binding was determined by competition with a fluorescently-labeled anti-CD3 antibody pre-bound to human T (Jurkat) cells. Stained cells showed 77.6%, 79.24%, and 78.7% (Q2+Q3) CD3 positive in control groups (DMEM, Ctrl, GFP), respectively, and 79.4% from vector #1323 that lacks a secretion peptide. In contrast, binding of the fluorescently labeled anti-CD3 antibody was reduced to 23.88%, 18.59%, and 30.76% (Q2+Q3) by supernatants from cells transfected with each of the vectors containing a secretion peptide. Furthermore, cells were co-stained with anti-CD69 as a marker of T cell activation. Control groups and #1323 all showed less than 13.38% CD69 positive (Q1+Q2), whereas the three different secretion containing vectors showed 63.9%, 67.3%, and 66.6% CD69 positive **(****FIG. 19****).**

Method details: Jurkat 2e5 cells/well in 400ul of RPMI+10%FBS were seeded in 24-well plates, to each well was then added 100 ul supernatants from 293T transfected cells (20% of total culture). After 48hrs incubation, Jurkat cells were spun down, washed 1X with PBS, then stained with PE-Anti-hCD69 (1:100) and PerCP-anti-hCD3e (1:300 #OKT3) for 30mins on ice. After being washed with FACS buffer, each sample was fixed in 1%PFA and analyzed by flow cytometry. DMEM is media alone added to Jurkat, Ctrl is supernatant added from untransfected 293T cells, and GFP is supernatant added from 293T cells transfected with an AAV plasmid expressing GFP.

**Only supernatants from cells transfected with AAV vectors containing a secretion peptide activate human T cells.**

Human T (Jurkat) cells were incubated with supernatants from 293T cells transfected with various AAV vectors, stained for CD69 with a PE-labeled antibody, and examined under fluorescence microscopy. See **FIG. 20****,** left panels are phase contrast, and right panels are fluorescence. Neither the EGFP vector control nor the AAV vector lacking a secretion peptide #1323 showed positive staining, whereas the other three vectors showed high levels of staining (red).

Method details: Jurkat 2e5 cells/well in 400ul of RPMI+10%FBS were seeded in 24-well plates, to each well was then added 100 ul supernatants from 293T transfected cells (20% of total culture). After 48hrs incubation, Jurkat cells were spun down, washed 1X with PBS, then stained with phycoerythrin (PE)-Anti-hCD69 (1:100) for 30mins on ice. After being washed with FACS buffer, each sample was fixed in 1%PFA for 5 mins. Scale bar: 100um.

**AAV secreted CD19xCD3 specifically binds CD19 but not CD45.**

A binding competition assay was used to determine if supernatants from supernatant from AAV vector-transfected 293T cells interferes with staining of Epstein Barr Virus (EBV)-transformed human B cells by two different antibodies, one that stains the B cell marker CD19 and another that stains the pan-leukocyte marker CD45.

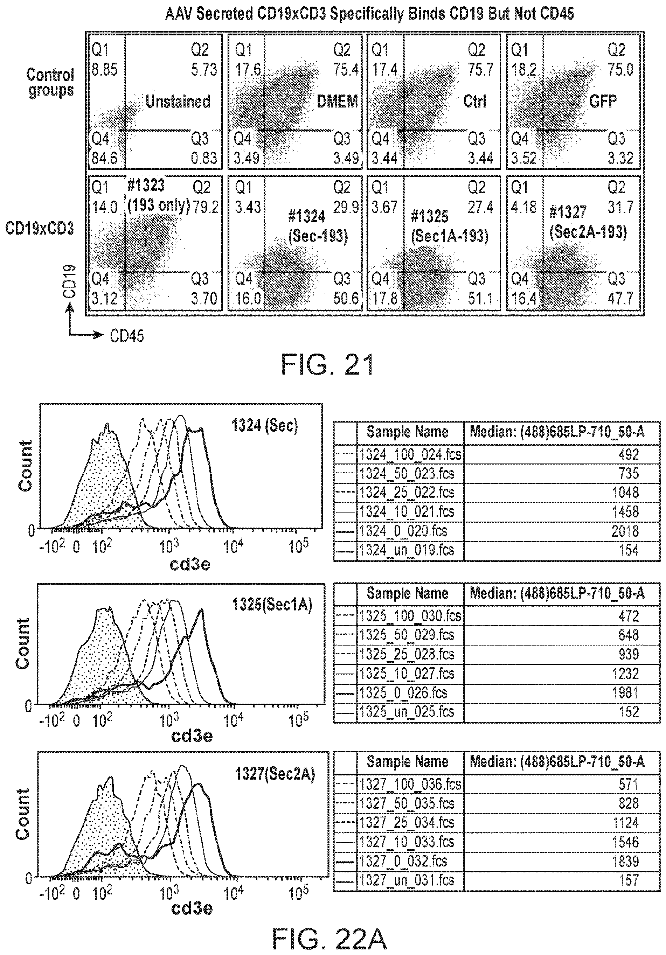

*See* **FIG. 21****,** in which cells in 3 control groups (DMEM, Ctrl, GFP) showed 93%, 93.1%, and 93.2% positive staining for CD19 (Q1+Q2), respectively, and supernatant from cells transfected with AAV vector #1323 that lacks a secretion peptide did not compete that staining, showing 93.2% positive for CD19. In contrast, each of the AAV vectors containing a secretion peptide competed out the CD19 signal down to 33.33%, 31.07%, and 35.88%. Note that the cutoff was set such that unstained cells showed 14.58% positive for CD19, suggesting that supernatant from cells transfected with those three AAV vectors competed down to 2-fold above background. In contrast, none of the vectors competed for CD45 staining, which was 78.89%, 79.14%, and 78.32% positive (Q2+Q3) in the 3 controls, 82.9% positive in the vector lacking a secretion peptide, and 80.5%, 78.5% and 79.4% positive using supernatants from cells transfected with the other three vectors.

Method details: B cells transformed by EBV called NB122R (Gene Ther. 2013 Jul;20(7):761-9. doi: 10.1038/gt.2012.93) at 2e5 cells/well in 400ul of RPMI+10%FBS were seeded in 24-well plate, then to each well was added 100ul of supernatant from 293T transfected cells (2~20% of total culture). After 24hrs incubation, cells were spun down, washed 1X with PBS, then stained with PEcy7-anti-hCD45(1:100) & APC-anti-hCD19(1:300) for 30mins on ice. After washing with FACS buffer, each sample was fixed in 1%PFA for 5mins before flow cytometry analysis. DMEM is media alone added to Jurkat, Ctrl is supernatant added from untransfected 293T cells, and GFP is supernatant added from 293T cells transfected with an AAV plasmid expressing GFP.

**Binding of CD19xCD3 to T cells is dose-dependent and the vector containing a single alanine downstream of the secretion peptide consensus sequence is superior compared to the other tested vectors.**

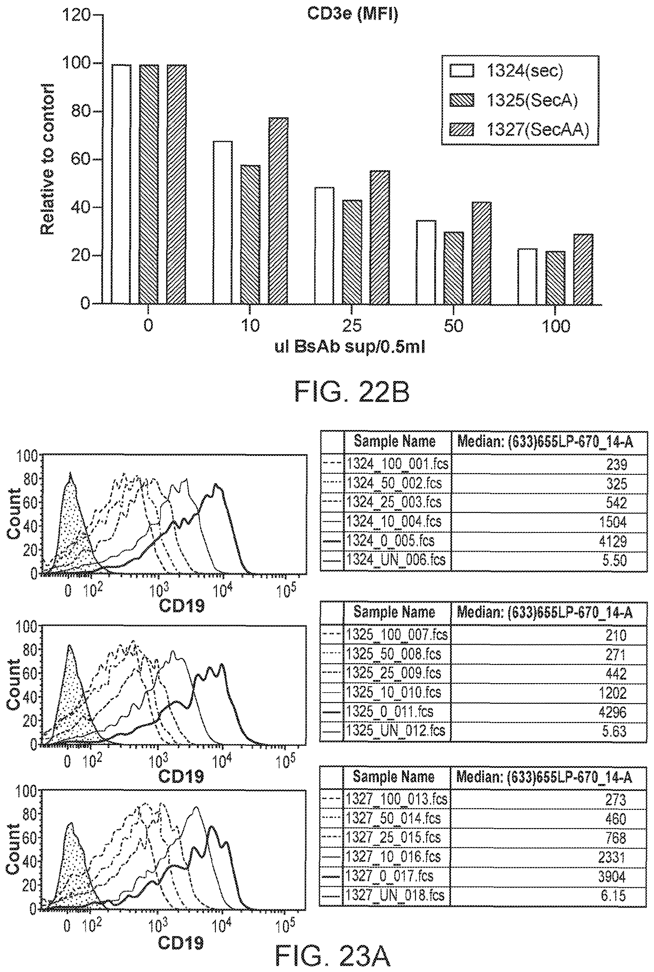

Various amounts of supernatant from 293T cells transfected with the different secretion peptide containing AAV plasmids were incubated and tested for competition with Peridinin Chlorophyll Protein Complex (PerCP)-conjugated anti-hCD3 on Jurkat T cells. *See* **FIG. 22A****,** in which left panels show FACS plots, and right panels show median staining level numbers. Gray shading in each of the left panels was unstained cells, and dark gray line in each the left panels was maximum stained cells without added supernatants. **FIG. 22B** shows bar graph of median staining levels normalized to unstained controls. The vector with a single alanine downstream of the secretion peptide consensus sequence, #1325, showed the most competition compared to other tested vectors and was selected for further experiments.

Method details: Jurkat 2e5 cells/well in 400ul of RPMI+10%FBS were seeded in 24-well plates, then to each well was added 10ul, 25ul, 50ul or 100ul of supernatant from 293T transfected cells (2~20% of total culture). After 24hrs incubation, Jurkat cells were spun down, washed 1X with PBS, then stained with PerCP-anti-hCD3e (1:300 #OKT3) for 30 mins on ice. After wash with FACS buffer, each samples were fixed in 1%PFA for 5 mins before being analyzed by flow cytometry. MFI = Mean fluorescence intensity, Sec = secretory peptide, A = alanine.

**Binding of CD19xCD3 to B cells is dose-dependent and the vector containing a single alanine downstream of the secretion peptide consensus sequence is superior compared to the other tested vectors.**

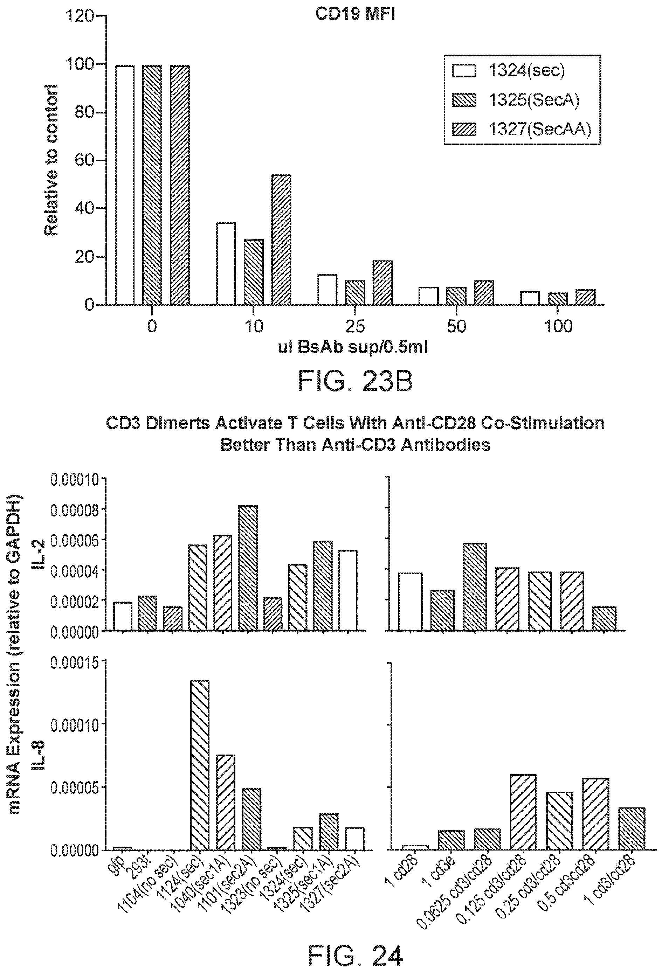

Various amounts of supernatant from 293T cells transfected with the different secretion peptide containing AAV plasmids were incubated and tested for competition with Allophycocyanin (APC)-conjugated anti-hCD19 on human B cells. *See* **FIG. 23A****,** in which left panels show FACS plots, and right panels show median staining level numbers. Gray shading in each of the left panels was unstained, and dark gray line in each of the left panels was maximum stained cells without added supernatants. **FIG. 23B** shows bar graph of median staining levels normalized to unstained controls. The vector with a single alanine at the end of the secretion peptide, #1325, showed the most competition consistent with the results for T cell binding shown in **FIG. 22A** and **FIG. 22B** and was selected for further experiments.

Method details: NB122R human B cells at 2e5 cells/well in 400ul of RPMI+10%FBS were seeded in 24-well plates, then to each well was added 10ul, 25ul, 50ul or 100ul of supernatant from 293T transfected cells (2~20% of total culture). After 24hrs incubation, cells were spun down, washed 1X with PBS, then stained with APC-anti-hCD19(1:300) for 30mins on ice. After wash with FACS buffer, each samples were fixed in 1%PFA for 5 mins before being analyzed by flow cytometry. MFI = Mean fluorescence intensity, Sec = secretory peptide, A = alanine.

**CD3 Dimerts activate T cells with anti-CD28 co-stimulation better than anti-CD3 antibodies.**

Human T (Jurkat) cells were co-incubated with anti-CD28 antibodies and supernatants derived from AAV vector transfected 293T cells (left panels of **FIG. 24****)** or increasing concentrations of anti-CD3 antibodies (right panels of **FIG. 24****).** Cellular mRNA was harvested and quantitative reverse transcriptase polymerase chain reaction (RT-PCR) was performed for IL-2 (top panels of **FIG. 24****)** and IL-8 (bottom panels of **FIG. 24****)** mRNA and calculated expression relative to the housekeeping mRNA GAPDH. Only supernatants from cells transfected with AAV vector constructs containing a secretory domain showed stimulation of either gene above controls (gfp, 293t) and above the vector constructs lacking the secretory domain (#1104 for CD3xGD2-HDD, #1323 for CD19xCD3).

**293T cells yield highest transduction efficiency for AAV8.**

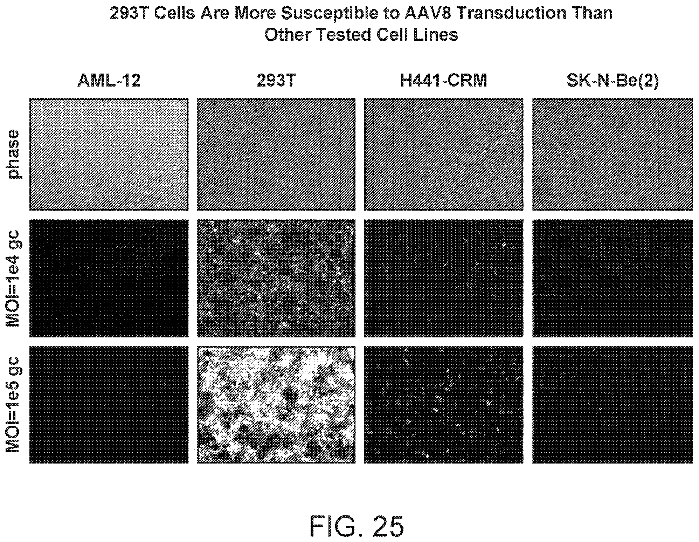

Four different cell lines were infected with 2 different concentrations of AAV8 expressing green fluorescent protein (GFP) at a multiplicity of infection (MOI)=1e4 or 1e5 genome copies (gc) with 2% fetal bovine serum containing culture media. GFP signal was evaluated at 48hrs post virus infection by fluorescence microscopy. *See* **FIG. 25****.** AML-12: mouse normal liver cells; 293T: human embryonic kidney cells transformed with SV40-T antigen; H441-CRM: human lung cancer cells with KRAS mutation; SK-N-Be(2): NMYC amplified human neuroblastoma cells.

**Dimert concentration in supernatants of AAV8-infected cells is depending on AAV dose and transduction efficiency.**

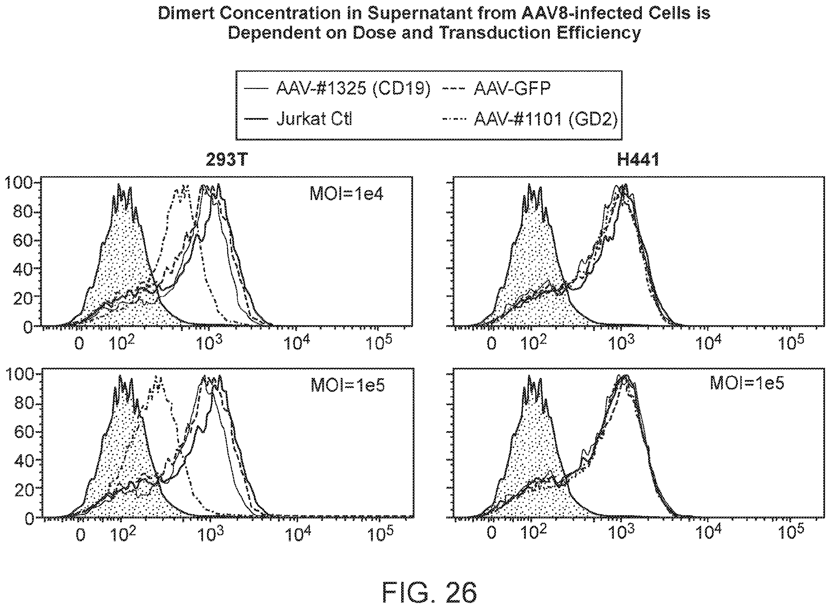

A T (Jurkat) binding assay was performed on supernatants from 293T and H441 cells transfected with TransJoin vectors or the AAV-GFP control. *See* **FIG. 26****,** in which gray shading in the panels of is unstained control cells, and dark gray lines in each panels is T cells fully stained with anti-CD3 antibody. Although the CD19xCD3 Dimert appeared to be much more effective than the CD3xGD2-HDD Dimert at competing for binding, the effect was dose-dependent (curves shifted farther to the left with the higher MOI) and not seen in the cell line with poor AAV8 transduction efficiency (H441 cells). MOI, multiplicity of infection.

**A single intravenous injection of CD19xCD3 TransJoin selectively eliminates B cells in humanized mice.**

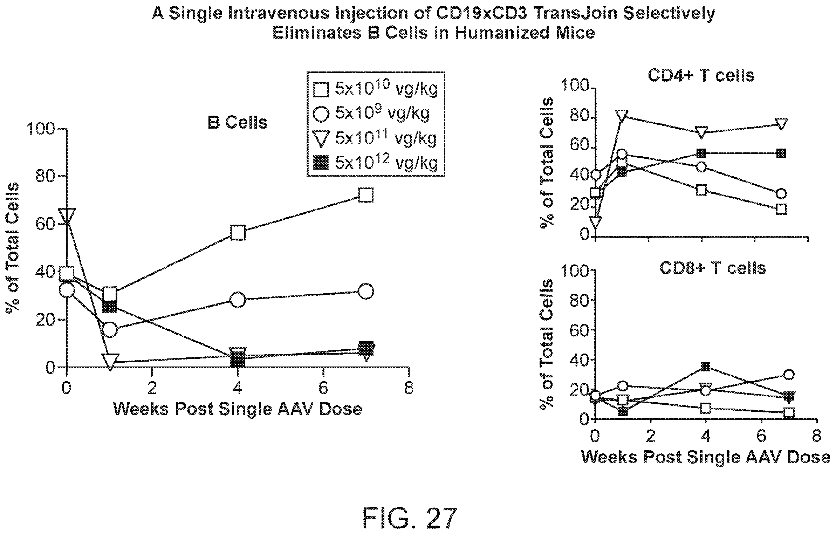

Immunodeficient mice (NSG-SGM3, Jackson Labs) were purchased that had been irradiated and intravenously injected with human CD34+ hematopoietic stem cells. By 12 weeks, the mice showed engraftment of human blood cells by flow cytometry of peripheral blood, including T and B lymphocytes. A single injection of a CD19xCD3 TransJoin (AAV8-Sec1A-CAG-193, vector #1325 packaged into AAV8 capsids) was administered at various doses in mice and analyzed blood by flow cytometry for human lymphocytes collected at different timepoints as shown in **FIG. 27****.** Although there was no effect at low doses (5e9 and 5e10 vector genomes (vg) per kilogram (kg) body weight), higher doses (5e11 and 5e12 vg/kg) were sufficient to eliminate circulating B cells without affecting CD4+ or CD8+ T cells.

**A single intravenous injection of CD19xCD3 TransJoin results in prolonged B cell depletion in humanized mice.**

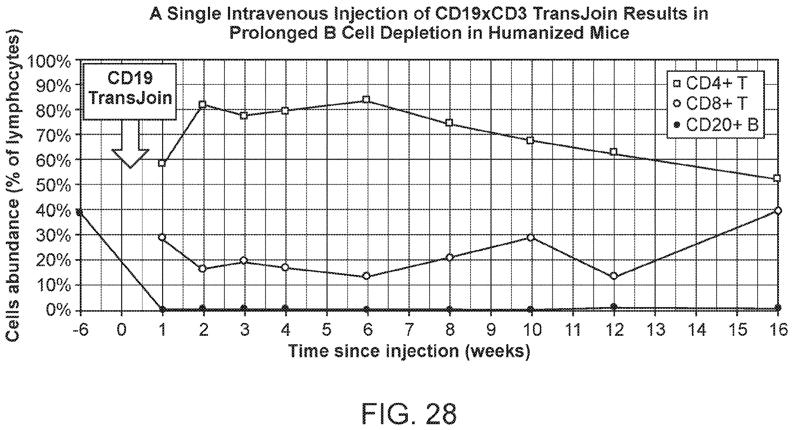

Humanized mice were treated with a single dose of CD19xCD3 AAV8 TransJoin and lymphocyte subsets in blood were subsequently measured. As shown in **FIG. 28** for a single mouse, prolonged and selective B cell depletion was observed in all mice analyzed for as a long as these mice remained alive.

**A single intravenous injection of CD19xCD3 TransJoin eliminates CD19+ lymphoma in humanized mice.**

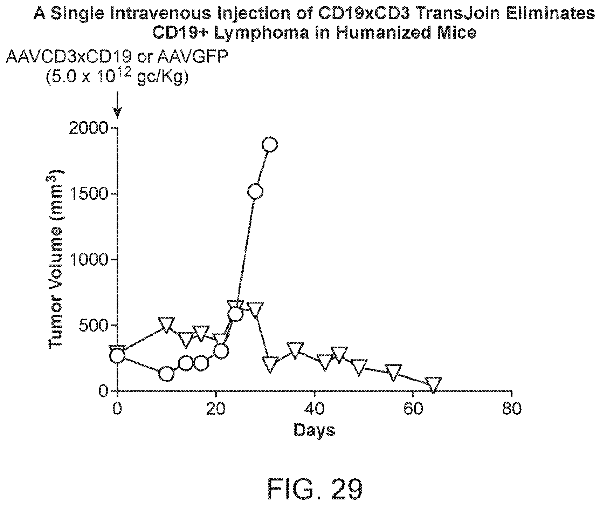

Human CD19+ Raji cells (derived from a patient with Burkitt lymphoma) were implanted into the flanks of two humanized mice and waited until they reached over 250 mm³ in size. The tail vein of one animal was then injected with a control AAV virus, AAVGFP, and the tail vein of a second animal was injected with the CD19xCD3 AAV8 TransJoin. The tumor in the control mouse eventually grew rapidly and the animal needed to be euthanized. The tumor in the TransJoin treated mouse grew slowly then eventually shrunk nearly completely. *See* **FIG. 29****.** The animal needed to be sacrificed for symptoms of graft-vs-host disease, which is a known outcome in humanized mice, but the experiment demonstrates proof-of-principle that a single TransJoin injection can treat cancer.

**Example 3 - Use of OncoSkip and TransSkip to simultaneously target oncogene expression and activate therapeutic transgene expression**

**Overview of OncoSkip and TransSkip described herein.**

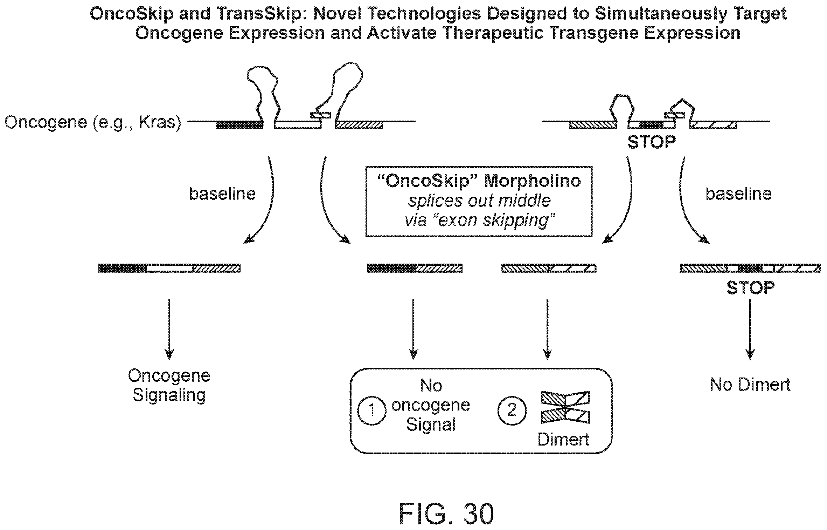

OncoSkip is an antisense morpholino that induces exon skipping of an oncogene. In some embodiments, OncoSkip is designed as an antisense morpholino that induces exon-skipping of a critical exon in an oncogene (left side of **FIG. 30****).** For proof-of-principle, KRAS (exons 1, 2, 3 represented by black-gold-gray on left side of **FIG. 30****)** was used and morpholinos (shown in **FIG. 30** as small light gray bar on top of oncogenes) designed to skip KRAS exon 2 was tested, which contains the ATG start site, so that normal expression of the oncogene would be decreased. A derivative of the same exon to be skipped is then created, but mutated to contain multiple stop codons in each reading frame, along with flanking intron sequences. The new intron-exon(STOP)-intron is then inserted into the transgene coding sequence at sites that recreate donor and acceptor splice sites, so that it is spliced into the transgene mRNA and interrupts the normal coding sequence. In the presence of the antisense morpholino (small light gray bar on top of oncogenes), the newly inserted exon is skipped, re-connecting the coding sequence to generate a functional product. The class of AAV vectors containing genes interrupted by the intron-exon-intron sequence are termed TransSkip viruses, and the class of morpholinos designed to downregulate an oncogene is termed OncoSkip.

**KRAS OncoSkip antisense morpholinos induce exon skipping of endogenous KRAS in lung cancer cells.**

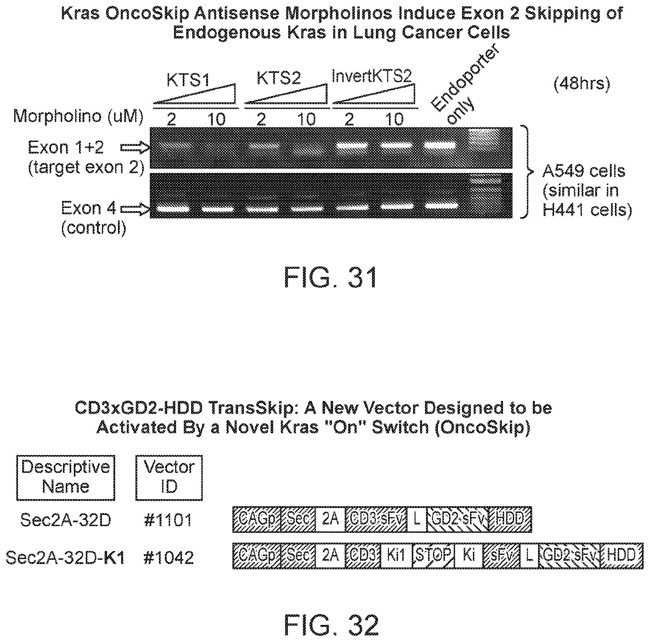

Lung cancer cell lines A549 and H441 (not shown) were incubated with the KTS1 and KTS2 OncoSkip antisense morpholinos, as well as the inverted control morpholino for KTS2. KTS 1 and KTS2 both bind at the 3-prime end of the KRAS exon 2 exon-intron junction and were designed to induce skipping of exon 2 as it contains the ATG start site. Endogenous KRAS mRNA were then analyzed by reverse transcriptase RT-PCR for the presence of exon 2 by PCR with primers present in exon 1 (forward) and exon 2 (reverse). Primers in exon 4 were used as a control for total KRAS mRNA. A dose-dependent reduction in transcripts containing exon 2 ("Exon 1+2") was observed with both OncoSkip morpholinos. *See* **FIG. 31****.**

**AAV vector maps of CD3xGD2-HDD TransSkip showing reverse engineered introns flanking an exon inserted into the CD3xGD2-HDD Dimert coding sequence.**

The 3-prime sequence of the human intron upstream of KRAS exon 2 (Ki1), a derivative of KRAS exon 2 containing mutated to multiple stop codons in all three reading frames (STOP), and the 5-prime sequence of the human intron downstream of KRAS exon 2 (Ki) were synthesized and the cassette was cloned into the gene sequence encoding the GD2 Dimert at a specific sequence that recapitulates consensus splice donor and acceptor sites. *See* **FIG. 32****.** When the new STOP exon is spliced into the transcript, a functional Dimert is not produced. When cells are exposed to an antisense morpholino that binds the 3-prime exon-intron junction, the STOP exon is skipped and a full length Dimert mRNA is expressed. The KRAS sequences were chosen so that an antisense morpholino designed to cause skipping of exon 2 would simultaneously alter mRNA splicing of the TransSkip transgene, resulting in expression of the full length Dimert in transduced cells, and decrease or eliminate native KRAS expression in cancer cells, as exon 2 contains the KRAS ATG start codon.

**Exemplary strategy for testing activity of OncoSkip and TransSkip.**

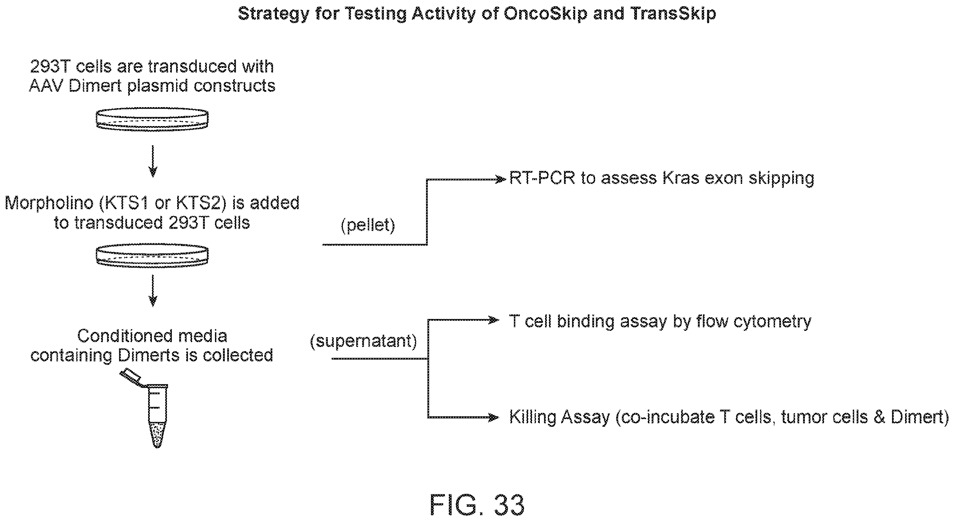

As shown in **FIG. 33****,** cell pellets and supernatants were collected from 293T cells transduced with AAV Dimert vectors. mRNA was isolated from the cell pellet and subject to reverse transcriptase RT-PCR to determine the extent to which the artificial exon in the transgene was spliced into the mRNA. The supernatant for Dimert expression was analyze via the T cell binding and killing assays.

**Antisense morpholinos induce exon skipping of the CD3xGD2-HDD TransSkip transgene.**

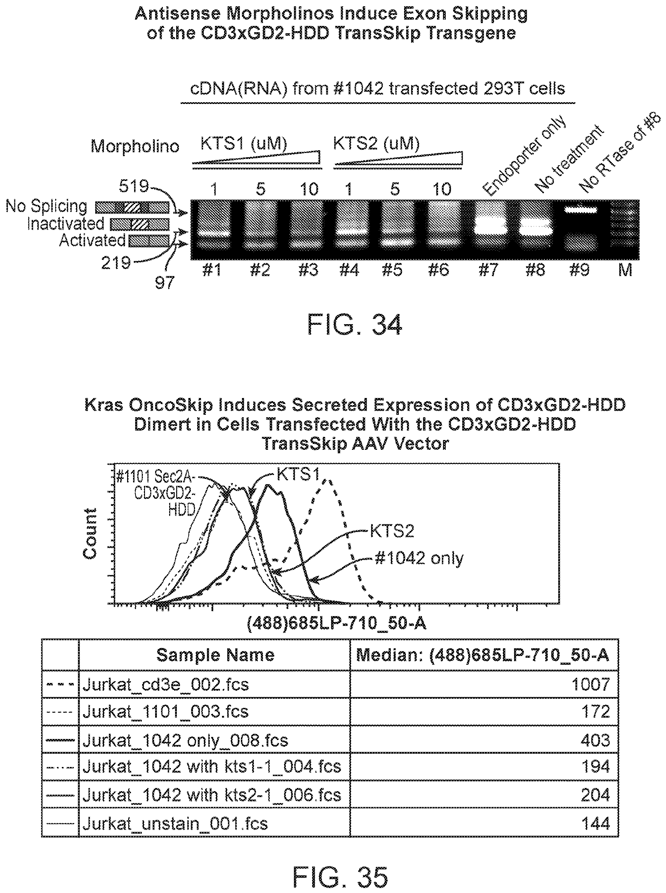

293T cells were transfected with the CD3xGD2-HDD Dimert plasmid #1042. Cells were harvested 48hrs post-transfection for total RNA isolation. About 1ug of RNA was used for RT-PCR. *See* **FIG. 34****,** in which lane #9 of shows the full length transgene RNA between primers without splicing, using the DNA plasmid as a template. Lanes 7 and 8 and control lanes are without antisense morpholino, and show the majority of transcripts include the internal exon (stripped rectangle). There is some transcripts with the exon excluded (smallest band), suggesting this construct has some "leak" of activated transcripts. Inclusion of either morpholino (KTS1, KTS2) reduce the proportion of the inactivated, exon-included transcript (219 bp) in a dose-dependent fashion and increase the activated transcript (97 bp) that excludes the exon.

**KRAS OncoSkip induces secreted expression of CD3xGD2-HDD Dimert in cells transfected with the CD3xGD2-HDD TransSkip AAV vector.**

Supernatants were collected from transfected 293T cells and tested for T cell binding (interference with fluorescence-labeled anti-CD3 antibody). As shown in **FIG. 35****,** gray line in top panel is unstained T (Jurkat) cells, dark gray line in top panel is fully stained T cells with anti-CD3 antibody. As a positive control, supernatant from cells transfected with the constitutively expressed CD3xGD2-HDD TransJoin #1011 nearly fully competed out the anti-CD3 staining. Supernatant from cells transfected with CD3xGD2-HDD TransSkip #1042 showed intermediate competition, consistent with some "leakage" of exon-skipped Dimert mRNA, and was further induced with either OncoSkip morpholino (KTS1, KTS2).

**Exon skipping of CD3xGD2-HDD TransSkip by KRAS OncoSkip is an on-target effect.**

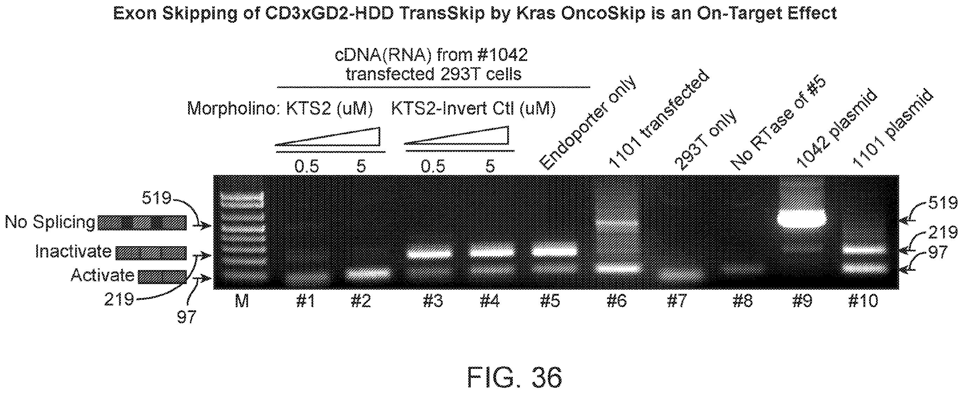

Exon skipping induced by the targeted anti-sense morpholino KTS2 was compared with a morpholino containing the same bases but reversed in sequence **(****FIG. 36****).** KTS2 induced exon skipping to nearly 100% of transcripts (97 bp) whereas the reversed control morpholino had no effect compared with the Endoporter only (carrier for morpholino) control.

**Induction of CD3xGD2-HDD Dimert expression as determined by T cell binding is an on-target effect.**

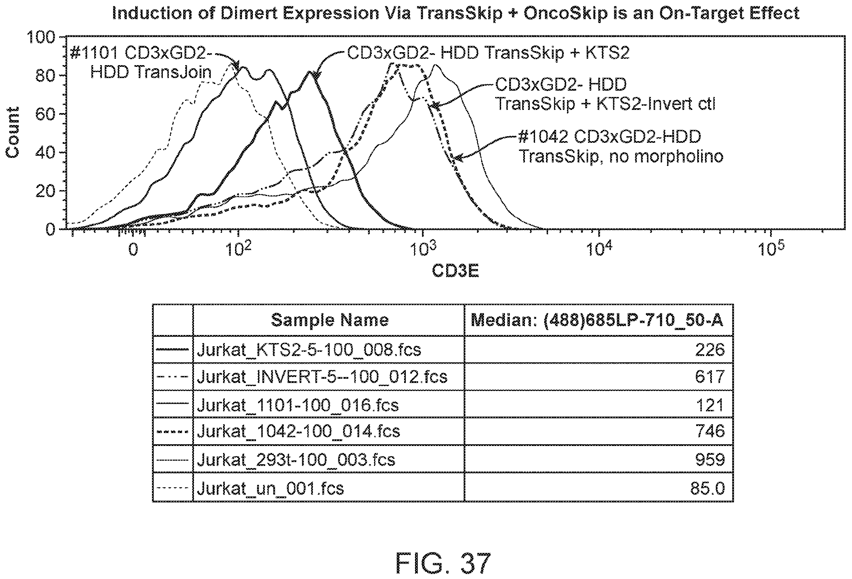

Supernatants from 293T cells transfected with CD3xGD2-HDD TransJoin (positive control) and CD3xGD2-HDD TransSkip incubated without or with different antisense morpholinos were collected and the human T (Jurkat) cell binding assay was performed by flow cytometry (competition for fluorescently labeled anti-CD3 antibody binding). *See* **FIG. 37****,** in which gray line in top panel is unstained T cells and dark gray line in top panel is fully stained T cells. Line indicating #1101 CD3xGD2-HDD TransJoin is nearly completely competed signal from the constitutively expressed GD3 TransJoin. As before, supernatant from CD3xGD2-HDD TransSkip transfected cells showed a slight competition at baseline (#1042 CD3xGD2-HDD TransSkip, no morpholino) that was not altered by a control morpholino (CD3xGD2-HDD TransSkip + "KTS2-Invert ctl") but was induced to compete more signal by the on-target "OncoSkip" morpholino, KTS2 (CD3xGD2-HDD TransSkip + KTS2).

**AAV genome maps of exemplary TransSkip splice variants created and tested to decrease baseline TransSkip "leak" but maintain inducible exon-skipping.**

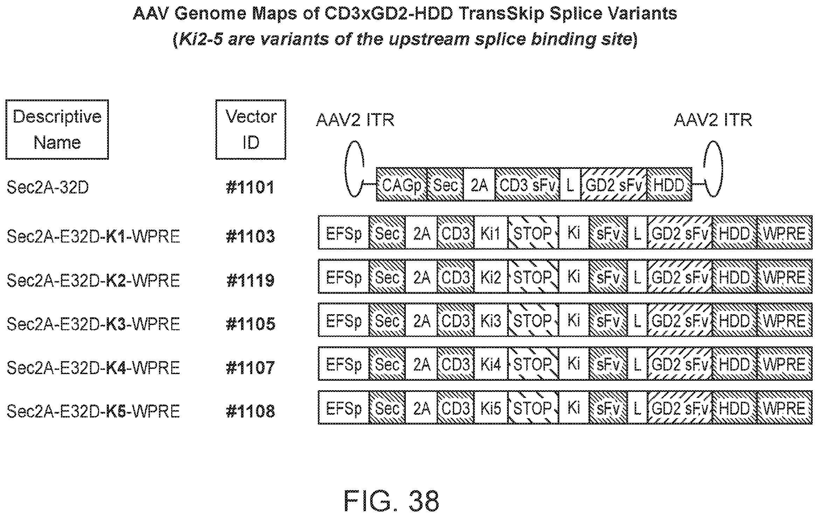

Splicing variants of TransSkip were engineered to reduce the baseline exon skipping that was observed with the CD3xGD2-HDD TransSkip. The Woodchuck Hepatitis Virus Posttranscriptional Regulatory Element (WPRE) downstream of the coding sequence was used to increase transgene expression. Because of AAV packaging size restrictions, inclusion of WPRE sequences necessitated using a shorter promoter, as such a new series of vectors were created with promoter derived from the short form of the human eukaryotic translation elongation factor 1 α1 promoter (EFSp). The variants were created by altering specific base pairs in the U2 accessory factor (U2AF) binding site located in the polypyrimidine track at the 3-prime end of the first intron (Ki1-5). *See* **FIG. 38****.**

**CD3xGD2-HDD TransSkip splice variant K3 eliminates baseline yet maintains inducible exon skipping.**

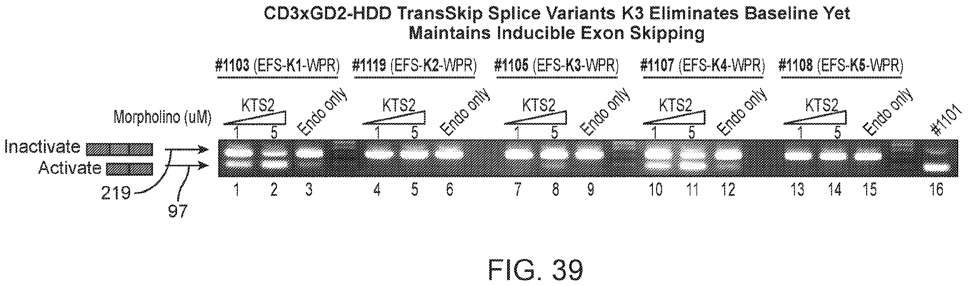

293T cells were transfected with the new panel of 5 different CD3xGD2-HDD TransSkip constructs without and with the KTS2 antisense "OncoSkip" morpholino and compared them to the "Endo only" control that has the morpholino carrier but no morpholino. The mRNA transcripts were analyzed by reverse transcriptase RT-PCR and found different phenotypes. As shown in **FIG. 39****,** variant K4 showed a similar level of (or even slightly more) baseline exon skipping to K1 and was inducible to skip even more than K1. Variants K2, K3, and K5 showed effectively no baseline skipping. Of those 3, variant K3 was able to be induced the most to skip the exon.

**CD3xGD2-HDD TransSkip Variant K3 Shows No Baseline Dimert Production But Is Most Inducible By KRAS OncoSkip.**

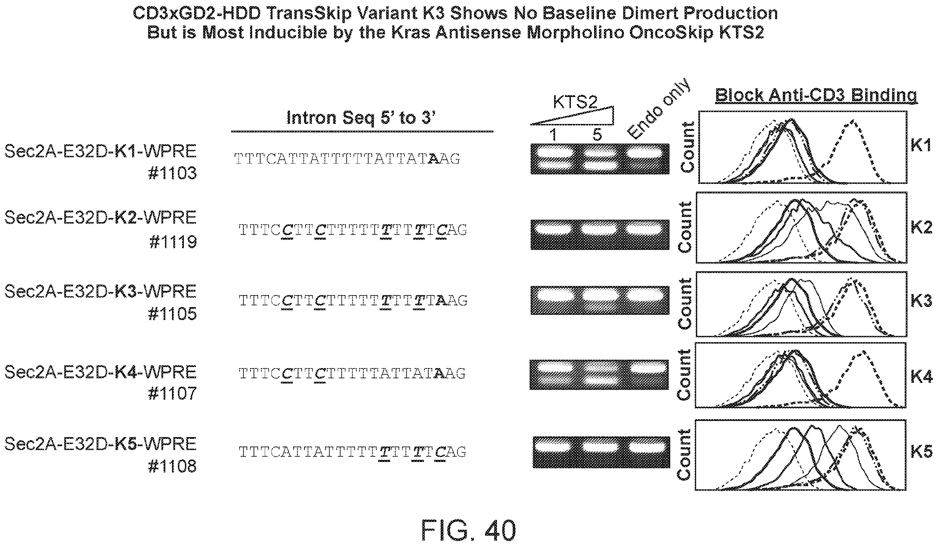

The panel of CD3xGD2-HDD TransJoin splice variants were tested for production of secreted CD3xGD2-HDD Dimert expression without or with the KRAS KTS2 OncoSkip antisense morpholino using the T (Jurkat) cell binding assay (far right). As shown in **FIG. 40****,** gray line in each of the right panels is unstained T cells, dark gray is fully stained T cells, blue is the constitutively expressed CD3xGD2-HDD TransJoin, green is the respective CD3xGD2-HDD TransSkip without OncoSkip ("Endo only"). The sequence shows the polypyrimidine U2AF binding site with basepair variants engineered in gray (underlined and italic) relative to the wild type KRAS intron sequence (K1). The top band in RT-PCR gel is the transcript containing the exon, the bottom band is the transcript with the skipped exon. Variants K1 and K4 show some detectable baseline exon skipping by RT-PCR and marked baseline Dimert expression (green, far right), whereas variants K2, K3 and K5 all show no baseline exon skipping by RT-PCR and no Dimert expression by binding competition (green, far right). All three of those variants show a small amount of inducible exon skipping by RT-PCR expression in the presence of the KRAS antisense morpholino OncoSkip KTS2 and various degrees of dose-dependent Dimert expression in the presence of the KRAS OncoSkip (light and dark purple representing lower and higher concentrations, respectively). The K3 variant appeared to be most inducible, so K3 was selected as a lead construct for further study.

**OncoSkip-mediated induction of Dimert expression from CD3xGD2-HDD TransSkip variants K3 and K5 is an on-target effect.**

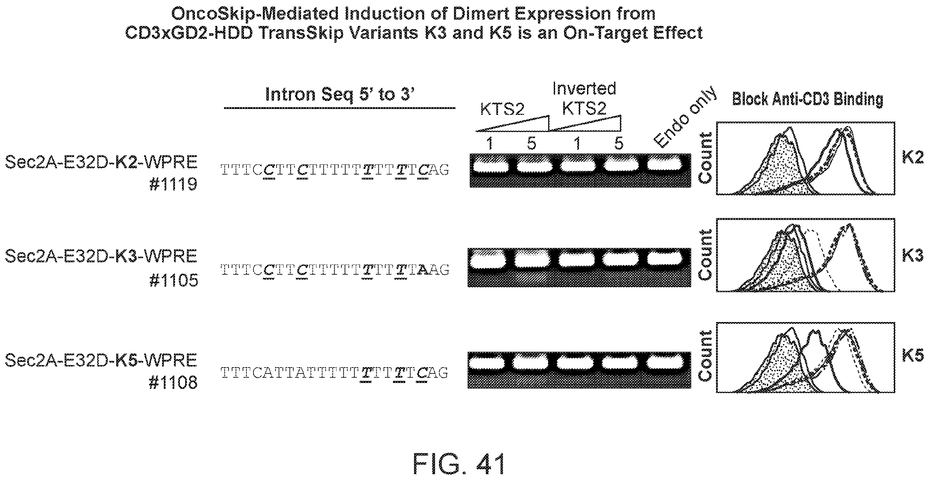

The RT-PCR and T (Jurkat) binding assays were repeated with CD3xGD2-HDD TransSkip variants K2, K3, and K5 and included the "inverted KTS2" antisense morpholino as a control. *See* **FIG. 41****.** The inverted KTS2 contains the same nucleotide bases as KTS2 except in the opposite order. The inverted KTS2 failed to induce exon skipping (lower band on the gel) in both K3 and K5 whereas the correct KTS2 sequence induced exon skipping in both K3 and K5 (neither induced it in the K2 variant). In the T cell binding assay for Dimert expression (far right panels), gray is unstained T cells, dark gray is fully stained T cells, blue is constitutively expressed CD3xGD2-HDD TransJoin, purple is with the KRAS OncoSkip KTS2 (light is lower dose, dark is higher dose), and yellow is inverted KTS2 control. The Dimert protein assay is consistent with the RT-PCR; none of the morpholinos induced Dimert expression from the K2 variant, and only the KTS2 OncoSkip but not the inverted control induced Dimert expression from the K3 and K5 variants.

**Secreted Dimert induced by OncoSkip from AAV CD3xGD2-HDD TransSkip vectors is functional in mediating T cell killing of neuroblastoma cells.**

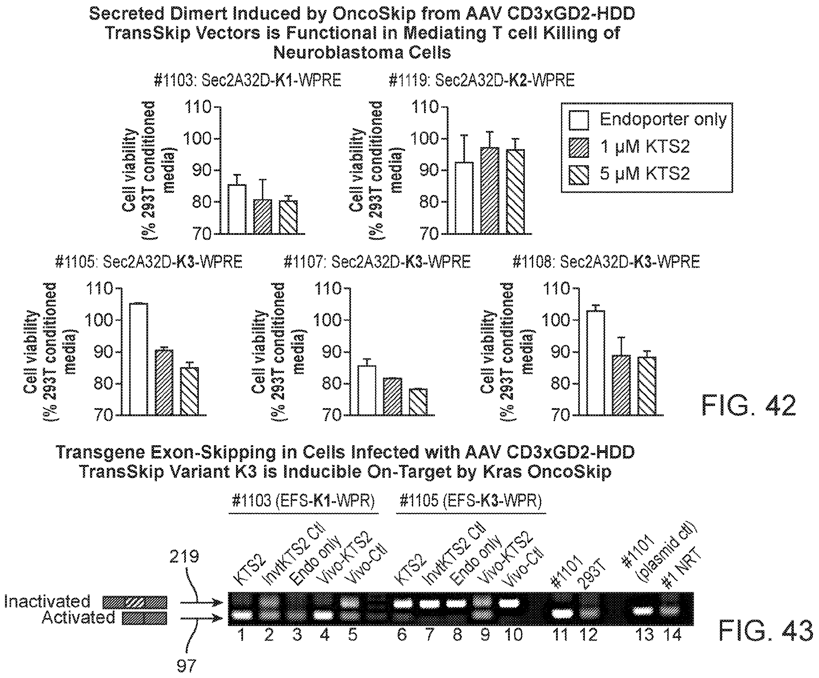

Cytotoxicity of human T cells plus supernatants from 293T cells transfected with various AAV CD3xGD2-HDD TransSkip variants without and with the KRAS OncoSkip KTS2 on GDS2+ SK-N-Be(2) neuroblastoma cells was tested **(****FIG. 42****).** A T cell to target cell ratio of 10:1 was used. Cells were assayed for viability after 48 hours in co-culture. Results were normalized to supernatants from untransfected cells. CD3xGD2-HDD TransSkip variants K1 and K4 showed cytotoxicity in the absence of the KRAS OncoSkip KTS2, consistent with baseline exon skipping, and further inducible cytotoxicity using K4. CD3xGD2-HDD TransSkip variant K2 showed no baseline nor inducible cytotoxicity consistent with its known efficient splicing and exon inclusion. In contrast, CD3xGD2-HDD TransSkip variants K3 and K5 showed no baseline but did show inducible cytotoxicity, being dose-dependent with the K3 variant.

**Transgene exon-skipping in cells infected with AAV CD3xGD2-HDD TransSkip variant K3 is inducible on-target by KRAS OncoSkip.**

Exon-skipping in the context of AAV virus infection was examined to determine whether it mirrored that seen with transfection of AAV plasmids in other studies. CD3xGD2-HDD TransSkip variants K1 and K3 were packaged into AAV8 and analyzed exon inclusion ("inactivated") and exclusion ("activated) by RT-PCR 48 hrs after AAV infection of 293T cells. Cells were incubated without ("Endo only") or with antisense morpholinos KRAS OncoSkip KTS2 or the inverted control ("InvtKTS2 Ctl"). As shown in **FIG. 43** and with the vector plasmids, the AAV GD2 K1 variant showed exon skipping at baseline that was further induced by the KRAS OncoSkip KTS2 but not by the control. In contrast, exon skipping at baseline was not observed with the K3 variant but was observed to be induced with the OncoSkip KTS2 (both alone or complexed with a carrier, "Vivo-KTS2"). In contrast, it was not induced by the control morpholino.

**AAV genome maps of CD19xCD3 TransSkip splice variants.**

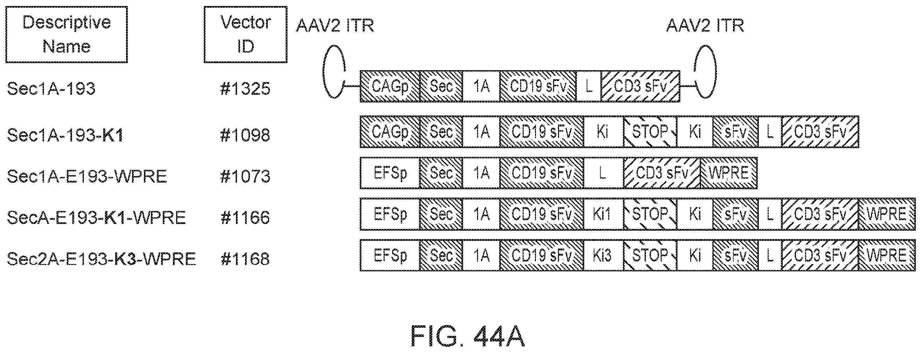

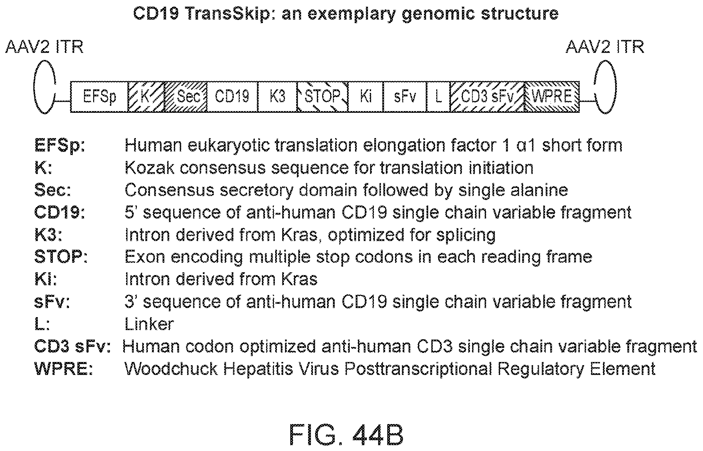

Based on the findings from the CD3xGD2-HDD TransSkip studies in which variant K3 showed lower baseline compared to K1 but still inducible Dimert expression, K1 and K3 intron variants inserted into the CD19xCD3 Dimert transgene were created. As with the GD2 series, the WPRE downstream of the coding sequence was used to increase transgene expression, requiring the use of a shorter promoter, EFSp. Ki, sequence derived from part of the KRAS intron. STOP, exon 2 from KRAS mutated to include stop codons in all three reading frames. *See* **FIG. 44A** **and** **FIG. 44B** **for illustrative examples of CD19xCD3 TransSkip constructs.**

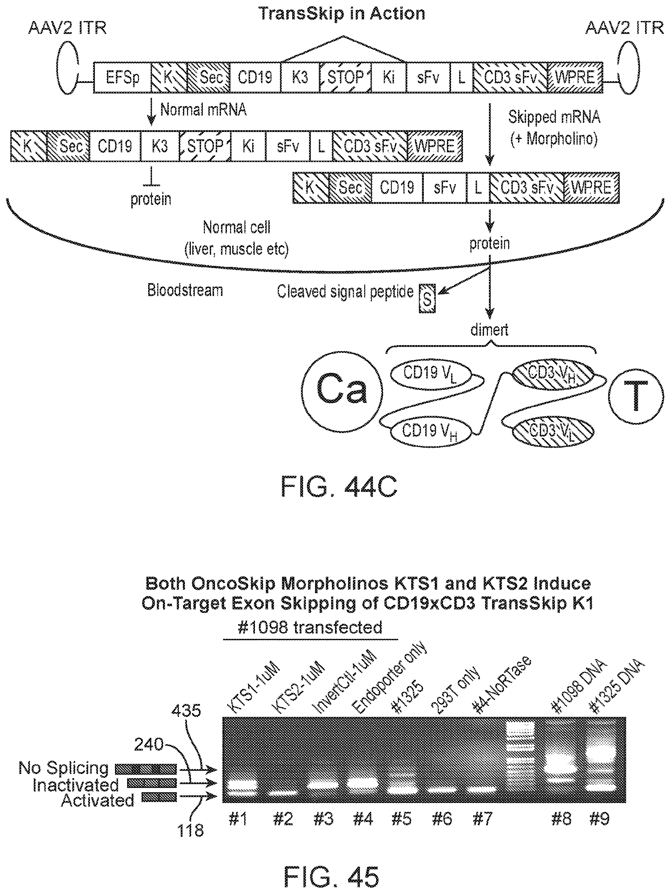

**FIG. 44C** illustrates a cartoon representation of a CD19 dimert interacting with a cancer cell and a T cell. The CD19 dimert is produced by a cell comprising a CD19 TransSkip. When administered to a subject, e.g., intravenously, the AAV TransSkip (e.g., the AAV CD19 TransSkip as illustrated in this figure) enters normal cells such as liver or muscle, but it does NOT express a polypeptide because of the insertion of introns and an exon containing stop codons into the normal coding sequence of the transgene. In the presence of an antisense polynucleotide (e.g., a morpholino antisense oligonucleotide) that induces exon skipping, the mRNA transcript processed by the antisense oligonucleotide contains an intact transgene that is not interrupted by a STOP exon. Under such circumstance, the polypeptide is synthesized and secreted, the secretion signal peptide is cleaved during secretion, leaving the active dimert that binds a cancer cell (Ca) on one end and a T immune cell on the other end.

**Both OncoSkip morpholinos KTS1 and KTS2 induce on-target exon skipping of CD19xCD3 TransSkip K1.**

As shown in **FIG. 45****,** 293T cells were transfected with AAV vectors including CD19xCD3 TransJoin (positive control, #1325) and CD19xCD3 TransSkip K1 (#1098) without (lane #4) or with co-incubation with a control morpholino (lane #3) or two different KRAS OncoSkip morpholinos (lanes 1 and 2). The CD19xCD3 TransSkip K1 shows some baseline exon skipping (presence of lower "activated" band in lanes 3 and 4), consistent with our findings in the parallel constructs for CD3xGD2-HDD TransSkip. These results show that the engineered TransSkip design works similarly with multiple different transgenes.

**KRAS OncoSkip induces secreted expression of CD19xCD3 Dimert in cells transfected with the CD19xCD3 TransSkip K1 AAV vector.**

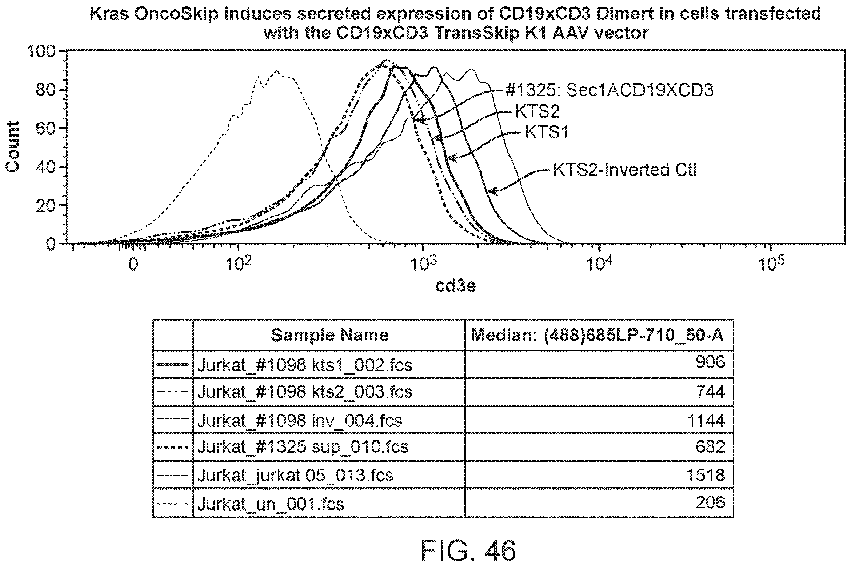

Supernatants were collected from transfected 293T cells and tested for T cell binding (interference with fluorescence-labeled anti-CD3 antibody). As shown in **FIG. 46****,** gray line in the top panel is unstained T (Jurkat) cells, and dark gray line in the top panel is fully stained T cells with anti-CD3 antibody. As a positive control, supernatant from cells transfected with the constitutively expressed CD19xCD3 TransJoin #1325 competed for anti-CD3 staining (#1325). Supernatant from cells transfected with CD19xCD3 TransSkip #1098 in the presence of the morpholino control showed some baseline competition (KTS2-Inverted Ctl), consistent with "leakage" of exon-skipped Dimert mRNA, and was further induced with either OncoSkip morpholino (KTS1; KTS2) to the levels achieved with the constitutive CD19xCD3 TransJoin.

**CD19xCD3 TransSkip splice variant K3 eliminates baseline yet maintains inducible exon skipping.**

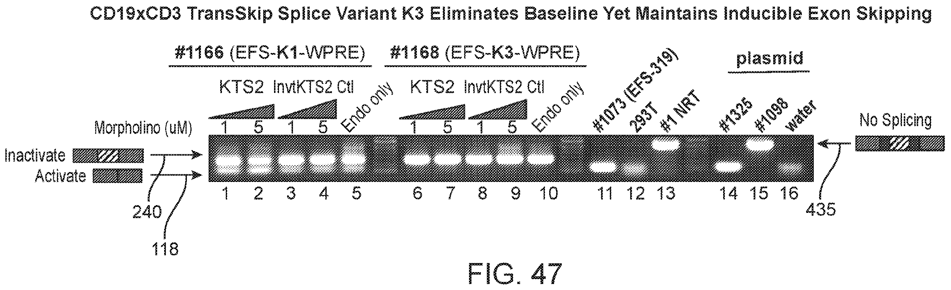

As shown in **FIG. 47****,** 293T cells were transfected with the K1 and K3 CD19xCD3 TransSkip constructs without and with the KTS2 antisense "OncoSkip" morpholino or control morpholino ("InvtKTS2 Ctl") and compared them to the "Endo only" control that has the morpholino carrier but no morpholino. The mRNA transcripts were analyzed by reverse transcriptase RT-PCR. Consisent with the parallel CD3xGD2-HDD TransJoin constructs, variant K1 showed baseline exon skipping. In contrast, variants K3 showed no baseline skipping ("Endo only") or skipping with the morpholino control. Skipping was observed with the KTS2 OncoSkip with the K3 (#1168) variant, which was confirmed by analyzing protein expression via the T cell binding assay.

**Induction of CD19xCD3 Dimert expression from CD19xCD3 TransSkip K3 as determined by T cell binding is an on-target effect.**

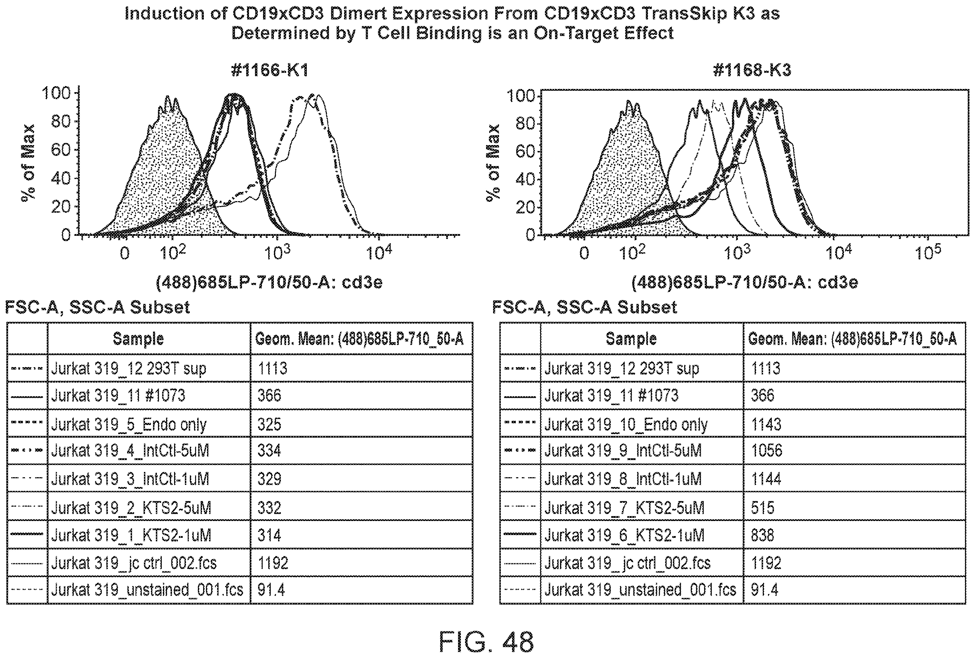

Supernatants were collected from 293T cells transfected with CD3xGD2-HDD TransJoin (positive control) and CD19xCD3 TransSkip incubated without or with different antisense morpholinos and performed the human T (Jurkat) cell binding assay by flow cytometry (competition for fluorescently labeled anti-CD3 antibody binding). As shown in **FIG. 48****,** gray in each of the top panels is unstained T cells, dark gray is fully stained T cells, pink is fully stained T cells in the presence of supernatant from untransfected 293T cells, blue is signal competed by supernatant from cells transfected with the constitutively expressed CD19xCD3 TransJoin #1073. Supernatant from CD19xCD3 TransSkip K1 #11166 all showed high expression equivalent to the positive control CD19xCD3 TransJoin at baseline and with control morpholinos, demonstrating this construct is not suitable for controlling gene expression. In contrast and consistent with the experience with the CD3xGD2-HDD TransSkip, CD19xCD3 TransSkip variant K3 #1168 showed no baseline expression (green) or expression induced by the control morpholino (light and dark orange, "IntCtl"), but a dose-dependent expression with the KRAS OncoSkip morpholino KTS2 (light and dark purple).

**Induction of CD19xCD3 Dimert expression from CD19xCD3 TransSkip K3 is repeatable.**

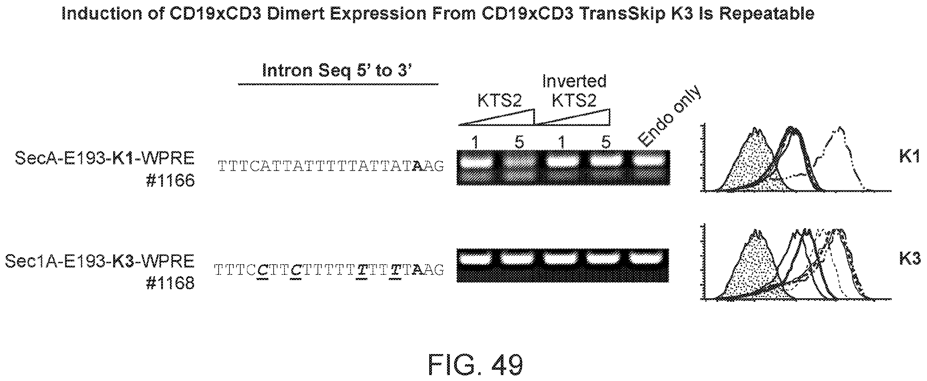

The exon skipping assay and T cell binding assay were repeated to confirm absence of leakage and presence of inducibility with the K3 CD19xCD3 TransSkip relative to the K1 CD19xCD3 TransSkip **(****FIG. 49****).** Also *see* **FIG. 48****.**

### Embodiments

Embodiment 1: a vector for use in a gene therapy comprising: a first polynucleotide sequence encoding a first antibody or its antigen-binding fragment thereof; and a second polynucleotide sequence encoding a second antibody or its antigen-binding fragment thereof.

Embodiment 2: the vector of embodiment 1, wherein the vector is a recombinant vector.

Embodiment 3: the vector of embodiment 1 or 2, wherein the vector is a viral vector.

Embodiment 4: the vector of any one of the embodiments 1-3, wherein the viral vector is a retroviral vector.

Embodiment 5: the vector of any one of the embodiments 1-4, wherein the viral vector is an adenovirus vector, an adeno-associated viral (AAV) vector, a lentiviral vector, a murine leukemia viral ("MLV") vector, an Epstein-Barr viral ("EBV") vector, or a herpes viral ("HSV") vector.

Embodiment 6: the vector of any one of the embodiments 1-5, wherein the AAV vector is an AAV1, AAV2, AAV4, AAV5, AAV6, AAV7, AAV8, AAV9, AAV10, AAV11, AAV12, AAV13, AAV PHP.B, AAV rh74, or AAV-DJ vector.

Embodiment 7: the vector of embodiment 6, wherein the AAV vector is AAV rh74 (GenBank accession number LP899424.1).

Embodiment 8: the vector of any one of the embodiments 1-7, wherein the first antibody or its antigen-binding fragment thereof specifically binds to an activating antigen on an immune effector cell and the second antibody or its antigen-binding fragment binds to a tumor antigen.

Embodiment 9: the vector of any one of the embodiments 1-7, wherein the first antibody or its antigen-binding fragment specifically binds to a tumor antigen and the second antibody or its antigen-binding fragment binds to an activating antigen on an immune effector cell.

Embodiment 10: the vector of any one of the embodiments 1-9, further comprising a third polynucleotide sequence encoding a third antibody or an antigen-binding fragment thereof, wherein the third antibody or the antigen-binding fragment thereof binds to an activating antigen on an immune effector cell or a tumor antigen.

Embodiment 11: the vector of any one of the embodiments 8-10, wherein the immune effector cell comprises a dendritic cell, a natural killer ("NK") cell, a macrophage, a T cell, or a B cell.

Embodiment 12: the vector of any one of the embodiments 8-11, wherein the immune effector cell is a T cell or an NK cell.

Embodiment 13: the vector of any one of the embodiments 8-12, wherein the activating antigen on the immune effector cell comprises CD3, CD2, CD4, CD8, CD19, LFA1, CD45, NKG2D, NKp44, NKp46, NKp30, DNAM, B7-H3, CD20, CD22, or a combination thereof.

Embodiment 14: the vector of any one of the embodiments 8-13, wherein the tumor antigen comprises one or more of an ephrin type-A receptor 2 (EphA2), interleukin (IL)-13r alpha 2, an EGFR VIII, a PSMA, an EpCAM, a GD3, a fucosyl GM1, a PSCA, a PLAC1, a sarcoma breakpoint, a Wilms Tumor 1, alphafetoprotein (AFP), carcinoembryonic antigen (CEA), CA-125, MUC-1, epithelial tumor antigen (ETA), tyrosinase, melanoma-associated antigen (MAGE), a hematologic differentiation antigen, a surface glycoprotein, a gangliosides (GM2), a growth factor receptor, a stromal antigen, a vascular antigen, receptor tyrosine kinase like orphan receptor 1 (ROR1), mesothelin, CD38, CD123, human epidermal growth factor receptor 2 (HER2), B-cell maturation antigen (BCMA), fibroblast activation protein (FAP) alpha, or a combination thereof.

Embodiment 15: the vector of any one of the embodiments 1-14, wherein the first antibody or its antigen-binding fragment thereof and the second antibody or its antigen-binding fragment thereof form a dimert.

Embodiment 16: the vector of embodiment 15, wherein the dimert is a bispecific antibody.

Embodiment 17: the vector of embodiment 15, wherein the dimert is a trispecific antibody.

Embodiment 18: the vector of embodiment 15 or 16, wherein the bispecific antibody comprises a polypeptide sequence at least 95% identical to any one of SEQ ID NOs: 13 or 15.

Embodiment 19: the vector of embodiment 15 or 17, wherein the trispecific antibody comprises a polypeptide sequence at least 95% identical to SEQ ID NO: 11.

Embodiment 20: the vector of any one of the embodiments 1-19, wherein the vector further comprises a polynucleotide sequence encoding a secretory peptide.

Embodiment 21: the vector of embodiment 20, wherein the secretory peptide comprises a secretory consensus sequence.

Embodiment 22: the vector of embodiment 20 or 21, wherein the secretory consensus sequence comprises at least 90%, 95%, 96%, 97%, 98%, 99%, or 100% sequence identity to SEQ ID NO: 51.

Embodiment 23: the vector of embodiment 20 or 21, wherein the secretory consensus sequence consist of SEQ ID NO: 51.

Embodiment 24: the vector of any one of the embodiments 20-23, wherein the secretory consensus sequence is encoded by a polynucleotide comprising SEQ ID NO: 52, or an equivalent thereof.

Embodiment 25: the vector of any one of the embodiments 20-24, wherein the secretory consensus sequence further comprises one, two, three, four, or more residues at the C-terminus of the sequence.

Embodiment 26: the vector of any one of the embodiments 20-25, wherein the secretory consensus sequence further comprises one, two, three, four, or more Ala residues at the C-terminus of the sequence.

Embodiment 27: the vector of any one of the embodiments 20-26, wherein the secretory consensus sequence further comprises one, two, or three Ala residues at the C-terminus of the sequence.

Embodiment 28: the vector of any one of the embodiments 20-27, wherein the secretory consensus sequence further comprises two Ala residues at the C-terminus of the sequence.

Embodiment 29: the vector of embodiment 28, wherein the secretory consensus sequence comprises at least 90%, 95%, 96%, 97%, 98%, 99%, or 100% sequence identity to SEQ ID NO: 55, or consists of SEQ ID NO: 55.

Embodiment 30: the vector of embodiment 28 or 29, wherein the secretory consensus sequence is encoded by a polynucleotide comprising SEQ ID NO: 56, or an equivalent thereof.

Embodiment 31: the vector of any one of the embodiments 20-30, wherein the secretory consensus sequence further comprises one Ala residue at the C-terminus of the sequence.

Embodiment 32: the vector of embodiment 31, wherein the secretory consensus sequence comprises at least 90%, 95%, 96%, 97%, 98%, 99%, or 100% sequence identity to SEQ ID NO: 53, or consists of SEQ ID NO: 53.

Embodiment 33: the vector of embodiment 31 or 32, wherein the secretory consensus sequence is encoded by a polynucleotide comprising SEQ ID NO: 54, or an equivalent thereof.

Embodiment 34: the vector of any one of the embodiments 1-33, wherein the secretory consensus sequence modulates expression and/or secretion of the dimert.

Embodiment 35: the vector of any one of the embodiments 1-34, wherein the secretory consensus sequence enhances expression and/or secretion of the dimert.

Embodiment 36: the vector of any one of the embodiments 1-35, wherein the vector further comprises a polynucleotide sequence encoding a dimerization domain.

Embodiment 37: the vector of embodiment 36, wherein the dimerization domain comprises a dimerization domain of human hepatocyte nuclear factor 1α (HNF1α).

Embodiment 38: the vector of embodiment 37, wherein the dimerization domain of HNF1α comprises a polypeptide sequence comprising at least 90%, 95%, 96%, 97%, 98%, 99%, or 100% sequence identity to SEQ ID NO: 47.

Embodiment 39: the vector of any one of the embodiments 1-38, wherein the vector further comprises a promoter.

Embodiment 40: the vector of embodiment 39, wherein the promoter is a constitutive promoter.

Embodiment 41: the vector of embodiment 39 or 40, wherein the promoter is a tissue-specific promoter.

Embodiment 42: the vector of any one of the embodiments 39-41, wherein the promoter comprises Rous sarcoma virus (RSV) LTR promoter, a cytomegalovirus (CMV) promoter, an SV40 promoter, a dihydrofolate reductase promoter, a β-actin promoter, a phosphoglycerol kinase (PGK) promoter, a U6 promoter, an EF1alpha short form (EFS) promoter, a phosphoglycerate kinase (PGK) promoter, a ubiquitin C (UbiC) promoter, an Alpha-1-antitrypsin, spleen focus-forming virus (SFFV) promoter, or chicken beta-actin (CBA) promoter.

Embodiment 43: the vector of any one of the embodiments 39-42, wherein the promoter is EFS, optionally comprising SEQ ID NO: 49, or an equivalent thereof.

Embodiment 44: the vector of any one of the embodiments 1-43, wherein the vector further comprises an enhancer.

Embodiment 45: the vector of embodiment 44, wherein the enhancer is an RSV enhancer, a CMV enhancer, and α-fetoprotein MERII enhancer.

Embodiment 46: the vector of any one of the embodiments 1-45, wherein the vector further comprises one or more additional regulatory elements.

Embodiment 47: the vector of any one of the embodiments 1-46, wherein the vector comprises a regulatory element comprises Woodchuck Hepatitis Virus (WHP) posttranscriptional regulatory element (WPRE), optionally SEQ ID NO: 50, or an equivalent thereof.

Embodiment 48: the vector of any one of the embodiments 1-47, wherein the vector comprises a 5' inverted terminal repeat (ITR) and a 3' ITR.

Embodiment 49: the vector of any one of the embodiments 1-48, where the vector comprises a sequence set forth in SEQ ID NOs: 4, 6, 8, 12, 14, 16-23, 30-33, or 40-46.

Embodiment 50: a composition comprises the vector of any one of embodiments 1-49, and a carrier, optionally a pharmaceutically acceptable carrier.

Embodiment 51: the composition of embodiment 50, wherein the composition is formulated for systemic administration.

Embodiment 52: the composition of embodiment 50, wherein the composition is formulated for local administration.

Embodiment 53: the composition of any one of the embodiments 50-52, wherein the composition is formulated for parenteral administration.

Embodiment 54: a method of treating a cancer in a subject in need thereof, comprising administering to the subject an effective amount of the vector of any one of embodiments 1-49 or the pharmaceutical composition of any one of the embodiments 50-53, wherein the vector expresses a therapeutic antic-cancer antibody or an antigen-binding fragment thereof.

Embodiment 55: the method of embodiment 54, further comprises administering to the subject an anti-cancer agent.

Embodiment 56: the method of embodiment 55, wherein the anti-cancer agent comprises an agent selected from a peptide, a polypeptide, a nucleic acid molecule, a small molecule, a viral particle, or combinations thereof.

Embodiment 57: the method of embodiment 56, wherein the viral particle is an oncolytic HSV particle.

Embodiment 58: the method of any one of the embodiments 54-57, wherein the subject is a mammal.

Embodiment 59: the method of any one of the embodiments 54-58, wherein the subject is human.

Embodiment 60: a method of producing a bispecific antibody or a trispecific antibody in a cell, comprising contacting a cell with a vector of any one of embodiments 1-49.

Embodiment 61: the method of embodiment 60, wherein the contacting comprises transfection, infection, transformation, electroporation, injection, microinjection, or a combination thereof.

Embodiment 62: the method of embodiment 60 or 61, wherein the cell comprises a fibroblast, a skeletal cell, an epithelial cell, a muscle cell, a neural cell, an endocrine cell, a melanocyte, a blood cell, or combination thereof.

Embodiment 63: the method of any one of the embodiments 60-62, wherein the bispecific antibody comprises a polypeptide sequence at least 95% identical to SEQ ID NOs: 13 or 15.

Embodiment 64: the method of any one of the embodiments 60-62, wherein the trispecific antibody comprises a polypeptide sequence at least 95% identical to SEQ ID NO: 11.

Embodiment 65: the method of any one of the embodiments 60-62, wherein the bispecific antibody is encoded by a polynucleotide sequence at least 95% identical to SEQ ID NOs: 14, 16, 22, 23, 30-33, or 40-46.

Embodiment 66: the method of any one of the embodiments 60-62, wherein the trispecific antibody is encoded by a polynucleotide sequence at least 95% identical to SEQ ID NO: 12.

Embodiment 67: a kit comprising the vector of any one of the embodiments 1-49 or the pharmaceutical composition of any one of the embodiments 50-53.

Embodiment 68: the kit of embodiment 67, further comprises an instructional material.

### Equivalents

It is to be understood that while the disclosure has been described in conjunction with the above embodiments, that the foregoing description and examples are intended to illustrate and not limit the scope of the disclosure. Other aspects, advantages and modifications within the scope of the disclosure will be apparent to those skilled in the art to which the disclosure pertains.

Unless otherwise defined, all technical and scientific terms used herein have the same meaning as commonly understood by one of ordinary skill in the art to which this disclosure belongs. All nucleotide sequences provided herein are presented in the 5' to 3' direction.

The embodiments illustratively described herein may suitably be practiced in the absence of any element or elements, limitation or limitations, not specifically disclosed herein. Thus, for example, the terms "comprising," "including," containing," etc. shall be read expansively and without limitation. Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the disclosure.

Thus, it should be understood that although the present disclosure has been specifically disclosed by specific embodiments and optional features, modification, improvement, and variation of the embodiments therein herein disclosed may be resorted to by those skilled in the art, and that such modifications, improvements and variations are considered to be within the scope of this disclosure. The materials, methods, and examples provided here are representative of particular embodiments, are exemplary, and are not intended as limitations on the scope of the disclosure.

The scoped of the disclosure has been described broadly and generically herein. Each of the narrower species and subgeneric groupings falling within the generic disclosure also form part of the disclosure. This includes the generic description with a proviso or negative limitation removing any subject matter from the genus, regardless of whether or not the excised material is specifically recited herein.

In addition, where features or aspects of the disclosure are described in terms of Markush groups, those skilled in the art will recognize that embodiments of the disclosure may also thereby be described in terms of any individual member or subgroup of members of the Markush group.
**Embodiments disclosed herein are enumerated as clauses below:**

### Clauses

1. A polynucleotide or vector comprising:
   (a) a first polynucleotide sequence comprising a first portion of an open reading frame encoding a first polypeptide;
   (b) a second polynucleotide sequence comprising a second portion of the open reading frame encoding the first polypeptide;
   (c) a third polynucleotide sequence encoding a second polypeptide; and
   (d) a gene regulation polynucleotide sequence located between the first polynucleotide and the second polynucleotide.
2. The polynucleotide or vector of clause 1, wherein the gene regulation polynucleotide sequence comprises a splice donor site, an upstream intron, an exon comprising more than one stop codon sequences in their respective reading frames, a downstream intron, and a splice acceptor site.
3. The polynucleotide or vector of clause 1 or 2, wherein the gene-regulation polynucleotide sequence comprises one or more of a binding sequence for an antisense oligonucleotide, a binding sequence for doxycycline, or a polynucleotide sequence encoding a riboswitch.
4. The polynucleotide or vector of any one of the clauses 1-3, wherein the antisense oligonucleotide is a morpholino oligonucleotide.
5. The polynucleotide or vector of clause 4, wherein the binding sequence for the morpholino oligonucleotide comprises a polynucleotide sequence at least 95% identical to SEQ ID NO. 24 or 25.
6. The polynucleotide or vector of clause 4, wherein the morpholino oligonucleotide comprises a polynucleotide sequence at least 95% identical to SEQ ID NOs: 27 or 28.
7. The polynucleotide or vector of clause 2, wherein the stop codon comprises:
   an oligonucleotide of the group of: TAA, TAG, or TGA;
   a polynucleotide sequence of TAAxTAGxTGAxTAGxTAAxTGAx (SEQ ID NO. 1), wherein x is any nucleotide; or
   a polynucleotide sequence of TAATTAGTTGATTAGTTAATTGAT (SEQ ID NO. 2) , or an equivalent thereof.
8. The polynucleotide or vector of any one of the clauses 1-7, wherein the gene regulation polynucleotide comprises a polynucleotide sequence at least 95% identical to SEQ ID NO. 21.
9. The polynucleotide or vector of any one of the clauses 1-8, wherein the first polypeptide is a first antibody or its antigen-binding fragment thereof and the second polypeptide is a second antibody or its antigen-biding fragment thereof.
10. The polynucleotide or vector of any one of the clauses 1-9, wherein the first antibody or its antigen-binding fragment thereof specifically binds to an activating antigen on an immune effector cell and the second antibody or its antigen-binding fragment binds to a tumor antigen.
11. The polynucleotide or vector of any one of the clauses 1-9, wherein the first antibody or its antigen-binding fragment specifically binds to a tumor antigen and the second antibody or its antigen-binding fragment binds to an activating antigen on an immune effector cell.
12. The polynucleotide or vector of any one of the clauses 1-11, further comprising a fourth polynucleotide sequence encoding a third antibody or an antigen-binding fragment thereof, wherein the third antibody or the antigen-binding fragment thereof binds to an activating antigen on an immune effector cell or a tumor antigen.
13. The polynucleotide or vector of any one of clauses 10-12, wherein the immune effector cell comprises a dendritic cell, a natural killer ("NK") cell, a macrophage, a T cell, a B cell, or combination thereof.
14. The polynucleotide or vector of any one of clauses 10-12, wherein the immune effector cell is a T cell or an NK cell.
15. The polynucleotide or vector of any one of clauses 10-12, wherein the activating antigen on the immune effector cell comprises CD3, CD2, CD4, CD8, CD19, LFA1, CD45, NKG2D, NKp44, NKp46, NKp30, DNAM, B7-H3, CD20, CD22, or combination thereof.
16. The polynucleotide or vector of any one of clauses 10-12, wherein the tumor antigen comprises one or more of an ephrin type-A receptor 2 (EphA2), interleukin (IL)-13r alpha 2, an EGFR VIII, a PSMA, an EpCAM, a GD3, a fucosyl GM1, a PSCA, a PLAC1, a sarcoma breakpoint, a Wilms Tumor 1, alphafetoprotein (AFP), carcinoembryonic antigen (CEA), CA-125, MUC-1, epithelial tumor antigen (ETA), tyrosinase, melanoma-associated antigen (MAGE), a hematologic differentiation antigen, a surface glycoprotein, a gangliosides (GM2), a growth factor receptor, a stromal antigen, a vascular antigen, receptor tyrosine kinase like orphan receptor 1 (ROR1), mesothelin, CD38, CD123, human epidermal growth factor receptor 2 (HER2), B-cell maturation antigen (BCMA), fibroblast activation protein (FAP) alpha, or a combination thereof.
17. The polynucleotide or vector of any one of the clauses 1-16, wherein the vector is a recombinant vector, optionally a viral vector, and is capable of expressing a pre-mRNA that encodes a dimert, when the pre-mRNA is in contact with an antisense oligonucleotide, optionally a morpholino oligonucleotide.
18. The polynucleotide or vector of clause 17, wherein the dimert is a bispecific antibody.
19. The polynucleotide or vector of clause 17, wherein the dimert is a trispecific antibody.
20. The polynucleotide or vector of clause 18 or 19, wherein the bispecific antibody or the trispecific antibody comprises the first antibody or its antigen-binding fragment thereof, and the second antibody or its antigen-binding fragment.
21. The polynucleotide or vector of clause 18, wherein the bispecific antibody comprises a polypeptide sequence at least 95% identical to any one of SEQ ID NOs. 13 or 15.
22. The polynucleotide or vector of any one of the clauses 1-21, wherein the vector expresses a pre-mRNA that encodes a trispecific antibody, when the pre-mRNA is in contact with an antisense oligonucleotide, optionally a morpholino oligonucleotide.
23. The polynucleotide or vector of clause 22, wherein the trispecific antibody comprises the first antibody or its antigen-binding fragment, the second antibody its antigen-binding fragment, and the third antibody or its antigen-binding fragment.
24. The polynucleotide or vector of clause 22, wherein the trispecific antibody comprises a polypeptide sequence at least at least 95% identical to SEQ ID NO. 11.
25. The polynucleotide or vector of any one of the clauses 1-24, wherein the first antibody and the second antibody are each independently a single-chain variable fragment.
26. The polynucleotide or vector of any one of the clauses 1-25, further comprising a polynucleotide sequence encoding a secretory peptide, optionally a secretory consensus sequence.
27. The polynucleotide or vector of any one of the clauses 1-26, further comprising a polynucleotide sequence encoding a dimerization domain.
28. The polynucleotide or vector of any one of the clauses 1-27, further comprising a 5 ' inverted terminal repeat (ITR) and a 3' ITR.
29. The polynucleotide or vector of any one of the clauses 1-28, where the vector comprises a sequence set forth in SEQ ID NOs: 4, 6, 8, 12, 14, 16-23, 30-33, or 40-46.
30. The polynucleotide or vector of any one of the clauses 1-29, wherein the vector is a recombinant viral vector that comprises a backbone vector selected from the group of a retroviral vector, a lentiviral vector, a murine leukemia viral ("MLV") vector, an Epstein-Barr viral ("EBV") vector, an adenoviral vector, a herpes viral ("HSV") vector, or an adeno-associated viral ("AAV") vector.
31. The polynucleotide or vector of any one of the clauses 1-30, wherein the vector is an AAV vector, optionally a self-complementary AAV vector, further optionally an AAV rh74 vector.
32. A composition comprises the polynucleotide or vector of any one of clauses 1-31, and a carrier, optionally a pharmaceutically acceptable carrier.
33. A method of treating a cancer in a subject in need thereof, comprising administering to the subject an effective amount of the recombinant polynucleotide or vector of any one of clauses 1-31 or the pharmaceutical composition of clause 32, wherein the polynucleotide or vector expresses a therapeutic antic-cancer antibody or an antigen-binding fragment thereof.
34. The method of clause 33, further comprising administering to the subject an effective amount of an antisense oligonucleotide, optionally a morpholino oligonucleotide.
35. The method of clause 33 or 34, further comprises administering to the subject an anti-cancer agent.
36. The method of clause 35, wherein the anti-cancer agent comprises an agent selected from a peptide, a polypeptide, a nucleic acid molecule, a small molecule, a viral particle, or combinations thereof.
37. The method of clause 36, wherein the viral particle is an oncolytic HSV particle.
38. The method of any one of clauses 33-37, wherein the subject is a mammal.
39. The method of any one of clauses 33-38, wherein the subject is human.
40. A method of producing a bispecific antibody or a trispecific antibody in a cell, comprising contacting a cell comprising a vector of any one of clauses 1-31 with an effective amount of an antisense oligonucleotide, optionally a morpholino oligonucleotide.
41. The method of clause 40, wherein the morpholino oligonucleotide comprise a sequence at least 95% identical with a stereopure polynucleotide.
42. The method of clause 40, wherein the vector is introduced to the cell by transfection, infection, transformation, electroporation, injection, microinjection, or the combination thereof.
43. The method of any one of clauses 40-42, wherein the cell comprises a fibroblast, a skeletal cell, an epithelial cell, a muscle cell, a neural cell, an endocrine cell, a melanocyte, a blood cell, or combination thereof.
44. The method of clause any one of clauses 40-43, wherein the bispecific antibody comprises a polypeptide sequence at least 95% identical to SEQ ID NOs. 13 or 15.
45. The method of clause any one of clauses 40-44, wherein the trispecific antibody comprises a polypeptide sequence at least 95% identical to SEQ ID NO. 11.
46. The method of clause any one of clauses 40-43, wherein the bispecific antibody is encoded by a polynucleotide sequence at least 95% identical to SEQ ID NOs. 14, 16, 22, 23, 30-33, or 40-46.
47. The method of clause any one of clauses 40-44, wherein the trispecific antibody is encoded by a polynucleotide sequence at least 95% identical to SEQ ID NO. 12.
48. A kit comprising the polynucleotide or vector of any one of clauses 1-31 or the pharmaceutical composition of clause 32.
49. The kit of clause 48, further comprises an instructional material.

## Claims

1. A recombinant adeno-associated viral (rAAV) vector for use in a gene therapy comprising:
a first polynucleotide sequence encoding a first antigen-binding fragment; and a second polynucleotide sequence encoding a second antigen-binding fragment;
wherein the first antigen-binding fragment and the second antigen-binding fragment form a dimert;
wherein the vector further comprises a polynucleotide sequence encoding a secretory peptide,
wherein the secretory peptide comprises a secretory consensus sequence;
wherein the vector further comprises a promoter; and
wherein the vector comprises a 5' inverted terminal repeat (ITR) and a 3' ITR.

2. The rAAV vector according to claim 1, wherein the AAV vector is an AAV1, AAV2, AAV4, AAV5, AAV6, AAV7, AAV8, AAV9, AAV10, AAV11, AAV12, AAV13, AAV PHP.B, AAV rh74, or AAV-DJ vector; optionally, wherein the AAV vector is an AAV2 vector.

3. The rAAV vector according to any preceding claim, wherein the promoter comprises Rous sarcoma virus (RSV) LTR promoter, a cytomegalovirus (CMV) promoter, an SV40 promoter, a dihydrofolate reductase promoter, a β-actin promoter, a U6 promoter, an EF1alpha short form (EFS) promoter, a phosphoglycerate kinase (PGK) promoter, a ubiquitin C (UbiC) promoter, an Alpha-1-antitrypsin promoter, spleen focus-forming virus (SFFV) promoter, or chicken beta-actin (CBA) promoter.

4. The rAAV vector according to any of claims 1 to 3, wherein the promoter comprises a CAG promoter.

5. The rAAV vector according to any preceding claim, wherein:
(i) the first antigen-binding fragment binds to CD3 and the second antigen-binding fragment binds to CD19; or
(ii) the first antigen-binding fragment binds to CD3 and the second antigen-binding fragment binds to GD2.

6. The rAAV vector according to any preceding claim, wherein the secretory consensus sequence is encoded by a polynucleotide comprising SEQ ID NO: 52;
or wherein the secretory consensus sequence further comprises one Ala residue at a C-terminus of the sequence, and is encoded by a polynucleotide comprising SEQ ID NO: 54; or
wherein the secretory consensus sequence further comprises two Ala residues at a C-terminus of the sequence, and is encoded by a polynucleotide comprising SEQ ID NO: 56.

7. The rAAV vector according to claim 1, wherein the secretory consensus sequence comprises at least 90%, 95%, 96%, 97%, 98%, 99%, or 100% sequence identity to SEQ ID NO: 51;
wherein the secretory consensus sequence further comprises one Ala residue at the C-terminus of the sequence and wherein the secretory consensus sequence comprises at least 90%, 95%, 96%, 97%, 98%, 99%, or 100% sequence identity to SEQ ID NO: 53, or consists of SEQ ID NO: 53; or
wherein the secretory consensus sequence further comprises two Ala residues at the C-terminus of the sequence, and wherein the secretory consensus sequence comprises at least 90%, 95%, 96%, 97%, 98%, 99%, or 100% sequence identity to SEQ ID NO: 55, or consists of SEQ ID NO: 55.

8. The rAAV vector according to claim 1, wherein the dimert comprises two or more single chain variable fragments (scFv) in which each scFv recognizes a surface polypeptide expressed on two different cells; optionally, wherein the two or more scFvs of the dimert are linked by a linker; further optionally, the linker is a peptide linker that facilitates binding of each scFv to its respective target polypeptide.

9. The rAAV vector according to claim 1, wherein the dimert is a bispecific antibody, or wherein the dimert is a trispecific antibody.

10. The rAAV vector according to claim 9, wherein the dimert is a bispecific T cell engager (BiTE) antibody, optionally comprising blinatumomab.

11. The rAAV vector according to claim 1, wherein the dimert comprises a first scFv that specifically binds to an activating antigen on an immune effector cell and a second scFv that specifically binds to a tumor antigen; or a first scFv that specifically binds to a tumor antigen and a scFv that specifically binds to an activating antigen on an immune effector cell.

12. The rAAV vector of claim 11, wherein the activating antigen on the immune effector cell comprises CD3, CD2, CD4, CD8, CD19, LFA1, CD45, NKG2D, NKp44, NKp46, NKp30, DNAM, B7-H3, CD20, CD22, interleukin 21 receptor (IL21R), GD2 or a combination thereof; and/or wherein the tumor antigen comprises one or more of an ephrin type-A receptor 2 (EphA2), interleukin (IL)-13r alpha 2, an EGFR VIII, a PSMA, an EpCAM, a GD3, a fucosyl GM1, a PSCA, a PLAC1, a sarcoma breakpoint, a Wilms Tumor 1, alphafetoprotein (AFP), carcinoembryonic antigen (CEA), CA-125, MUC-1, epithelial tumor antigen (ETA), tyrosinase, melanoma-associated antigen (MAGE), a hematologic differentiation antigen, a surface glycoprotein, a gangliosides (GM2), a growth factor receptor, a stromal antigen, a vascular antigen, receptor tyrosine kinase like orphan receptor 1 (ROR1), mesothelin, CD38, CD123, human epidermal growth factor receptor 2 (HER2), B-cell maturation antigen (BCMA), fibroblast activation protein (FAP) alpha, GD2, IL21R or a combination thereof.

13. A pharmaceutical composition comprising the rAAV vector according to any preceding claim and a pharmaceutically acceptable carrier.

14. A recombinant adeno-associated viral (rAAV) vector for use in a method of treating cancer in a subject in need thereof, wherein said method comprises the step of administering an effective amount of the rAAV vector according to any one of claims 1 to 12, or the pharmaceutical composition according to claim 13 to the subject.

15. The rAAV vector for use of claim 14, wherein the cancer is a hematologic malignancy comprising acute myeloid leukemia (AML), chronic lymphocytic leukemia (CLL), small lymphocytic lymphoma (SLL), follicular lymphoma (FL), diffuse large B-cell lymphoma (DLBCL), mantle cell lymphoma (MCL), Waldenstrom's macroglobulinemia, multiple myeloma, extranodal marginal zone B cell lymphoma, nodal marginal zone B cell lymphoma, Burkitt's lymphoma, non-Burkitt high grade B cell lymphoma, primary mediastinal B-cell lymphoma (PMBL), immunoblastic large cell lymphoma, precursor B-lymphoblastic lymphoma, B cell pro lymphocytic leukemia, lymphoplasmacytic lymphoma, splenic marginal zone lymphoma, plasma cell myeloma, plasmacytoma, mediastinal (thymic) large B cell lymphoma, intravascular large B cell lymphoma, primary effusion lymphoma, and lymphomatoid granulomatosis.

16. The rAAV vector for use of claim 14, wherein the cancer is acute lymphocytic leukemia (ALL).

17. The rAAV vector for use of claim 14, wherein the cancer is a B-cell leukemia or a B-cell lymphoma, optionally, wherein the B cell leukemia comprises B-cell chronic lymphocytic leukemia (or B-cell small lymphocytic lymphoma); acute lymphoblastic leukemia, mature B-cell type; B-cell prolymphocytic leukemia; precursor B lymphoblastic leukemia; and hairy cell leukemia.

18. The rAAV vector for use of claim 14, wherein the cancer is a solid tumor, optionally, wherein the solid tumor comprises bladder cancer, bone cancer, brain cancer, breast cancer, colorectal cancer, esophageal cancer, eye cancer, head and neck cancer, kidney cancer, lung cancer, melanoma, mesothelioma, ovarian cancer, pancreatic cancer, prostate cancer, and stomach cancer.

19. The rAAV vector for use of claim 18, wherein the brain cancer is glioblastoma.

20. The rAAV vector for use of claim 18, wherein the solid tumor is neuroblastoma.
